(19) Europäisches Patentamt

European Patent Office

Office européen des brevets

(11) **EP 3 596 465 B1**

(12) # EUROPEAN PATENT SPECIFICATION

(45) Date of publication and mention
of the grant of the patent:
**07.05.2025  Bulletin 2025/19**

(21) Application number: **18767465.0**

(22) Date of filing: **16.03.2018**

(51) International Patent Classification (IPC):
*G01N 33/53* (2006.01)       *B01L 3/00* (2006.01)
*B81B 7/04* (2006.01)       *C12M 1/18* (2006.01)
*C12M 1/34* (2006.01)       *C12N 5/078* (2010.01)
*C12M 3/06* (2006.01)       *C12M 1/00* (2006.01)
*G01N 33/50* (2006.01)       *G01N 33/543* (2006.01)
*G01N 33/80* (2006.01)

(52) Cooperative Patent Classification (CPC):
**C12M 23/16; B01L 3/502715; B01L 3/502761;
C12M 23/20; C12M 41/36; G01N 33/5005;
G01N 33/53; G01N 33/543; G01N 33/80;**
B01L 2200/0652; B01L 2300/0636;
B01L 2300/0816; B01L 2400/0487; G01N 2800/10;
G01N 2800/22

(86) International application number:
**PCT/US2018/022888**

(87) International publication number:
**WO 2018/170412 (20.09.2018 Gazette 2018/38)**

(54) **BIOCHIP HAVING MICROCHANNEL PROVIDED WITH CAPTURING AGENT FOR PERFORMING CYTOLOGICAL ANALYSIS**

BIOCHIP MIT MIKROKANAL MIT AUFNAHMEMITTEL ZUR DURCHFÜHRUNG VON ZYTOLOGISCHER ANALYSE

BIOPUCE AYANT UN MICROCANAL POURVU D'UN AGENT DE CAPTURE POUR EFFECTUER UNE ANALYSE CYTOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.03.2017  US 201762472437 P
10.10.2017  US 201762570380 P
08.12.2017  US 201762596630 P**

(43) Date of publication of application:
**22.01.2020  Bulletin 2020/04**

(73) Proprietor: **Case Western Reserve University
Cleveland, OH 44106 (US)**

(72) Inventors:
• **GURKAN, Umut A.**
  **Cleveland**
  **Ohio 44106 (US)**

• **KUCUKAL, Erdem**
  **Cleveland**
  **Ohio 44106 (US)**
• **ALAPAN, Yunus**
  **Cleveland**
  **Ohio 44106 (US)**
• **KIM, Myeongseop**
  **Cleveland**
  **Ohio 44106 (US)**
• **KREBS, John C.**
  **Cleveland**
  **Ohio 44106 (US)**
• **LITTLE, Jane A.**
  **Cleveland**
  **Ohio 44106 (US)**
• **WERA, Glenn D.**
  **Cleveland**
  **Ohio 44106 (US)**

**(Cont. next page)**

(74) Representative: **Maiwald GmbH**
**Elisenhof**
**Elisenstraße 3**
**80335 München (DE)**

(56) References cited:
**WO-A1-2016/019142**     **WO-A1-2016/019142**
**WO-A1-2016/090264**     **WO-A1-2016/164359**
**US-A1- 2009 298 067**     **US-A1- 2010 151 474**
**US-A1- 2015 376 701**

• **CHENG XUANHONG ET AL: "A microfluidic device for practical label-free CD4(+) T cell counting of HIV-infected subjects", LAB ON A CHIP, ROYAL SOCIETY OF CHEMISTRY, vol. 7, no. 2, 24 November 2006 (2006-11-24), pages 170 - 178, XP008133987, ISSN: 1473-0197**
• **KREBS ET AL.: "Microfluidic Processing of Synovial Fluid for Cytological Analysis", BIOMEDICAL MICRODEVICES, vol. 2, no. 19, 3 April 2017 (2017-04-03), pages 1 - 9, XP036242823**
• **ALAPAN ET AL.: "Dynamic Deformability of Sickle Red Blood Cells in Microphysiological Flow", TECHNOLOGY (SINGAP WORLD SCI), vol. 4, no. 2, 19 February 2016 (2016-02-19), pages 71 - 79, XP055542208**
• **CHENG ET AL.: "A microfluidic device for practical label-free CD 4+ T cell counting of HIV-infected subjects", LAB ON A CHIP, vol. 7, no. 2, 24 November 2006 (2006-11-24), pages 170 - 178, XP008133987**
• **TRAMPUZ ET AL.: "Synovial Fluid Leukocyte Count and Differential for the Diagnosis of Prosthetic Knee Infection", THE AMERICAN JOURNAL OF MEDICINE, vol. 117, no. 8, 15 October 2004 (2004-10-15), pages 556 - 562, XP004591474**

**Description**

**RELATED APPLICATIONS**

This application claims the benefit of U.S. Provisional Appln. Serial No. 62/596,630, filed December 8, 2017, U.S Provisional Appln. Serial No. 62/570,380, filed October 10, 2017, and U.S. Provisional Appln. Serial No. 62/472,437, filed March 16, 2017.

**FIELD OF THE INVENTION**

This application is related to biochips, and particularly relates to biochips having at least one microchannel provided with an agent for capturing cells of interest within a fluid sample delivered to the microchannel in order to perform cytological analysis. The application also relates to a method of evaluating a fluid sample from a subject.

**BACKGROUND**

About 3 million people worldwide suffer from sickle cell disease (SCD), mostly in Africa, India, and the Middle East, with an estimated 100,000 affected in the U.S., according to the Centers for Disease Control, and Prevention. SCD affects 1 in 375 African American newborns born in the U.S.

The World Health Organization (WHO) has declared SCD a public health priority. The greatest burden of SCD is in low-income countries, especially in Africa. An estimated 50-80% of the babies born with SCD in Africa die before the age of 5, *i.e.,* more than 600 babies die every day, due to lack of diagnosis. Very few infants are screened in Africa because of the high cost and level of skill needed to run traditional tests. Current methods are too costly and take too much time - 2-6 weeks - to enable equitable and timely diagnosis. It is estimated by the WHO that 70% of SCD-related deaths are preventable with simple, cost-efficient interventions, such as early point-of-care (POC) diagnosis by newborn screening, followed by treatment and care. Early diagnosis through newborn screening, followed by simple interventions, has dramatically reduced the SCD-related mortality in the US. These strategies, however, have not been widely available in Africa and other third world countries due to limited resources.

Moreover, the initiation of vaso-occlusive crisis (VOC) events in SCD is a multicellular paradigm, likely triggered by aberrant adhesive interactions between red blood cells (RBCs) and microvascular bed, and further mediated by impaired RBC biophysical properties. In most cases, these events are followed by or simultaneous with the activation of other microcirculatory components, including white blood cells (WBCs), platelets, and endothelial cells. The interplay between these components, including the collective adhesive events, takes place under a wide spectrum of shear rates determined by the unique geometrical and morphological features of the human microvasculature, as well as by the local changes in the vascular dimensions upon cell-endothelium interactions. The flow conditions may dynamically and continuously change even within the same branch of the microvessel during this entire process. WO 2016/019142 A1 relates to biochips for rapidly and easily diagnosing or identifying hemoglobin disorders like hemoglobinopathies. Cheng Xuanhong et al., Lab on a chip, Royal Society of Chemistry, vol. 7, no. 2, 24 November 2006, pages 170-178, describes a microfluidic device for practical label-free CD4+ T cell counting of HIV-infected subjects. WO 2016/164359 A1 relates to microfluidic devices and methods for isolating and analyzing rare cell clusters like cancer stem cells and cancer stem cell clusters in biological samples.

**SUMMARY**

The invention is defined by the appended claims. This application describes a microfluidic device in the form of a biochip having microchannels provided on or functionalized with a capturing agent for capturing cells of interest to be analyzed from a fluid sample obtained from a subject. The microfluidic device includes a housing including at least one microchannel that defines at least one cell adhesion region. The at least one cell adhesion region includes at least one capturing agent that adheres or captures to a cell of interest in a fluid sample when the fluid sample containing the cells is passed through the at least one microchannel. The microfluidic device also includes an imaging system for measuring the morphology and/or quantity of the cells of interest adhered by the at least one capturing agent to the at least one microchannel when the fluid sample is passed therethrough.

The cells of interest are red blood cells (RBCs). The capturing agents include bioaffinity ligands such as fibronectin (FN), laminin (LN), thrombospondin (TSP), Willebrand Factor (vWF) and/or a Cl46 antibody for detecting hemoglobin phenotypes. In each case, the biochip is compact and requires a very small fluid sample from the subject, *e.g.,* on the microscale.

The imaging system detects and measures the morphology and/or quantity of captured cells of interest within each microchannel. The imaging system can be a lens-based imaging system or a lensless/mobile imaging system, *e.g.,*

cellular phone camera. The imaging system can use software to analyze the images of the microchannels and can provide real-time feedback to the subject of the results of the image acquisition/analysis. These results, in turn, can be readily transmitted to a primary care provider and/or stored in a medical record database.

The microchannels in the biochip can have a constant or variable width along their length. Varying the microchannel width provides continuously changing shear rates (shear gradient) along its length. Providing a shear gradient along the flow direction allows for the investigation of shear-dependent adhesion of cells at a single flow rate. The microchannel geometry can be configured such that both the mean flow velocity and shear stress decrease along the flow direction while the flow rate is constant.

The microfluidic device can simulate physiologically relevant shear gradients of microcirculatory blood flow at a constant single volumetric flow rate. Using this system, shear-dependent adhesion and deformability of, for example, RBCs from patients with SCD can be investigated using vascular endothelial protein functionalized microchannels. It was shown that shear dependent adhesion of RBCs exhibits a heterogeneous behavior based on adhesion type and cell deformability in a microfluidic flow model, which correlates clinically with inflammatory markers and iron overload in patients with SCD. This revealed the complex dynamic interactions between RBC-mediated microcirculatory occlusion and clinical outcomes in SCD. These interactions may also be relevant to other microcirculatory disorders.

The microfluidic device can be used with a micro-gas exchanger fluidly connected to the at least one microchannel for varying the oxygen content of the fluid sample containing the cells. The micro-gas exchanger can include a gas-permeable inner tube inserted within a gas-impermeable outer tube. Fluid, such as blood or synovial fluid, containing the cells of interest can be delivered through the inner tube such that the fluid exchanges gases through the permeable tubing wall with a control gas, *e.g.,* 5% $CO_2$ and 95% $N_2$, between the tubes. The oxygen content of the fluid exiting the micro-gas exchanger is controlled to thereby control the oxygen content of the fluid delivered to the microchannel.

## BRIEF DESCRIPTION OF THE FIGURES

Figs. 1A-B illustrate an example microfluidic biochip that evaluates cellular, membrane and adhesive interactions.
Fig. 2A illustrates an example biochip with a microchannel configured to provide a shear gradient at a single flow rate.
Fig. 2B is a schematic view of the human microvasculature system, with characteristic shear rates determined by the vessel geometry and local flow conditions.
Figs. 2C-2D illustrate an imaging system for evaluating cell adhesion within the microfluidic biochip.
Fig. 3 illustrates another comparative example microfluidic device for capturing WBCs from synovial fluid samples.
Figs. 4A-4C illustrate an imaging system for evaluating RBC adhesion and deformability in physiological flow conditions.
Figs. 5A-5B are images showing the aspect ratio (AR) and flow of healthy and sickle RBCs presented under no flow, flow, and detachment conditions.
Figs. 6A-6D show data related to HbS-containing, non-deformable RBC detachment at relatively higher flow velocity, shear stress, and drag force as compared with HbA and HbS-containing deformable RBCs.
Figs. 7A-7L are images showing the determination of cell adhesion sites based on analysis of projected cell outlines at flow initiation for HbA- and HbS-containing RBCs.
Figs. 8A-8B show data related to the AR and deformability of healthy and sickle RBCs presented under no flow, flow, and detachment conditions.
Figs. 9A-9F are images and data showing variations in RBC adhesion in FN- and LN-functionalized microchannels amongst SCD hemoglobin phenotypes.
Figs. 10A-10B show data related to RBC adhesion in HbSS subjects with low HbF and high HbF.
Figs. 11A-11H show data related to the association between RBC adhesion and lactate dehydrogenase (LDH), platelet counts (plts), and reticulocyte counts (retics) in HbSS.
Figs. 12A-12G show data related to heterogeneity in adhered RBCs in FN functionalized microchannels and its association with serum LDH levels.
Figs. 13A-13D are images and cellular analysis for quantifying captured WBC subpopulations from synovial fluid (comparative).
Figs. 14A-14C are images and data showing the specificity of WBCs captured in the microfluidic device of Fig. 12A (comparative).
Figs. 15A-15D illustrate the effect of PBS dilution on cell capture efficiency in synovial fluid samples (comparative).
Figs. 16A-16C show data related to the effect of flow rate on cell capture efficiency in synovial fluid samples (comparative).
Figs. 17A-17D show data related to the effect of hyaluronidase enzyme on synovial fluid viscosity and cell capture efficiency (comparative).

Figs. 18A-18D show data comparing cytometer cell counts and fluorescent activated cell sorting (FACS) cell counts for synovial samples (comparative).

Figs. 19A-19C illustrate another example microfluidic device for measuring RBC adhesion under physiological flow and hypoxic conditions

Figs. 20A-20C illustrate a micro-gas exchanger system using a model finger and pulse-oximeter.

Figs. 21A-21C illustrate computational modeling of flow in the micro-gas exchanger of Figs. 20A-20C.

Figs. 22A-22B are images showing deoxygenation of adhered RBCs in blood samples from HbSS and HbAA subjects.

Figs. 23A-23C are images and data showing heterogeneity in RBC adhesion response to hypoxia.

Figs. 24A-24B show data related to heterogeneity in analyzed SCD subjects based on changes in the number of adhered RBCs in response to hypoxia.

Figs. 25A-26D show data related to RBC adhesion responsiveness to hypoxia over different clinical phenotypes.

Figs. 26A-26D show data related to RBC adhesion responsiveness to hypoxia over different clinical phenotypes.

Fig. 27 shows data related to SCD subject RBC adhesion responsiveness to hypoxia over different ages.

Figs. 28A-28D show data related to SCD subject RBC adhesion responsiveness to hypoxia over different clinical phenotypes.

Fig. 29 shows data reflecting the effect of anti-BCAM antibody on RBC adhesion in normoxic and hypoxic conditions for responsive and non-responsive patient populations.

Figs. 30A-30B are images and data showing the quantification of fluorescently labeled LN and FN immobilized in shear-gradient microchannels.

Fig. 30C is a graph showing flow velocity and shear rate contours on in a microchannel having a variable width.

Figs. 31A-31C are images and data showing shear adhesion of RBCs in a variable width microchannel.

Fig. 32A is a graph reflecting average shear-dependent adhesion curves in LN functionalized microchannels.

Figs. 32B-32C show data related to adherent deformable and non-deformable cell numbers in LN- and FN-functionalized shear gradient microchannels.

Fig. 33 is a graph showing adherent RBC counts across different shear rates, illustrating the defined parameters herein.

Figs. 34A-34C show data related to the adhesion of deformable and non-deformable sickle RBCs to LN- and FN-functionalized microchannels.

Figs. 35A-35C show data related to patient-specific, shear-dependent adhesion curves in LN-functionalized microchannels.

Figs. 36A-36C show data related to patient-specific, normalized, shear-dependent adhesion curves in LN-functionalized microchannels.

Figs. 37A-37C show data related to the shear dependence of sickle RBCs to LN and clinical parameters.

Figs. 38A-38E show a microfluidic device having a mobile imaging system and cell adhesion metrics associated with the mobile imaging system.

Figs. 39A-39C show data comparing the mobile imaging system to conventional microscope counts.

Figs. 40A-40D show images related to using the mobile imaging system with a cellular application.

Other objects and advantages and a fuller understanding of the invention will be had from the following detailed description and the accompanying drawings.

## DETAILED DESCRIPTION

This application is related to microfluidic biochip devices having at least one microchannel provided on or functionalized with an agent for capturing cells of interest within a fluid sample from a subject delivered to the microchannel in order to perform cytological analysis, and methods for evaluating a fluid sample from a subject.

### Definitions

To facilitate the understanding of this invention, a number of terms are defined below. Terms defined herein have meanings as commonly understood by a person of ordinary skill in the areas relevant to the present invention. Terms such as "a", "an", and "the" are not intended to refer to only a singular entity but also plural entities and also includes the general class of which a specific example may be used for illustration. The terminology herein is used to describe specific aspects of the invention, but their usage does not delimit the invention, except as outlined in the claims.

The term "microchannels" as used herein refer to pathways through a medium, *e.g.,* silicon, that allow for movement of liquids and gasses. Microchannels can therefore connect other components, *i.e.,* keep components "in liquid communication." While it is not intended that the present application be limited by precise dimensions of the channels, illustrative ranges for channels are as follows: the channels can be between 0.35 and 100 $\mu$m in depth (*e.g.,* 50 $\mu$m) and

between 50 and 1000 $\mu$m in width (*e.g.,* 400 $\mu$m). The channel length can be between 4 mm and 100 mm (*e.g.*, about 27 mm).

The term "microfabricated", "micromachined", and/or "micromanufactured" as used herein means to build, construct, assemble or create a device on a small scale, *e.g.*, where components have micron size dimensions or microscale.

The term "polymer" as used herein refers to a substance formed from two or more molecules of the same substance. Example polymers are gels, crosslinked gels, and polyacrylamide gels. Polymers may also be linear polymers in which the molecules align predominately in chains parallel or nearly parallel to each other. In a non-linear polymer, the parallel alignment of molecules is not required.

The term "lensless or mobile imaging system" as used herein refers to an optical configuration that collects an image based upon electronic signals as opposed to light waves. For example, a lensless image may be formed by excitation of a charged coupled device (CCD) sensor by emissions from a light emitting diode.

The term "charge-coupled device (CCD)" as used herein refers to a device for the movement of electrical charge, usually from within the device to an area where the charge can be manipulated, for example, a conversion into a digital value. A CCD provides digital imaging when using a CCD image sensor where pixels are represented by *p*-doped MOS capacitors.

The term "symptom" as used herein refers to any subjective or objective evidence of disease or physical disturbance observed by the patient. For example, subjective evidence is usually based upon patient self-reporting and may include, but is not limited to, pain, headache, visual disturbances, nausea, and/or vomiting. Alternatively, objective evidence is usually a result of medical testing including, but not limited to, body temperature, complete blood count, lipid panels, thyroid panels, blood pressure, heart rate, electrocardiogram, tissue, and/or body imaging scans.

The term "disease" or "medical condition", as used herein, refers to any impairment of the normal state of the living animal or plant body or one of its parts that interrupts or modifies the performance of the vital functions. Typically manifested by distinguishing signs and symptoms, it is usually a response to: i) environmental factors (as malnutrition, industrial hazards or climate); ii) specific infective agents (as worms, bacteria or viruses); iii) inherent defects of the organism (as genetic anomalies); and/or iv) combinations of these factors.

The term "patient" or "subject" as used herein is a human or animal and need not be hospitalized. For example, out-patients, persons in nursing homes are "patients." A patient may comprise any age of a human or non-human animal and therefore includes both adult and juveniles, *i.e.,* children. It is not intended that the term "patient" connote a need for medical treatment and, thus, a patient may voluntarily or involuntarily be part of experimentation whether clinical or in support of basic science studies.

The term "derived from" as used herein refers to the source of a compound or sample. In one respect, a compound or sample may be derived from an organism or particular species.

The term "antibody" as used herein refers to immunoglobulin evoked in animals by an immunogen (antigen). It is desired that the antibody demonstrates specificity to epitopes contained in the immunogen.

The terms "specific binding" or "specifically binding" as used herein when used in reference to the interaction of an antibody and a protein or peptide means that the interaction is dependent upon the presence of a particular structure, *e.g.*, an antigenic determinant or epitope, on a protein. In other words, an antibody is recognizing and binding to a specific protein structure rather than to proteins in general. For example, if an antibody is specific for epitope "A", the presence of a protein containing epitope A (or free, unlabeled A) in a reaction containing labeled "A" and the antibody will reduce the amount of labeled A bound to the antibody.

The term "functionalized" or "chemically functionalized" as used herein means the addition of functional groups onto the surface of a material by chemical reaction(s). As will be readily appreciated by a person skilled in the art, functionalization can be employed for surface modification of materials in order to achieve desired surface properties, such as biocompatibility, wettability, and so on. Similarly, the term "biofunctionalization," "biofunctionalized," or the like, as used herein, means modification of the surface of a material to have desired biological function, which will he readily appreciated by a person of skill in the related art, such as bioengineering.

The term "sample" as used herein is used in its broadest sense and includes environmental and biological samples. Environmental samples include material from the environment such as soil and water. Biological samples may be animal, including, human, fluid, *e.g.,* blood, plasma, and serum; solid, *e.g.,* stool; tissue; liquid foods, *e.g.,* milk; and solid foods, *e.g.*, vegetables. A biological sample may comprise a cell, tissue extract, body fluid, chromosomes or extrachromosomal elements isolated from a cell, genomic DNA (in solution or bound to a solid support such as for Southern blot analysis), RNA (in solution or bound to a solid support such as for Northern blot analysis), cDNA (in solution or bound to a solid support) and the like.

The terms "bioaffinity ligand", "binding component", "molecule of interest", "agent of interest", "ligand" or "receptor" as used herein may be any of a large number of different molecules, biological cells or aggregates, and the terms are used interchangeably. Each binding component may be immobilized on a solid substrate and binds to an analyte being detected. Proteins, polypeptides, peptides, nucleic acids (nucleotides, oligonucleotides and polynucleotides), antibodies, ligands, saccharides, polysaccharides, microorganisms such as bacteria, fungi, and viruses, receptors, antibiotics, test compounds (particularly those produced by combinatorial chemistry), plant and animal cells organdies or fractions of each and other biological entities may each be a binding component. Each, in turn, also may be considered as analytes if same bind to a binding component on a microfluidic biochip.

The terms "bind" or "adhere" as used herein include any physical attachment or close association, which may be permanent or temporary. Generally, an interaction of hydrogen bonding, hydrophobic forces, van der Waals forces, covalent and ionic bonding etc., facilitates physical attachment between the molecule of interest and the analyte being measuring. The "binding" interaction may be brief as in the situation where binding causes a chemical reaction to occur. That is typical when the binding component is an enzyme and the analyte is a substrate for the enzyme. Reactions resulting from contact between the binding agent and the analyte are also within the definition of binding for the purposes of this application.

The term, "substrate" as used herein refers to surfaces as well as solid phases which may include a microchannel. In some cases, the substrate is solid and may comprise PDMS. A substrate may also include components including, but not limited to, glass, silicon, quartz, plastic or any other composition capable of supporting photolithography.

The term, "photolithography", "optical lithography" or "UV lithography" as used herein refers to a process used in microfabrication to pattern parts of a thin film or the bulk of a substrate. It uses light to transfer a geometric pattern from a photomask to a light-sensitive chemical "photoresist" or simply "resist," on the substrate. A series of chemical treatments then either engraves the exposure pattern into or enables deposition of a new material in the desired pattern upon, the material underneath the photo resist. For example, in complex integrated circuits, a modern CMOS wafer will go through the photolithographic cycle up to 50 times.

Microfluidic Biochip

Examples described herein relate to a microfluidic biochip device and system and an analytic method for simultaneous interrogation of cell deformability and adhesion to a microvasculature-mimicking surface at a single cell level. The microfluidic biochip device or system quantifies membrane, cellular, and adhesive properties of red blood cells (RBCs) of a subject. This can be used, for example, to monitor disease severity, upcoming pain crisis, treatment response, and treatment effectiveness in a clinically meaningful way. In one example, the cells are derived from the whole blood of patients being screened and/or monitored for SCD progression.

The microfluidic device includes a housing and at least one microchannel that defines at least one cell adhesion region. Each microchannel can have a constant width or a width that continuously changes in a direction of the fluid sample flow through the microchannel. The at least one cell adhesion region is functionalized with at least one capturing agent that captures or adheres a cell of interest to a surface of the microchannel when a sample fluid containing cells is passed through the at least one microchannel. If the housing includes multiple microchannels, each microchannel can be functionalized with a different capturing agent to adhere different cells of interest thereto. In any case, each microchannel is configured to receive and provide cell adhesion analysis of a microvolume fluid sample.

The capturing agent is a bioaffinity ligand, such as fibronectin (FN), laminin (LN), thrombospondin (TSP), selectin, von Willebrand Factor (vWF) or a C146 antibody. FN is an adhesive glycoprotein found in the extracellular matrix and in plasma as a linker molecule. FN has been shown to promote abnormal RBC and WBC (particularly neutrophil) adhesion to the endothelial wall in SCD. In contrast with FN, LN is embedded inside the sub-endothelial layer and has been implicated in sickle RBC adhesion to the vascular wall. Under normal conditions, LN is not in contact with flowing blood. In SCD, however, the endothelial lining cracks, thereby allowing sub-endothelial matrix proteins - including LN - to interact with circulating cells. Regardless of the bioaffinity ligand chosen, an imaging system of the microfluidic device measures the quantity of cells adhered to the at least one bioaffinity ligand within each microchannel when the fluid sample is passed therethrough.

In another example, the microfluidic device further includes a micro-gas exchanger fluidly connected to the at least one microchannel for varying the oxygen content of the fluid sample containing the cells. The micro-gas exchanger can include a gas-permeable inner tube inserted within a gas-impermeable outer tube. Fluid, such as blood or synovial fluid, containing the cells of interest is delivered through the inner tube such that the fluid exchanges gases through the permeable tubing wall with a control gas, *e.g.,* 5% $CO_2$ and 95% $N_2$, between the tubes. The oxygen content of the fluid exiting the micro-gas exchanger is controlled to thereby control the oxygen content of the fluid delivered to the microchannel.

By way of example, the micro-gas exchanger can include concentric inner and outer tubes. The inner tube has a gas-permeable wall defining a central passage extending the entire length of the inner tube. The outer tube has a gas impermeable wall defining a central passage extending the entire length of the outer tube. An annular space is formed between the tubes. The central passage receives the fluid sample and is in fluid communication with one or more inlet ports of the microfluidic device. Each inlet port can be fluidly connected to the same micro-gas exchanger or a different micro-gas exchanger to specifically tailor the fluid delivered to each microchannel. An outlet tube is connected to each outlet port of the micro-gas exchanger. A controlled gas flow takes place in the annular space between the concentric tubes and fluid flows inside the inner tube. When the fluid sample is blood, deoxygenation of the sample occurs due to gas diffusion (5% $CO_2$ and 95% $N_2$) through the inner gas-permeable wall.

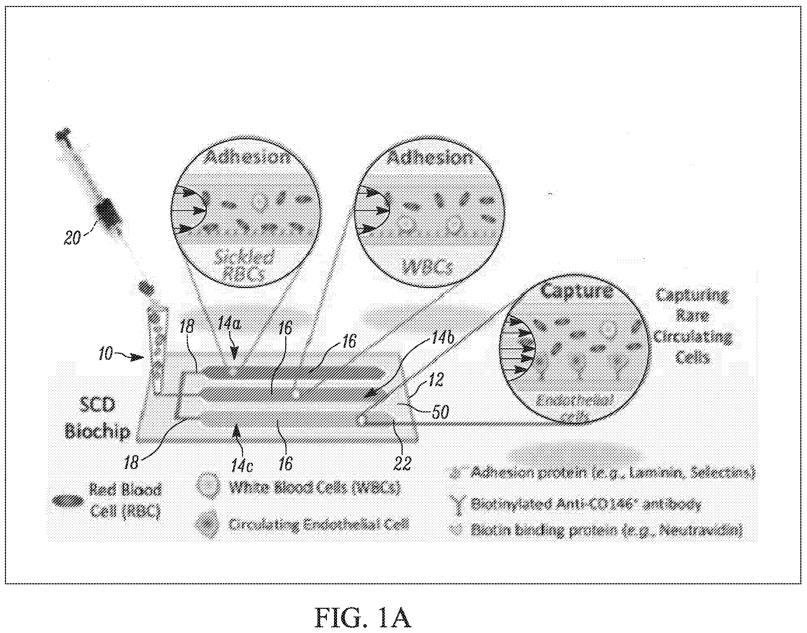

Fig. 1A illustrates an example microfluidic biochip device 10 for measuring cellular, membrane, and adhesive interactions. The microfluidic device 10 includes a housing 12 defining at least one channel 14 - here a plurality of channels 14a-14c - that each includes a cell adhesion or adherence region 16. Each channel 14a-14c is fluidly connected to an inlet port 18 at one end and an outlet port (not shown) at another end. Although Fig. 1A depicts three channels 14a-14c, the microfluidic device 10 can include more or fewer than three channels. The size of each channel 14a-14c should be large enough to prevent clogging of the channels when a fluid sample 20, *e.g.,* blood, synovial fluid or a solution containing cells to be analyzed, is passed through the channels.

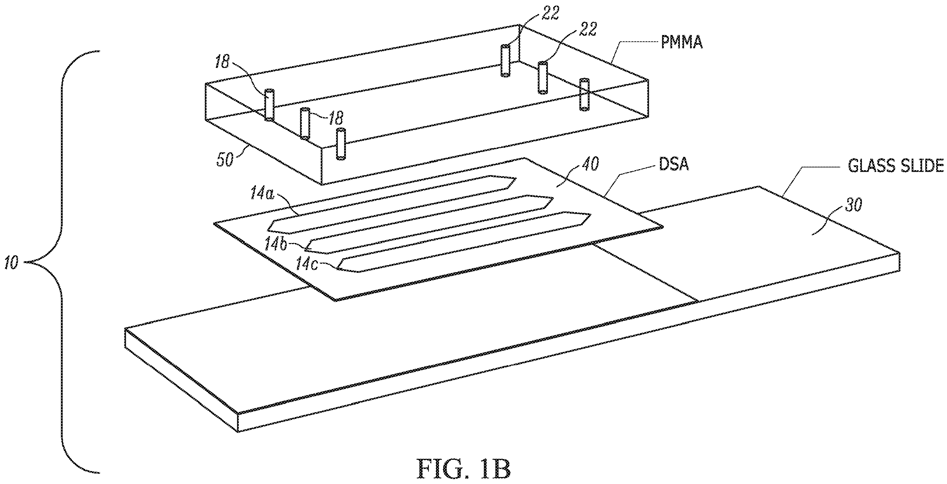

Referring to Fig. 1B, the microfluidic biochip 10 can include a multilayer structure formed of a base layer 30, an intermediate layer 40, and a cover layer 50. The channels 14a-14c are formed in the intermediate layer 40. A first end of each channel 14a-14c is aligned with a corresponding inlet port 18. A second end of each channel is aligned with a corresponding outlet port 22. This creates a flow channel from an inlet port 18 to the corresponding outlet port 22 *via* the channel 14. The channels 14a-14c can also extend slightly beyond their respective inlet 18 and outlet ports 22 (not shown). The channels 14a-14c are sized to accept volumes, *e.g.*, $\mu$L or mL, of the sample 20 containing cells to be adhered or captured in the respective regions 16 (See Fig. 1A). The channels 14a-14c may be further sized and shaped to affect adherence or capturing of the cells from the sample 20.

The base layer 30 provides structural support to the cell adherence region 16 and is formed of a sufficiently rigid material, such as poly(methyl methacrylate) (PMMA) or glass. The base layer 30 can have a suitable thickness, for example of about 0.1 mm to about 2 mm, or about 1.6 mm, determined by manufacturing and assembly restrictions.

The cover layer 50 contains the inlet ports 18 and outlet ports 22 used to feed the sample 20 in/out of the channel 14. The cover layer 50 thickness can be about 1 mm to about 10 mm, for example, about 3.6 mm, and is determined by the integration and assembly requirements. The inlet and outlet port 18, 22 diameters can be about 0.3 mm to about 3 mm, for example about 1mm. The lower size limit is determined by the manufacturing restrictions. The upper size limit is determined by the desired flow conditions of sample 20 through the channel 14. In another example (not shown), a laser cutter can be used to cut a larger piece of PMMA into a desired size for the microfluidic device 10 and to cut holes for the inlet ports 18 and the outlet ports 22.

The intermediate layer 40 can be formed of a material that adheres to both the base layer 30 and the cover layer 50. Each channel 14 can be formed, for example, by laser cutting polygons, such as rectangular sections, in the intermediate layer 40, which can itself be laser cut to the desired size, *e.g.,* the size of the base layer 30. The height or depth of each channel 14 can be determined by the thickness of the intermediate layer 40, which is discussed in greater detail below.

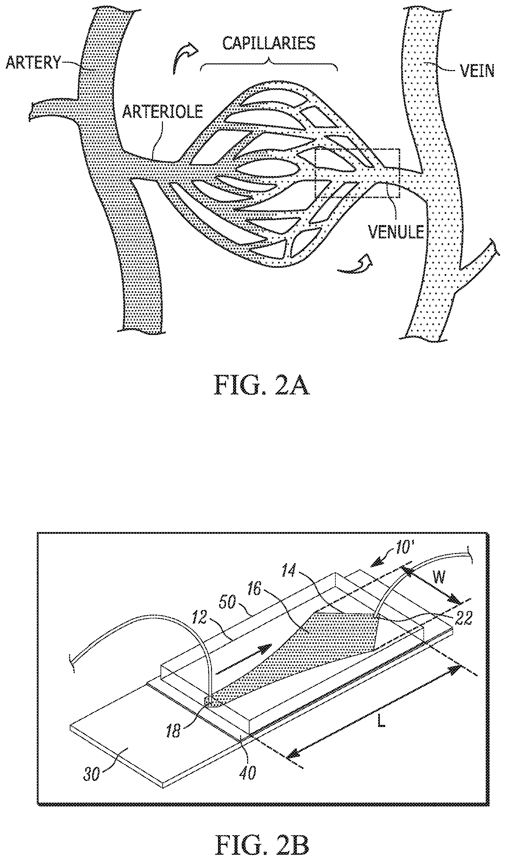

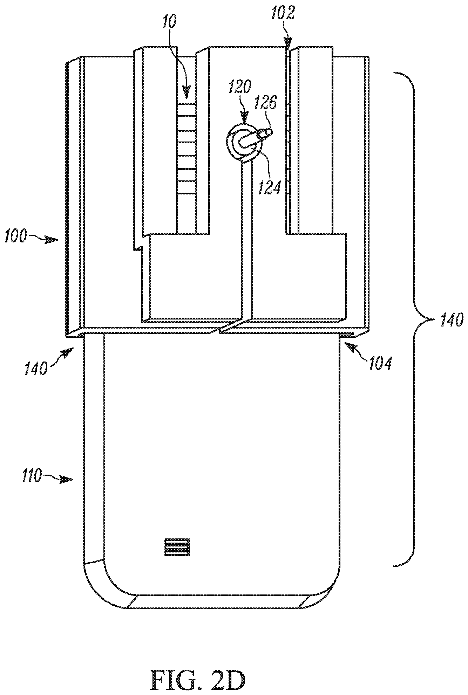

The intermediate layer 40 is adhered to the base layer 30 after each channel 14 is cut in the intermediate layer. The cover layer 50, which can have the same lateral dimensions as the base layer 30 and the intermediate layer 40, can be adhered onto the exposed side of the intermediate layer 40, thereby enclosing each channel 14. In the examples depicted in Figs. 1A and 2B, the microfluidic device 10 is oriented such that the cover layer 50 is on top. Alternatively, the microfluidic device 10 can be oriented such that the cover layer 50 is on the bottom (not shown).

Fig. 2B illustrates another example microfluidic biochip device 10'. The microfluidic device 10' includes a housing 12 defining a single channel 14 having cell adhesion or adherence regions 16. The channel 14 is fluidly connected to an inlet port 18 at one end and an outlet port 22 at another end. Although Fig. 2B depicts only one channel 14, the microfluidic device 10' can include multiple channels. The channel 14 receives a sample 20 from a patient. The channel 14 can have a length L of about 45-50 mm, a depth of about 50-57 $\mu$m ($\pm$ 1 $\mu$m), and a width W that varies along the length L from about 4 mm (at the inlet end) to about 16 mm (closer to the outlet end).

The geometry of the channel 14 in the microfluidic device 10' is such that, when fluid is introduced into the channel 14, shear stress in the fluid flow along the longitudinal axis of the channel varies linearly along the channel length L. In one example, the shape of the channel 14 is such that the shear stress in the fluid flow along the axis of the chamber decreases linearly along the channel length L. To this end, the channel 14 can have a tapered, triangular, trapezoidal and/or diamond-shaped configuration. This allows for cell adhesion analysis over a range of shear stresses in a single experiment. Consequently, the configuration of the channel 14 allows for the study of the effect of flow conditions on the attachment of cells of interest, *e.g.*, RBCs, to the surface of the channel defining the cell adhesion region 16.

In some examples, the microfluidic device 10, 10' geometry and dimensions are determined to accommodate a uniform, laminar flow condition for the fluid sample 20, which determines capture efficiency and flow rate. In such examples, the channel 14 width W can vary from about 1 mm to about 15 mm. The minimum width W is determined by the diameters of the inlet and outlet port 18, 22. The upper limit width W is determined by the flow characteristics of fluid sample 20 in a confined channel 14. The channel 14 length L can be about 4 mm to about 100 mm. The lower channel 14 length L dimension is determined by the flow characteristics of the fluid sample 20 in a confined channel. The upper limit length L is determined by cell capture efficiency. The channel 14 height/depth can be about 10 $\mu$m to about 500 $\mu$m, for example,

about 50 μm, which is determined by fluid mechanics laws and constraints and flow characteristics of the fluid sample 20 in a confined channel. In any case, the channel(s) 14 in either device 10, 10' can be a microchannel sized to receive and capable of testing a fluid sample 20 on the μL scale in volume.

In each microfluidic device 10, 10' each cell adherence regions 16 can include a surface on which is provided a layer or coating of the at least one capturing agent. The at least one capturing agent comprises a bioaffinity ligand. The same or different bioaffinity ligand 16 can be provided in each channel 14. The bioaffinity ligand can include, for example, at least one of FN, LN, TSP, selectins, von Willebrand Factor or a C146 antibody. The bioaffinity ligand can be adhered to, functionalized or chemically functionalized to the cell adhesion region 16 of each channel 14. The bioaffinity ligands may be functionalized to the cell adhesion region 16 covalently or non-covalently. A linker can be used to provide covalent attachment of a bioaffinity ligand to the cell adhesion region 16. The linker can be a linker that can be used to link a variety of entities.

In some examples, the linker may be a homo-bifunctional linker or a hetero-bifunctional linker, depending upon the nature of the molecules to be conjugated. Homo-bifunctional linkers have two identical reactive groups. Hetero-bifunctional linkers have two different reactive groups. Various types of commercially available linkers are reactive with one or more of the following groups: primary amines, secondary amines, sulphydryls, carboxyls, carbonyls and carbohydrates. Examples of amine-specific linkers are bis(sulfosuccinimidyl) suberate, bis[2-(succinimidooxycarbonyloxy)ethyl]sulfone, disuccinimidyl suberate, disuccinimidyl tartarate, dimethyl adipimate 2HCl, dimethyl pimelimidate 2HCl, dimethyl suberimidate HCl, ethylene glycolbis-[succinimidyl-[succinate]], dithiolbis(succinimidyl propionate), and 3,3'-dithiobis(sulfosuccinimidylpropionate). Linkers reactive with sulfhydryl groups include bismaleimidohexane, 1,4-di-[3'-(2'-pyridyldithio)-propionamido)]butane, 1-[p-azidosalicylamido]-4-[iodoacetamido]butane, and N-[4-(p-azidosalicylamido)-butyl]-3'-[2'-pyridyldithio]propionamide. Linkers preferentially reactive with carbohydrates include azidobenzoyl hydrazine. Linkers preferentially reactive with carboxyl groups include 4-[p-azidosalicylamido]butylamine.

Heterobifunctional linkers that react with amines and sulfhydryls include N-succinimidyl-3-[2-pyridyldithio] propionate, succinimidyl[4-iodoacetyl]aminobenzoate, succinimidyl 4-[N-maleimidomethyl]cyclohexane-1-carboxylate, m-maleimidobenzoyl-N-hydroxysuccinimide ester, sulfosuccinimidyl 6-[3-[2-pyridyldithio]propionamido]hexanoate, and sulfosuccinimidyl 4-[N-maleimidomethyl]cyclohexane-1-carboxylate. Heterobifunctional linkers that react with carboxyl and amine groups include 1-ethyl-3-[3-dimethylaminopropyl]-carbodiimide hydrochloride. Heterobifunctional linkers that react with carbohydrates and sulfhydryls include 4-[N-maleimidomethyl]-cyclohexane-1-carboxylhydrazide HCl, 4-(4-N-maleimidophenyl)-butyric acid hydrazide.2HCl, and 3-[2-pyridyldithio]propionyl hydrazide.

Alternatively, the bioaffinity ligands may be non-covalently coated onto the cell adhesion region 16. Non-covalent deposition of the bioaffinity ligand to the cell adhesion region 16 may involve the use of a polymer matrix. The polymer may be naturally occurring or non-naturally occurring and may be of any type including but not limited to nucleic acid, e.g., DNA, RNA, PNA, LNA, and the like or mimics, derivatives or combinations thereof, amino acid, e.g., peptides, proteins (native or denatured), and the like or mimics, derivatives or combinations thereof, lipids, polysaccharides, and functionalized block copolymers. The bioaffinity ligand may be adsorbed onto and/or entrapped within the polymer matrix. Alternatively, the bioaffinity ligand may be covalently conjugated or crosslinked to the polymer, e.g., it may be "grafted" onto a functionalized polymer.

An example of a suitable peptide polymer is poly-lysine, e.g., poly-L-lysine. Examples of other polymers include block copolymers that comprise polyethylene glycol (PEG), polyamides, polycarbonates, polyalkylenes, polyalkylene glycols, polyalkylene oxides, polyalkylene terepthalates, polyvinyl alcohols, polyvinyl ethers, polyvinyl esters, polyvinyl halides, polyvinylpyrrolidone, polyglycolides, polysiloxanes, polyurethanes, alkyl cellulose, hydroxyalkyl celluloses, cellulose ethers, cellulose esters, nitrocelluloses, polymers of acrylic and methacrylic esters, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), polyvinyl acetate, polyvinyl chloride, polystyrene, polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate), poly(lactide-glycolide), copolyoxalates, polycaprolactones, polyesteramides, polyorthoesters, polyhydroxybutyric acid, polyanhydrides, poly(styrene-b-isobutylene-b-styrene) (SIBS) block copolymer, ethylene vinyl acetate, poly(meth)acrylic acid, polymers of lactic acid and glycolic acid, polyanhydrides, poly(ortho)esters, polyurethanes, poly(butic acid), poly(valeric acid), and poly(lactide-cocaprolactone), and natural polymers such as alginate and other polysaccharides including dextran and cellulose, collagen, albumin and other hydrophilic proteins, zein and other prolamines and hydrophobic proteins, copolymers and mixtures thereof, and chemical derivatives thereof including substitutions and/or additions of chemical groups, for example, alkyl, alkylene, hydroxylations, oxidations, and other modifications routinely made by those skilled in the art.

In some examples, each channel 14 can include multiple, separate cell adhesion regions 16 functionalized with at least one bioaffinity ligand. At least two or at least three of the channels 14 can include different bioaffinity ligands. In other examples, the plurality of channels 14 can include the same bioaffinity ligands.

In still other examples, at least one channel 14 can include at least two different bioaffinity ligands functionalized on the cell adhesion region 16. The different bioffinity ligands can be located at different positions within the cell adhesion region 16 of each channel 14. For example, at least one of LN, selectin, von Willebrand Factor, thrombospondin, and the C146 antibody can be localized at different positions along the length L of the at least one channel 14.

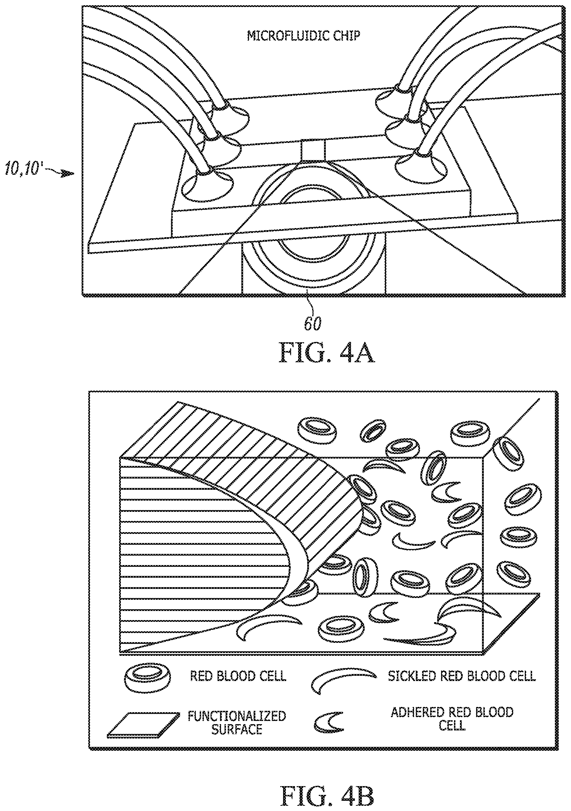

In some examples, a fluid sample 20, which includes at least one blood cell from a subject is introduced into each channel 14. The capturing agent or bioaffinity ligand can bind cells of interest in the fluid sample to a surface or wall(s) of the microchannel along the cell adhesion region 16. The quantity of blood cells bound to the microchannel walls by the capturing agent can be imaged using an imaging system 60 (see Fig. 4A). The imaging system 60 can determine, for example, the aspect ratio (AR) of the blood cells as well as quantify membrane, cellular and adhesive properties of the blood cells to monitor disease severity, upcoming pain crisis, treatment response, and treatment effectiveness of a subject in a clinically meaningful way.

In some examples, the imaging system 60 can be a lens-based imaging system or a lensless/mobile imaging system. For example, the lensless imaging system 60 can be a CCD sensor and a light emitting diode. In some examples, a mobile imaging and quantification algorithm can be integrated into or with the microfluidic device 10, 10'. The algorithm can achieve reliable and repeatable test results for data collected in all resource settings of the microfluidic device 10, 10'.

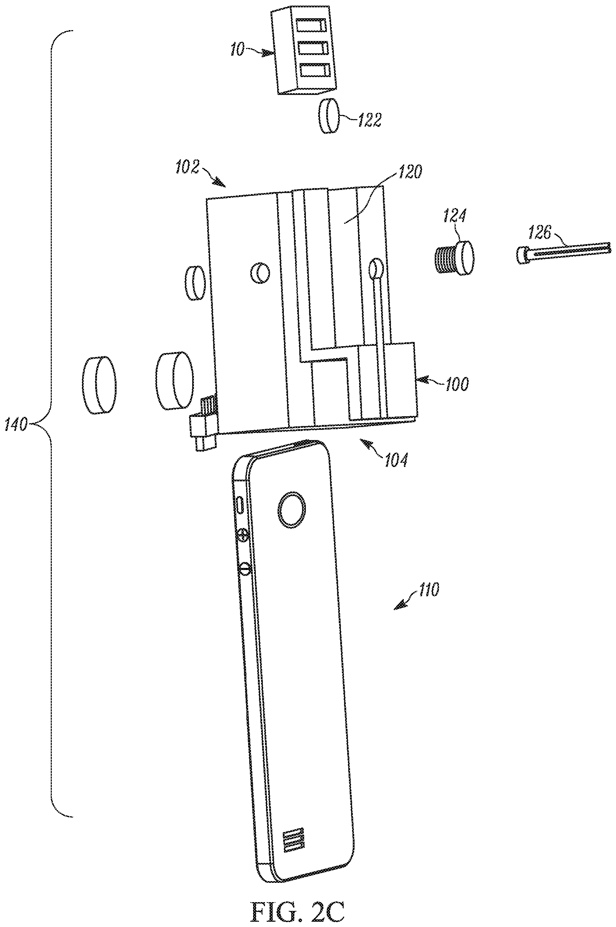

In other examples, the microfluidic device 10, 10' can be configured to cooperate with a cellular phone having imaging capabilities. In such a case, the cellular phone can be provided with or capable of obtaining image analysis algorithms/software, *e.g.*, via an online application. Referring to Figs. 2C-2D, in such a construction an optical attachment member 100 connects the microfluidic device 10 to a cellular phone 110. The optical attachment member 100 includes a first slot 102 for receiving the microfluidic device 10 and a second slot 104 for receiving the cellular phone 110.

An optical tunnel 120 extends through the optical attachment member 100 to the first slot 102. The optical tunnel 120 receives a lens 122, a diffuser (not shown), an LED holder 124, and an LED 126. In use, the LED 126 provides light to the optical tunnel 120. The light passes through the diffuser, which polarizes the light and back-illuminates the blood sample 20 within the microfluidic device 10. Light and the silhouettes of adhered RBCs are collected on the lens 122 within the optical tunnel 120 and sent to the lens and CMOS sensor of the cellular phone 110 camera.

Images can be recreated by the cellular phone 110 camera software and loaded into a custom phone application that identifies adhered RBCs, quantifies the number of adhered RBCs in the image, and displays the results. The optical attachment member 100 can include, for example, a battery for powering the LED 126 and other components of the optical attachment member, if necessary. In any case, the imaging system 140 in this example includes the optical attachment member 100 for connecting the microfluidic device 10 and the cellular phone 110 as well as the application on the cellular phone for performing the cytological analysis.

The cells of interest are red blood cells obtained from the subject and the imaging system 60, 140 can quantify the adhered cells in each respective channel 14 to monitor the health of a subject from which the cells are obtained. In other examples, the imaging system 60, 140 can quantify the adhered cells in each respective channel 14 to monitor the progression of a disease, such as SCD, of a subject from which the cells are obtained. In still other examples, the imaging system 60, 140 can quantify the adhered cells in each channel 14 to measure the efficacy of a therapeutic treatment administered to a subject from which the cells are obtained.

It can be expected that a microfluidic device 10, 10' platform disclosed herein is applicable to the study of single cell heterogeneity of adherent cells within subjects in larger clinically diverse populations and may provide important insights into complex disease phenotypes other than SCD. For example, abnormal RBC adhesion to microvascular surfaces has previously been implicated in other multi-system diseases, such as β-thalassemia, diabetes mellitus, hereditary spherocytosis, polycythemia vera, and malaria.

By way of example, referring back to Fig. 2B, sickled RBC adherence to blood vessel walls has been shown to take place in post-capillary venules. To this end, this application contemplates a microfluidic SCD biochip including at least one microchannel having a width W of approximately 60 $\mu$m, a depth of about 50 $\mu$m, and fluid flow velocities within a range of approximately 1-13 mm/sec - similar to that reported for post-capillary venules. No study, however, to date has analyzed HbS-containing RBC adhesion and deformability using whole blood at the microvasculature scale of ~ 60 $\mu$m. That said, the data presented herein demonstrates heterogeneity in HbS-containing RBC adhesion and deformability measured at a single cell level in SCD blood samples examined in microfluidic channels mimicking a microvasculature.

In one example, the microfluidic biochip 10, 10' can be used in an SCD testing method utilizing pathophysiologic correlates, including but not limited to, analyses of adhesion and membrane properties in HbSS and HbSC, at baseline and during vaso-occlusive crises, with treatment, and in the presence of end-organ damage. The SCD testing method described herein can be completed in less than ten minutes. In some examples, the SCD testing method provides a highly specific analyses of CMA properties in RBCs, WBCs, circulating hematopoietic precursor cells and circulating endothelial cells. In one example, the SCD testing method is performed using a portable, high efficiency, microfluidic biochip and a miniscule blood sample (<15 µL). The SCD testing method can provide a sophisticated and clinically relevant strategy with which patient blood samples may be serially examined for cellular/membrane/adhesive properties during SCD disease progression.

In some examples, the microfluidic device 10, 10' can accurately quantitate cellular interactions and membrane properties using a single drop of blood. The biochip and method may validate insights about mechanisms of disease in SCD and may reveal correlations between disease heterogeneity and acute and/or chronic SCD complications.

The microfluidic device 10, 10' can also evaluate membrane and cellular abnormalities by interrogating a number of recognized abnormalities in a range of clinical phenotypes. To date, these phenotypes are discussed in various correlative SCD studies ranging between clinical reports, testing results, interventions, and/or chart reviews.

The examples described herein have advantages because existing conventional methods cannot assess longitudinal and large-scale SCD clinical correlations with cellular, membrane, and adhesive properties. To this end, this application contemplates a method for using an SCD biochip for examining cellular properties and interactions. These cellular properties and interactions include, but are not limited to, RBC cellular and adhesive properties, WBC cellular and adhesive properties, circulating endothelial characteristics and hematopoietic precursor cell characteristics. For example, a microfluidic biochip can include a plurality of microchannels that are functionalized with lignin, selectins, avidin and/or biotinylated antibodies to BCAM/LU, CD11b, CD34, and/or CD146. A method is contemplated for correlating SCD biochip function in heterogeneous SCD populations, including but not limited to, HbSS and HbSC over a range of ages, and in those with acute and chronic complications and compared with normal HbAA controls.

A simple test for adhesion would allow exploration of its role in chronic complications in SCD, in addition to during crisis. Selectins may be tested using microfluidic biochips as an adhesive surface, in place of cultured endothelial cells. Endothelial selectins are believed to mediate leukocyte adhesion and rolling on the endothelial surface. For example, in experimental models of SCD, P-selectin is widely expressed on vascular endothelium and endothelial E-selectin is important for vascular occlusion. This application contemplates a microfluidic SCD biochip including at least one microchannel provided with at least one immobilized P-selectin and/or E-selectin adhesion.

It is expected that SCD samples show greater WBC adherence to selectins, compared with HbAA controls. Examination of SCD samples, at baseline and with crisis, may evaluate changes with disease activity. For example, MAC-1, LFA-1, and VLA-4 expression may be measured by FACS on selectin-captured blood WBCs, as compared with unmanipulated WBCs on an SCB microfluidic biochip from the same sample.

It is possible that immobilized selectins may interact with RBCs. If this hinders analysis of WBC interactions, RBCs may be lysed prior to analysis. RBC adherence may be variable between patients and therefore informative, and can be quantified if so. Of note, conventional *in vitro* measures of WBC adherence entail endothelial cell culture, which is avoided by use of a microfluidic biochip system.

The SCD microfluidic biochip can include a CD11b for isolating an activated WBC. The activated WBC can be a monocyte, which has been recognized as major inflammatory mediators of endothelial activation in SCD. The microfluidic biochip can also include a microchannel coated with CD146 antibodies to quantitate mature circulating endothelial cells.

The microfluidic SCD biochip can also include vWF or thrombomodulin to quantitate CECs by image analysis. Although isolation of rare CECs is technically challenging, these cells have been identified by FACS in unmanipulated blood using a CD146 marker. Biophysical probing of individual cells in a microfluidic biochip described herein necessitates accurate control, measurement, and estimation of flow velocities in close vicinity to the adhered cells. These measured and estimated values allow theoretical calculations about flow in the microfluidic channels. Validation of the accuracy of these predicted flow velocities in comparison with measured local flow velocities may be performed through particle image tracking around the adhered cells and using the non-adhered flowing cells as free flowing particles with which to measure the local flow velocities.

### EXAMPLES

### Example 1

This example describes a microfluidic device having a functionalized microchannel that can be used to isolate and analyze RBCs.

*Microfluidic Biochip Fabrication*

The microfluidic device was constructed as shown in Figs. 1A and 1B. PMMA cover layers were prepared by cutting an inlet and outlet port (0.61 mm in diameter and 26 mm apart) using a VersaLASER system (Universal Laser Systems Inc., Scottsdale, AZ). Double sided adhesive (DSA) film (iTapestore, Scotch Plains, NJ) acted as the intermediate layer and was cut to fit the size of the PMMA part. 28x4 mm microchannels 50 $\mu$m deep were formed along the length of the DSA. DSA was then attached to the PMMA cover layer to position the inlet and outlet ports between the DSA film outline. A Gold Seal glass slide acted as the base layer and was assembled with the PMMA-DSA structure to form a biochip having a microfluidic channel.

### Surface Chemistry

GMBS stock solution was prepared by solving 25 mg of GMBS in 0.25 mL DMSO. The stock solution was diluted with ethanol to obtain 0.28% v/v GMBS working solution. FN was diluted with PBS to create a (1:10) FN working solution. BSA solution was prepared by solving 3 mg of lyophilized BSA in 1 mL PBS.

The microchannels were washed with 30 $\mu$L of PBS and ethanol after assembly. Next, 20 $\mu$L of cross-linker agent GMBS working solution was injected into the microchannels twice and incubated for 15 min at room temperature. Following GMBS incubation, the microchannels were washed twice with 30 $\mu$L of ethanol and PBS. Next, 20 $\mu$L of FN solution was injected into the microchannels and incubated for 1.5 hours at room temperature. The surface was then passivated by injecting 30 $\mu$L of BSA solution incubated overnight at 4°C, thereby forming a FN functionalized glass surface. The microchannels were rinsed with PBS before processing blood samples.

### Blood Processing

Discarded, de-identified patient blood samples were obtained from University Hospital's Hematology and Oncology Division under institutional review board (IRB) approval. Blood samples were collected into EDTA-containing purple cap Vacutainer tubes. Before being used in experiments, the blood samples were aliquoted into sealed microtubes and sealed syringes to minimize exposure to ambient air. Blood flow through the microchannels and following FCSB flow steps were applied using New Era NE-300 syringe pump system (Farmingdale, NY). Blood samples were kept sealed and upright in 1 mL disposable syringes before and during injection into the microchannels. The blood was then introduced into the microchannels at 28.5 $\mu$L/min until the channel was filled. 15 $\mu$L of blood sample was then injected at a flow rate of 2.85 $\mu$L/min. Next, the syringe was changed and 120 $\mu$L of FCSB at a flow rate of 10 $\mu$L/min was introduced into the microchannel to remove the non-adhered cells.

### Microfluidic Channel Visualization and Image Processing

A fluorescent microscopy camera (EXi Blue EXI-BLU-R-F-M-14-C) and an Olympus IX83 inverted, fluorescent motorized microscope with Olympus Cell Sense live-cell imaging and analysis software were used to obtain real-time microscopic recordings in this study. Olympus (20x/0.45 ph2 and 40x/0.75 ph3) long working distance objective lenses were utilized for phase contrast imaging of single RBCs adhered in the microchannels (see Fig. 4A). During real-time microscope imaging and high resolution video recording at 7 fps rate, controlled fluid flow with stepwise increments was applied until RBC detachment from the microchannel surface was observed. Videos were converted to single frame images for further processing and analysis. The cell dimensions were analyzed by using Adobe Photoshop software (San Jose, CA).

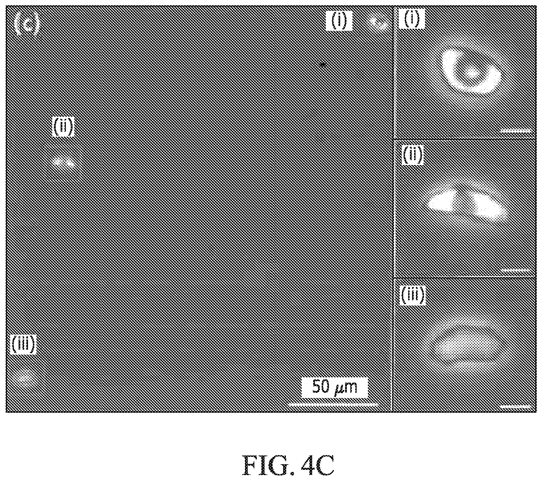

Fig. 4B is a diagrammatic depiction of flowing and adhered RBCs on a FN functionalized surface in the presence of 3D laminar flow velocity profile in the microfluidic biochip. The resulting data showed an adhesion of a morphologically heterogeneous RBC population in blood samples from subjects with HbS (Fig. 4C). This adhesion was not observed when testing HbA blood samples (data not shown). Adhered RBCs included mildly sickled (Fig. 4C(i)), moderately sickled (Fig. 4C(ii)), and highly sickled (Fig. 4C(iii)) cell morphologies within the same field from a single HbS-containing blood sample. The scale bar represents 5 $\mu$m length

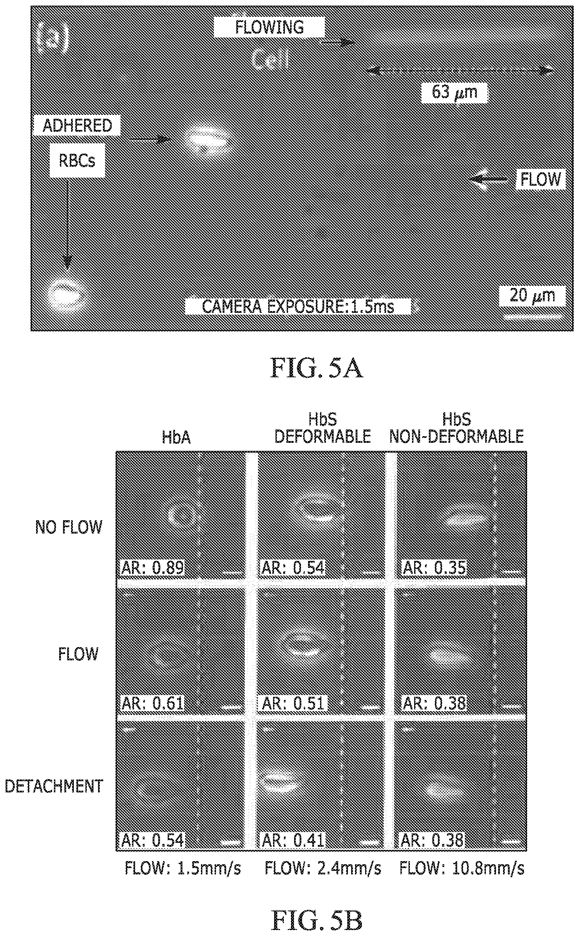

Adhered RBCs were analyzed in terms of biophysical properties in flow in the recorded images (Fig. 5A at 1.5 ms camera exposure time) to determine local flow velocities. Healthy and sickle RBCs at no flow, flow, and detachment conditions are shown in Fig. 5B. Flow velocities that resulted in detachment of RBCs in different experimental groups are noted below each column. White dashed lines denote the initial positions of RBCs at no flow condition. Scale bar represents 5 $\mu$m length.

The aspect ratio (AR) of cells in each frame is provided at the lower left of the images. When free flowing RBCs were imaged at relatively long camera exposure times, they appeared as straight lines due to motion blur (Fig. 5A) - also known as streaking. The streak length was proportional to the flowing particle velocity. Local flow velocities ($V_p$) for flowing cells were determined by using Equation 1:

$$V_p = (L_p - d_p)/t_p \qquad (1)$$

where $L_p$ is the streaking line length, $d_p$ is the average cell size, and $t_p$ is the camera exposure duration. Then, a correlation was analyzed between the measured local flow velocity and the predicted mean flow velocity ($v_m$) determined by the volumetric flow rate ($Q$) and the dimensions of the microchannels (width: $w_c$, and height: $h_c$ ) using Equation 2:

$$v_m = Q/(w_c \ x \ h_c) \hspace{3cm} (2)$$

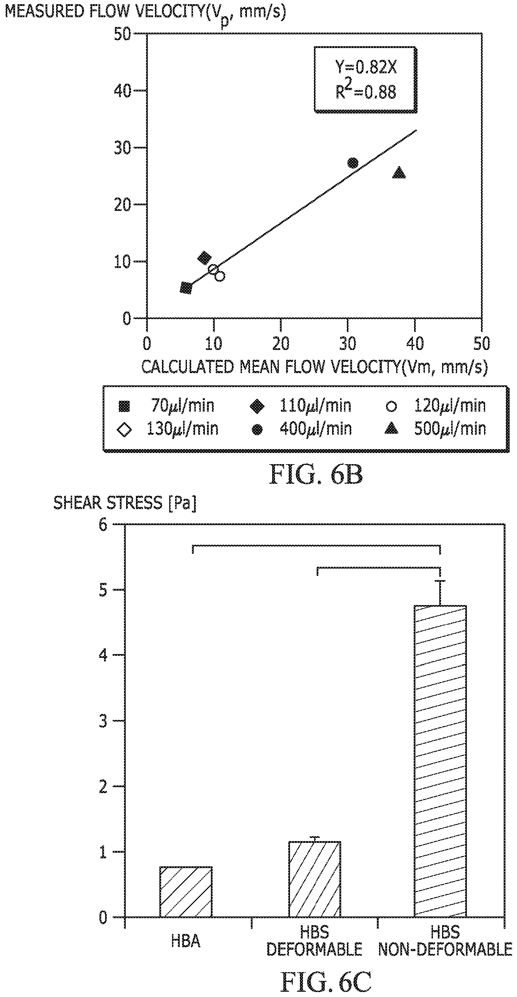

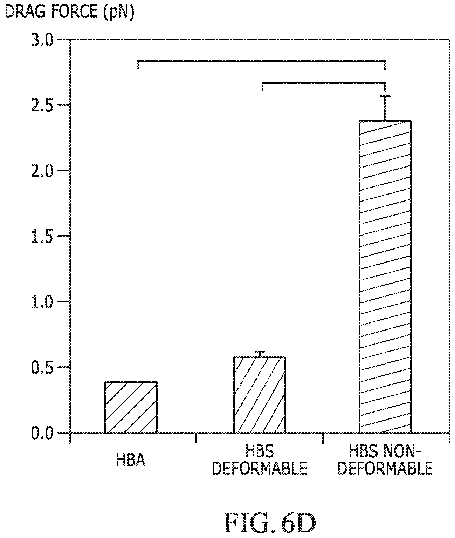

The data show in Fig. 6B that measured local flow velocities display a significant correlation with the predicted flow velocities (Pearson correlation coefficient of 0.94, p<0.001, n=9, R2=0.88). Hence, a mean theoretical flow velocity was used in determining the shear stress (Fig. 6C) and drag force levels (Fig. 6D) for detachment of HbA and HbS-containing RBCs.

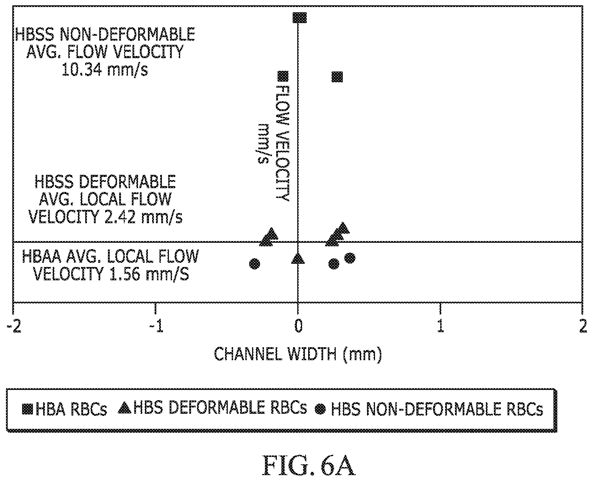

The relative positions of adhered RBCs and flow velocities were also determined for the adhered RBCs. Adhered RBCs were positioned in the mid-region of the microchannels where the flow velocity was uniform across the channel. The data show: i) up to 4.3 times greater flow velocity (Fig. 6A); ii) 4.3 times greater shear stress (Fig. 6C); and iii) 4.1 times greater drag force (Fig. 6D) was required to detach non-deformable HbS-containing RBCs as compared to HbS-containing deformable RBCs (p<0.05, one way ANOVA with Fisher's post-hoc test).

In contrast, HbA-containing RBCs and deformable HbS-containing RBCs did not differ in terms of flow velocity, shear stress, and drag force at detachment (p<0.05, one way ANOVA with Fisher's post-hoc test). These results confirm that HbS-containing RBCs are heterogeneous in terms of their adhesion strength to FN.

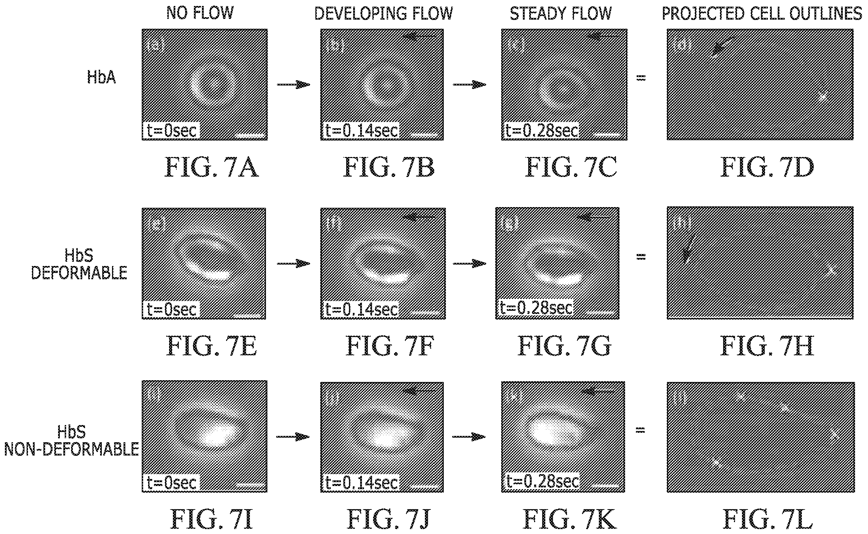

Referring to Figs. 7A-7L, the motion of adhered RBCs at flow initiation was evaluated using consecutive high resolution microscopic images taken over 0.28 seconds in order to analyze sites of adhesion in HbA, HbS deformable, and HbS non-deformable RBCs. Outlines of individual RBCs in three consecutive frames taken over 0.28 seconds were projected to reflect the motion of the cells in response to the initiation of fluid flow for: HbA-containing RBCs (Figs. 7A-7D); HbS-containing deformable RBCs (Figs. 7E-7H); and HbS-containing non-deformable RBCs (Figs. 7I-7L).

The data demonstrated that HbA and HbS deformable cells displayed rotational motion in response to fluid flow direction, indicating a single adhesion pivot point (*see* Figs. 7D and 7H). On the other hand, HbS non-deformable cells did not display a rotational motion in response to fluid flow direction, implying multiple adhesion sites (*see* Fig. 7L). Importantly, these observations suggested that the higher adhesion strength of HbS non-deformable cells may be due to a greater number of adhesion sites.

*Data Analysis*

The flow velocity of the adhered RBCs was calculated using Equations 3 and 4 describing pressure-driven flow in a rectangular channel:

$$u_x(y,z) = \frac{16h^2}{\eta \pi^3}\left(-\frac{dp}{dx}\right)\sum_{n=1,3,5...}^{\infty}(-1)^{(n-1)/2}\left[1-\frac{cosh\left(\frac{n\pi z}{2h}\right)}{cosh\left(\frac{n\pi w}{2h}\right)}\right]\frac{cos\left(\frac{n\pi y}{2h}\right)}{n^3} \hspace{1.5cm} (3)$$

$$Q = \frac{4}{3\eta}wh^3\left(-\frac{dp}{dx}\right)\left[1-\frac{192}{\pi^5}\frac{h}{w}\sum_{n=1,3,5...}^{\infty}\frac{1}{n^5}tanh\left(\frac{n\pi w}{2h}\right)\right] \ , \hspace{1.5cm} (4)$$

where *x, y* and *z* are the principal axes, *h* and *w* are the microchannel height and width, *n* is the fluid viscosity $dp/dx$ is the pressure change along the x axis, and *Q* is the volumetric flow rate *(see* Table 1).

Table 1

|  | Value |
|---|---|
| Measured RBC Width | 4.47-8 (µM) |
| HBA | 6.73-8 (µM) |
| HbS deformable | 4.8-7.84 µm |
| HbS non-deformable | 4.47-7.36 µm |
| RBC Thickness | 2.25 µm |
| Microchannel Heights | 50 µm |

(continued)

|  | Value |
|---|---|
| Microchannel Width | 4 mm |
| Buffer Density | 993 Kg/m$^3$ |
| Buffer Viscosity | 0.001 Pa-d |

The shear stress (*T*) on the adhesion surface is calculated using Equation 5:

$$T = 6nQ/wh^2 \hspace{3cm} (5)$$

Results of these calculations are shown in Fig. 6B.

The drag force applied TO the adhered RBCs was calculated using Equation 6:

$$F_d = \left(\frac{1}{2}\right) \rho u_x^2 C_d A$$

$$(6)$$

where $F_d$ is the drag force, $p$ is the fluid density, $C_d$ is the drag coefficient, and $A$ is the reference area. $C_d$ is calculated as 13.6/$Re$, where $Re$ is the Reynolds number, according to the circular disk parallel to flow assumption of cells at low Reynolds number flow. Reference area $A$ was calculated by using the typical RBC thickness and the measured RBC width at detachment. The results of these calculations are shown in Fig. 6C.

*Statistical Analysis*

The cell AR change with respect to no flow condition was used as a measure of deformability, in which a greater change in cell AR translated to more deformability and hence, less stiffness. Flow velocities were increased in a step-wise manner. Cell deformability for each RBC was assessed at a maximal flow velocity just prior to detachment from the microchannel in a time-lapse experiment. This analysis provided a maximum deformation estimate of an adhered cell.

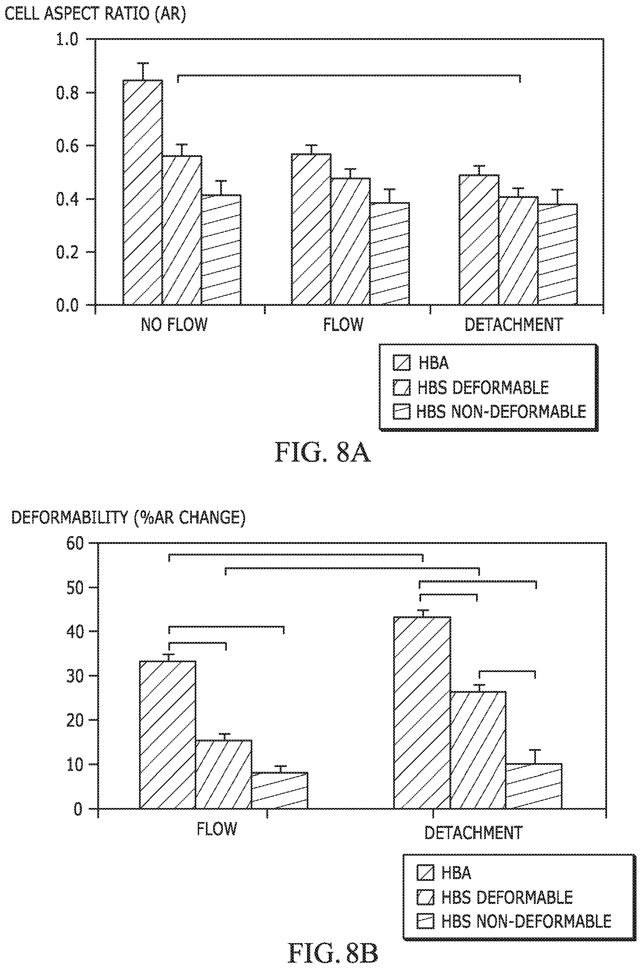

Referring to Figs. 8A-8B, HbA-containing RBCs at no-flow show significantly greater cell aspect ratios, *e.g.*, circularity, than HbS-containing RBCs ($p < 0.05$). The AR of HbA RBCs continuously decreased in the presence of flow ($p < 0.05$), which implied higher deformability. HbS non-deformable cells conserved their initial AR from no flow all the way through detachment ($p < 0.05$).

The HbS deformable RBCs displayed a significantly greater cell AR at no-flow and significantly greater deformability at detachment compared with HbS non-deformable RBCs ($p < 0.05$). The deformability of HbA-containing RBCs was significantly greater than HbS-containing RBCs in all flow conditions ($p < 0.05$). The deformability of both HbA and HbS-containing deformable RBCs was significantly different when measured during flow and when measured at detachment ($p < 0.05$). However, under these same conditions, HbS nondeformable RBCs did not display any significant difference in deformability during this interval ($p < 0.05$). While adhered to the FN-functionalized surface that mimicked features of the normal blood stream in SCD, HbS-containing RBCs were heterogeneous in AR and in deformability (Fig. 8C).

Data obtained in this study was reported as mean $\pm$ standard error of the mean. Flow rate (Fig. 6A), shear stress (Fig. 6B), drag forces (Fig. 6C), cell aspect ratio (Fig. 8A), and aspect ratio change (deformability) (Fig. 8B), were statistically assessed (Minitab 16 software, Minitab Inc., State College, PA) using Analysis of Variance (ANOVA) with Fisher's post hoc test for multiple comparisons (n=3-6 blood samples per group). Statistical significance was set at 95% confidence level for all tests ($p < 0.05$). Error bars in figures represent the standard error of the mean.

*Comparison with LN Functionalized Microchannels*

In another biochip construction, the microfluidic channels were composed of a glass surface functionalized with LN, a PMMA layer, and sandwiched 50 $\mu$m thick DSA tape that defined the height and shape of the microchannels. LN is sub-endothelial and binds to an important RBC surface protein from the immunoglobulin superfamily, BCAM/LU, which is phosphorylated during beta-adrenergic stimulation. The FN and LN-coated microchannels from the respective biochip constructions were each imaged and compared to one another following cell adhesion testing.

The number of adhered RBCs was quantified inside the FN or LN immobilized microfluidic channels. We observed abnormal adhesion of RBCs in blood samples from subjects with SCD. On the other hand, adhesion of RBCs in blood samples from normal subjects was negligible (not shown). High resolution phase-contrast images of an FN and LN coated microchannel surface inside the SCD-Biochip revealed heterogeneous sickle morphologies of adhered RBCs. A range of RBC adhesion was observed in patients with various clinical phenotypes.

*Data Clustering*

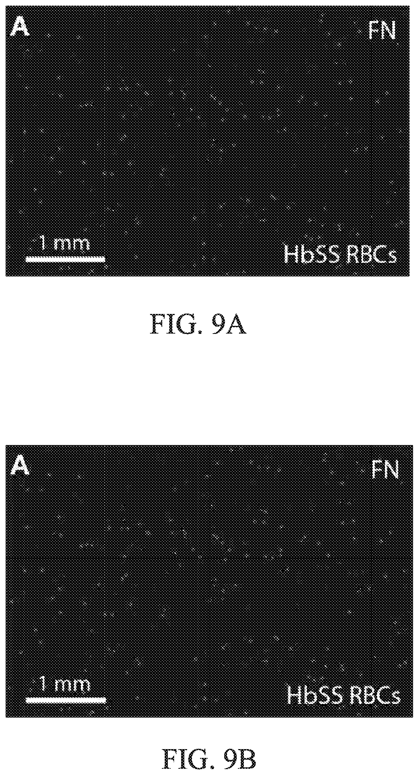

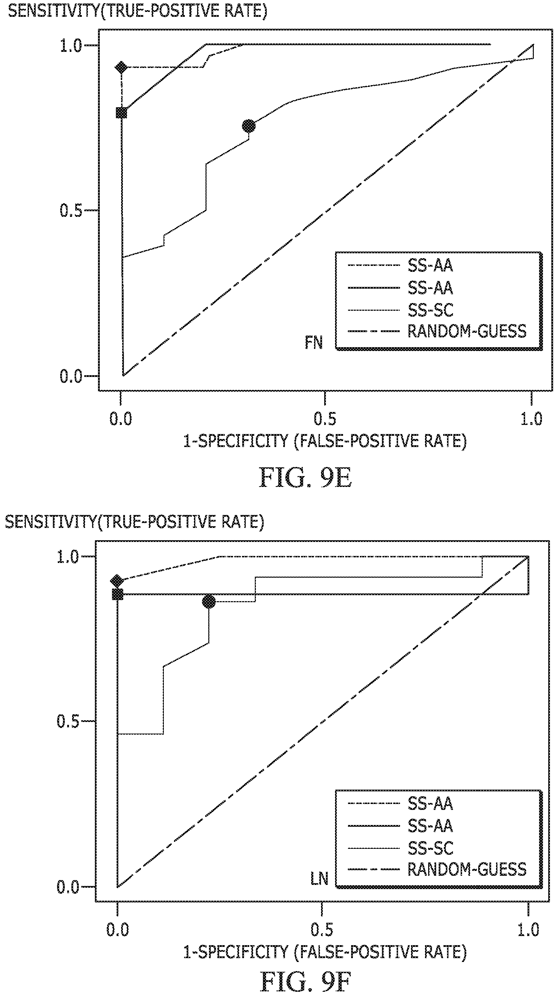

Figs. 9A-9B are high resolution images of the microchannels functionalized with FN and LN, respectively. The relationship between the individual components of the complete blood count and the number of adhered RBCs was analyzed using K-means clustering method. The patients with HbSS were clustered into two groups using K-means and the resultant groups were evaluated for differences in disease severity. Single components of complete blood counts as well as multiple components were used in K-means clustering to identify the two sub-groups.

Once the sub-groups were identified, the difference between the numbers of adhered RBCs between these groups was tested for statistical significance using one way ANOVA test. The testing level (alpha) were set as 0.05 (two-sided). The K-means clustering was performed using Matlab® (The MathWorks, Inc, Natick, MA).

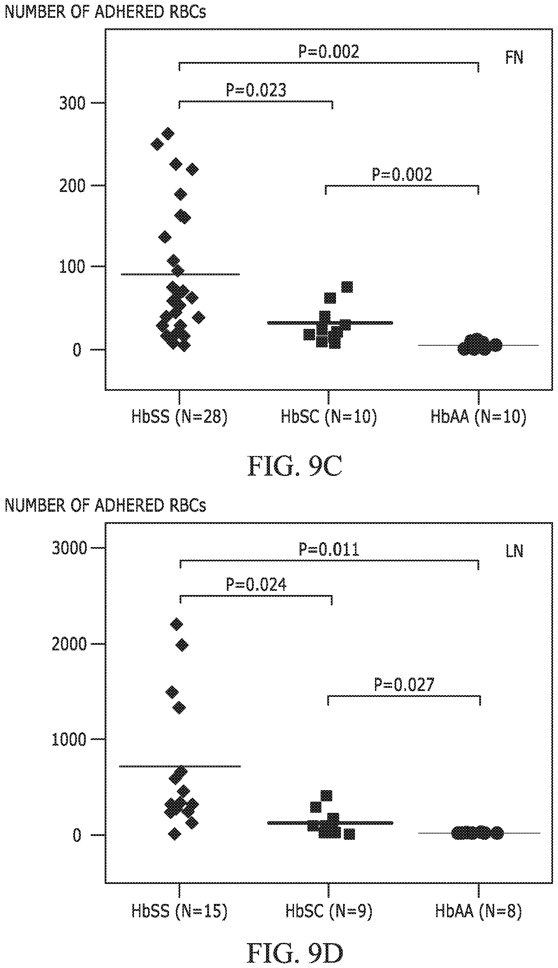

We analyzed the number of adhered RBCs per unit area (32 mm$^2$) in FN and LN functionalized microchannels in blood samples from subjects with HbAA, compound heterozygous HbSC or HbSβ+-thalassemia (HbSC/Sβ+) or homozygous HbSS, using high resolution images from microfluidic channels (*see* Figs. 9A-9B). Among blood samples with different hemoglobin phenotypes, the number of adhered RBCs was significantly higher in HbSS > HbSC/Sβ+ > HbAA blood samples in FN (P=0.023 and P=0.002 in Fig. 9C, respectively) and LN (P=0.024 and P=0.011 in Fig. 9D, respectively). Furthermore, HbSC/Sβ+ blood samples displayed a significantly higher number of adhered RBCs than HbAA blood samples in both FN (P=0.002) and LN (P=0.027) functionalized microchannels.

Next, we plotted receiver operating-characteristic (ROC) curves to assess the SCD-biochip's ability to accurately determine hemoglobin phenotypes through adhesion (*see* Figs. 9E-9F). For selected thresholds of adhered RBC numbers, we observed a 0.93 true-positive rate and 0.00 false-positive rate for differentiating between HbSS and HbAA phenotypes. The area under the curve for differentiating between HbSS and HbAA was more than 0.85 both for FN and LN. These results demonstrated the ability of the SCD biochip to discriminate amongst hemoglobin phenotypes through cellular adhesion. These data may further support the role that abnormal cellular adhesion plays in accounting for SCD phenotypes, in which HbSS is more hemolytic and HbSC less so.

In addition to the area under the curve, sensitivity, specificity, positive and negative likelihood ratios, and positive and negative predictive values were calculated as follows: Sensitivity was calculated as # true positives / (# true positives + # false negatives), specificity as # true negatives / (# true negatives + # false positives). Positive likelihood ratio was defined as sensitivity / (1-specificity). Negative likelihood ratio was (1-sensitivity) / specificity. Positive predictive value was # true positives / (# true positives + # false positives), and negative predictive value was # true negatives / (# true negatives + # false negatives).

*Statistical Analysis*

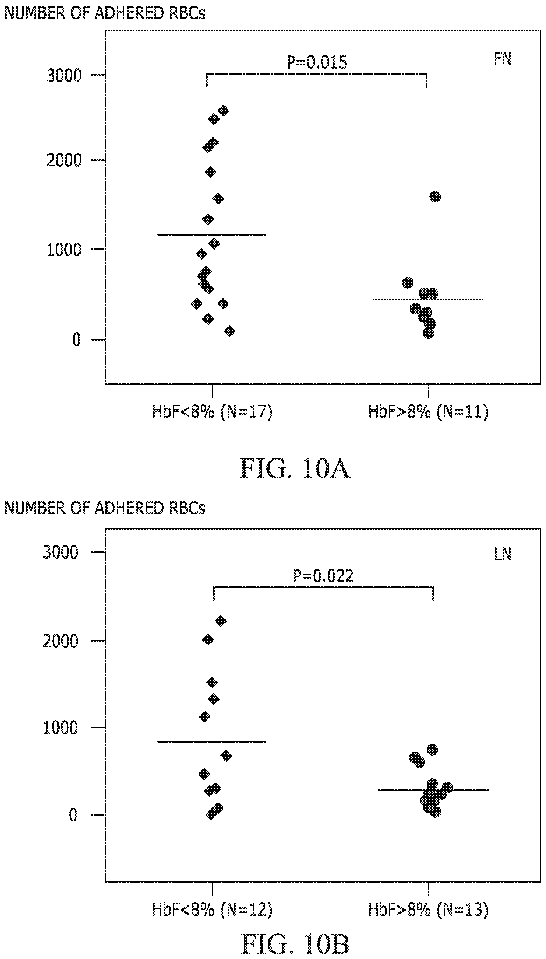

Because of the ameliorative role of HbF in SCD, we investigated RBC adhesion in HbSS blood samples with low (<8%) and high (>8%) HbF levels. When compared, the number of adhered RBCs was significantly higher in blood samples from subjects with low HbF than high HbF levels in both FN (P=0.015, Fig. 10A) and LN (P=0.022, Fig. 10B) functionalized microchannels. These findings established the base ground for the SCD biochip as an *in vitro* adhesion assay for functional phenotypes of SCD as well as the impact on these phenotypes of novel therapies.

ROC curves displayed a true-positive rate (sensitivity) and a false-positive rate (1-specificity) for differentiation between SS-AA, SC-AA, and SS-SC hemoglobin phenotypes based on the adhesion of RBCs on FN- and LN-functionalized microchannels. Defined thresholds for adhered RBC numbers on the ROC are as shown (◊=9, □=9, and ∘=30 for FN; ◊=16, □=16, and ∘=170 for LN). The ROC were strongest in discriminating between AA and SS or SC, compared with discrimination between SS and SC.

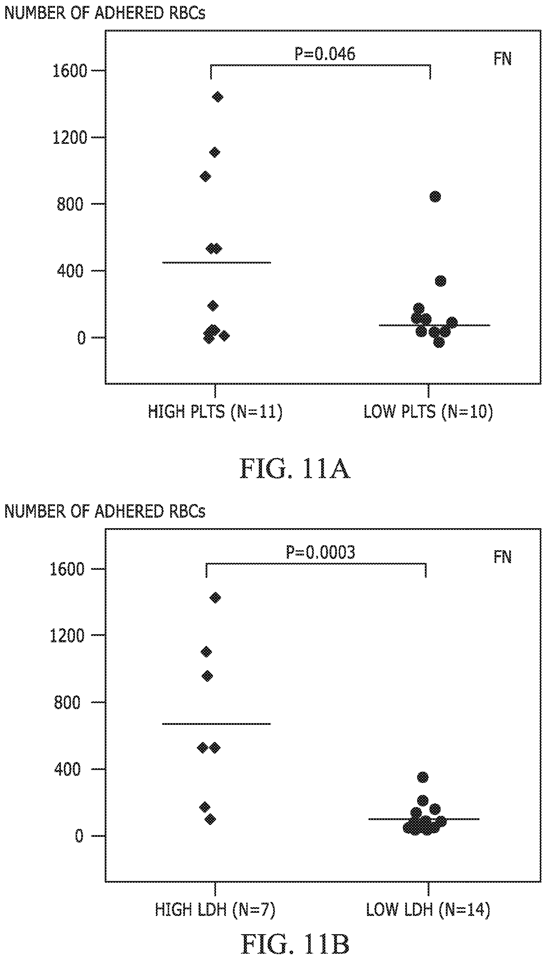

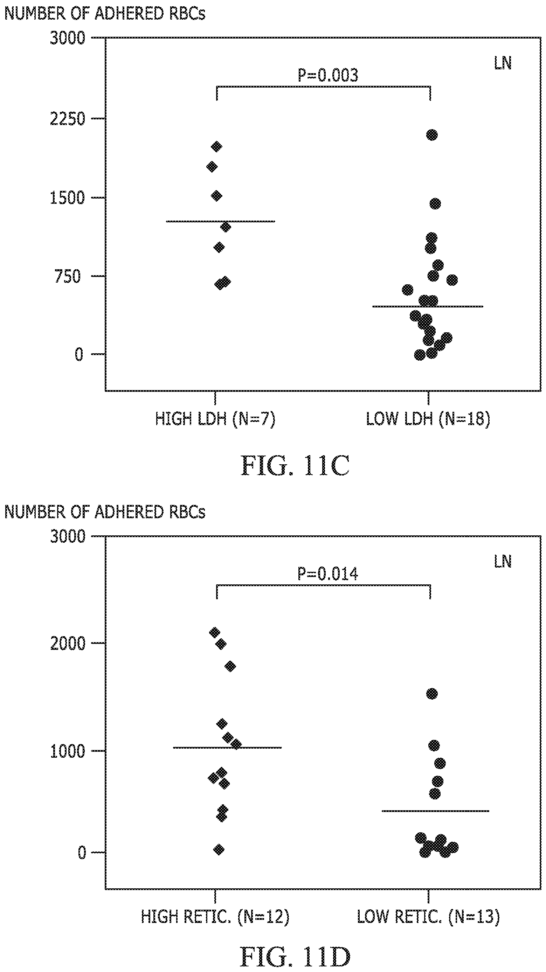

We utilized K-means clustering analysis to identify sub-groups among HbSS patients based on single components of the standard of care blood test results and hemoglobin testing results. As shown in Figs. 11A-11H, we analyzed RBC adhesion to FN and LN functionalized microchannels in HbSS blood samples from patients with various clinical phenotypes, including high and low lactate dehydrogenase (LDH), platelet counts (plts), and retics (retics). Samples from recently transfused subjects are shown with triangle markers. RBCs in blood samples from patients with high plts (>320 10$^9$/L) showed significantly higher adhesion to FN functionalized microchannels (P=0.046) than patients with low plts (<320 10$^9$/L) (Fig. 11A). We observed a significantly higher number of adhered RBCs to both FN (P=0.0003) and LN (P=0.003) functionalized microchannels in blood samples from patients with high LDH levels (>500 u/L) compared to patients with low LDH levels (<500 u/l) (Figs. 11B and 11C).

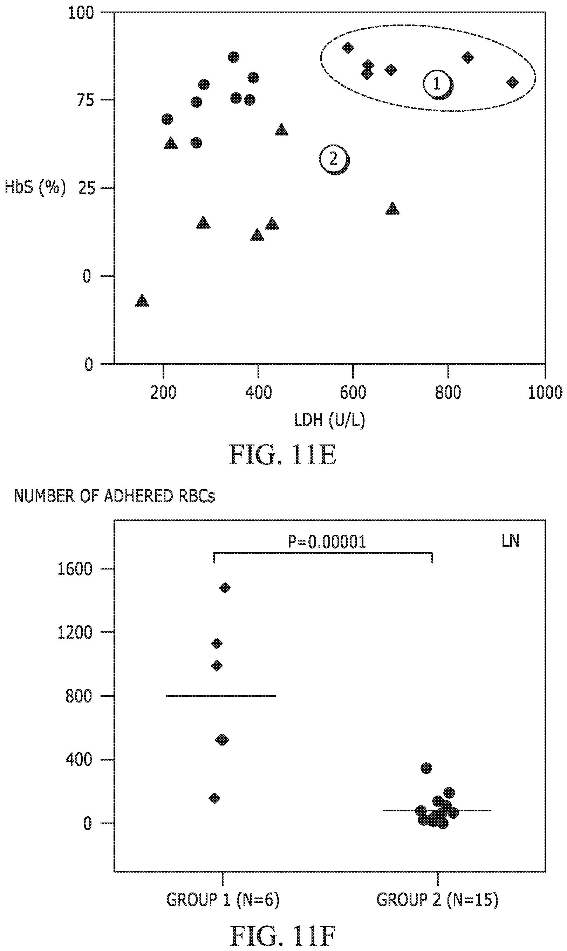

We also observed significantly higher RBC adhesion to LN functionalized microchannels (P=0.014) in blood samples from patients with high retics (>320 $10^9$/L) compared to patients with low retics (<320 $10^9$/L) (Fig. 11D). Levels of HbS varied due to recent transfusions (HbA >= 10%) or due to increased levels of HbF. High levels of HbS were associated with increased LDH levels (group 1 relative to group 2, Fig. 11E). RBC adherence to FN was significantly greater in blood samples with higher LDH and higher HbS levels (group 1 compared with group 2, Fig. 11F).

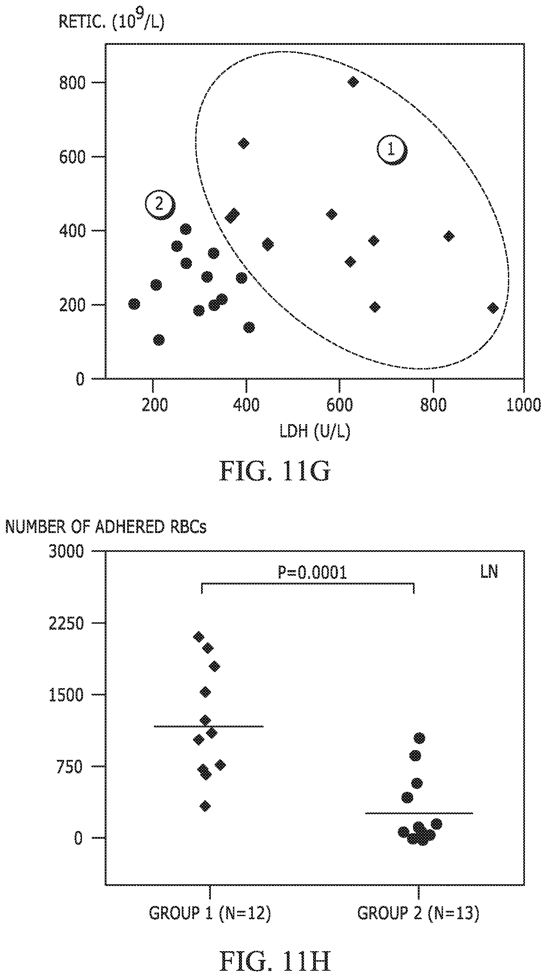

High levels of LDH were associated with elevated retics (group 1 relative to group 2, Fig. 11G). In univariate models, we observed significantly greater adhesion to LN in HbSS blood samples with higher LDH and higher retics (group 1 compared to group 2, Fig. 11H), independent of HbS.

These results show clinical associations between RBC adhesion to FN or LN and LDH, platelet counts or retics. Nonetheless, heterogeneity was present in all analyses, for example, low adhesion in some patients with a high LDH or elevated retics. This was also seen in a subset of chronically transfused patients. The converse was less ambiguous, for example, low adhesion was present in most patients with a low LDH.

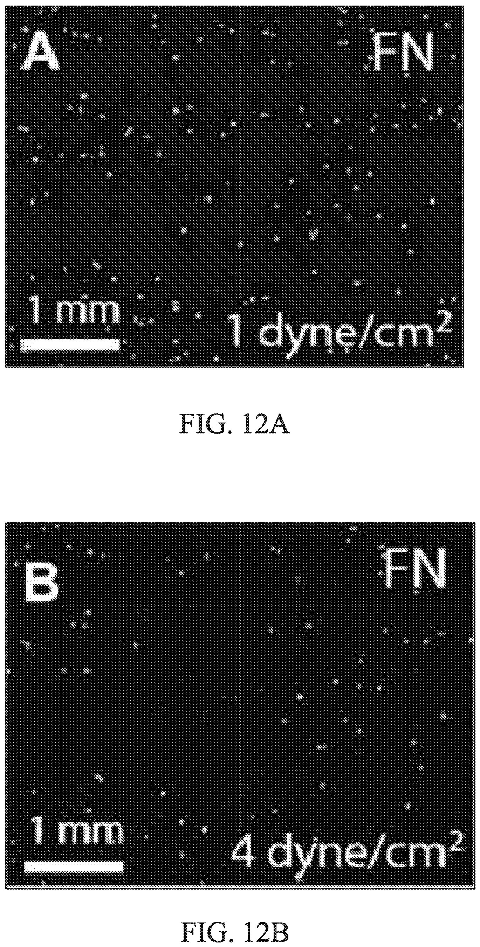

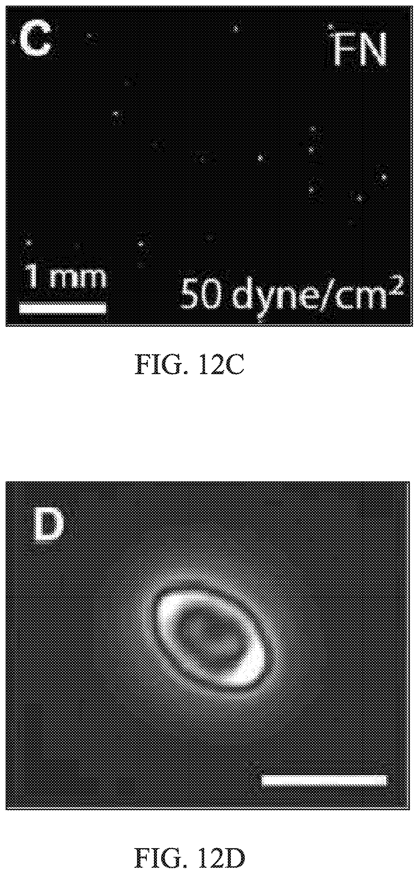

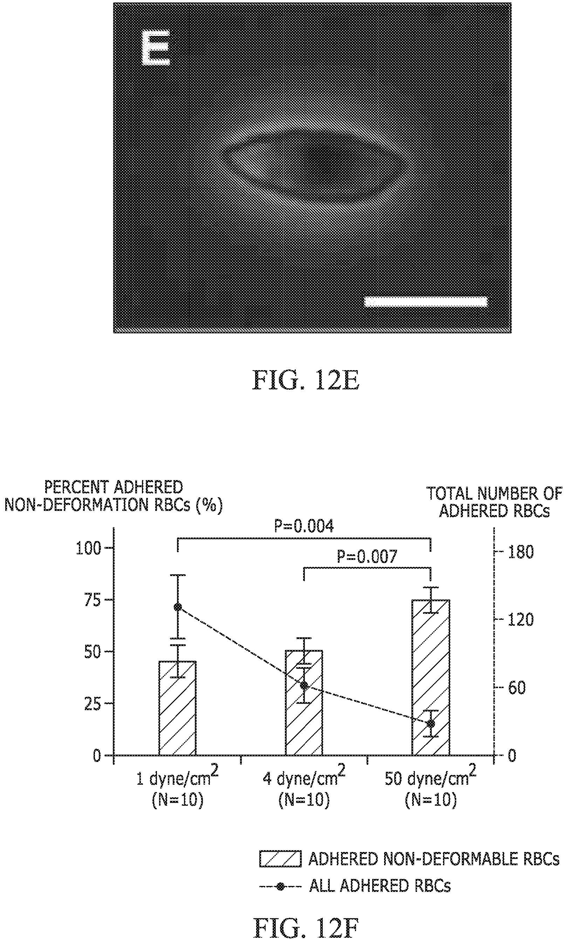

We also analyzed the heterogeneity of adhered RBCs in FN functionalized microchannels. The morphology and number of adhered RBCs in HbSS blood samples were quantified after controlled detachment of cells at step-wise increased flow velocities of 0.8 mm/s, 3.3 mm/s, and 41.7 mm/s (Figs. 12A-12C). Based on morphological characterization, adhered RBCs were categorized as deformable (biconcave shape shown in Fig. 12D) and non-deformable (without biconcave shape shown in Fig. 12E) RBCs. The scale bars represent a length of 5 $\mu$m.

Referring to Fig. 12F, the percentages of deformable and non-deformable RBCs of total adhered RBCs at 0.8 mm/s flow velocity were calculated. Deformable and non-deformable RBC percentages were not significantly different at 0.8 mm/s (P=0.266). On the other hand, the percentage of non-deformable RBCs was significantly higher than the percentage of non-deformable RBCs at 41.7 mm/s (P=0.047). A statistically significant correlation was observed between the percentage of adhered non-deformable RBCs and LDH at 0.8 mm/s (Pearson correlation coefficient of 0.79, n=21 samples). Moreover, the percentage of deformable RBCs was significantly lower at 3.3 mm/s and 41.7 mm/s compared to 0.8 mm/s (P=0.001 and P<0.001, respectively). Also, the percentage of deformable RBCs was significantly lower at 41.7 mm/s than 3.3 mm/s (P<0.001).

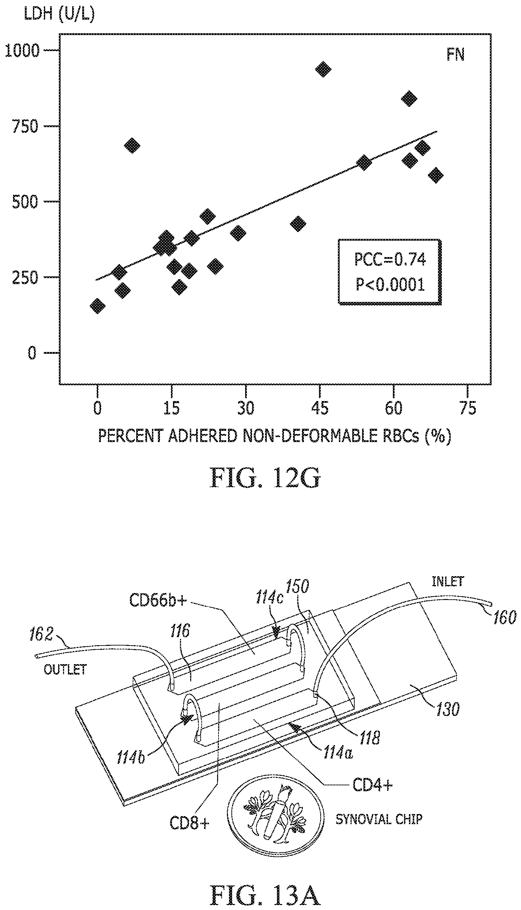

However, the only significant difference in the percentage of non-deformable RBCs were observed between 0.8 mm/s and 41.7 mm/s (P=0.003). Furthermore, we observed a significant association between adhered non-deformable RBCs (%) and serum LDH levels in our subjects (Pearson correlation coefficient of 0.74, p<0.0001 in Fig. 12G). These results indicated a morphological and qualitative heterogeneity in adhered RBCs and an association between hemolysis and adherent non-deformable RBCs.

In this example, we identified a unique adherent nondeformable RBC population, significantly correlated with serum LDH levels, which reflects a functional outcome in terms of both deformability and adhesion of RBCs and is a pathophysiologically plausible source for active hemolysis in SCD.

The new SCD biochip platform, introduced here, has the potential to informatively interrogate leukocyte adhesion, leukocyte activation, and RBC adhesion to other endothelial surface proteins, which we will incorporate in future studies.

## Example 2 (comparative only)

In this example, we designed and tested a disposable microfluidic device to capture and quantify three WBC subpopulations (CD4+, CD8+, and CD66b+ cells) simultaneously from synovial fluid aspirates. To this end, WBCs in a synovial fluid sample were isolated and analyzed by a microfluidic device.

### Methods

### Synovial chip fabrication

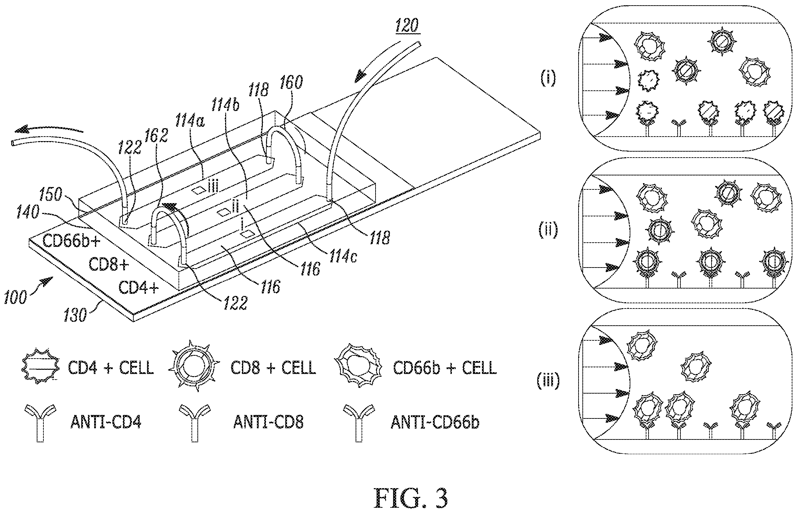

The synovial chip was constructed as shown in Fig. 3. The synovial chip was assembled using a glass slide 130 (adhesion coating: APTES, 3-Aminopropyl Triethoxysilane, Electron Microscopy Sciences, Hatfield, PA), a 50 $\mu$m thick DSA film 140 (iTapestore, Scotch Plains, NJ) with three channel 114a-114c cut outs defining the channel shape and height/depth, and a biomedical grade PMMA substrate 150 encompassing inlets and outlets 118, 122. Channel cut outs in the DSA 140 along with inlets and outlets 118, 122 in the PMMA substrates 150 were fabricated using VersaLaser $CO_2$ laser micromachining system (Universal Laser Systems Inc., Scottsdale, AZ). Microchannels 114a-114c were assembled by sandwiching the DSA film 140 between the slide 130 and the PMMA substrate 150, creating three microchannels of 10 $\mu$L volume.

### Surface chemistry

The cell adhesion surfaces 116 of each microchannel 114a-114c was functionalized with antibodies against specific cell membrane antigens for isolating of WBC subpopulations. After assembly, the microchannels 114a-114c were flushed with PBS followed by ethanol and incubated with N-gamma-Maleimidobutyryl-oxysuccinimide ester (GMBS, 0.28% v/v in ethanol) for 15 minutes at room temperature. Incubation is desirable to make sure sufficient electrostatic interaction has occurred between negatively charged residues of GMBS molecule and positively charged APTES coating of glass slides.

The microchannels 114a-114c were flushed again with ethanol and PBS in order to remove any remaining GMBS solution not adhered to the cell adhesion surfaces 116. Following PBS, 20 $\mu$L of NeutrAvidin (1 mg/mL in PBS) solution was injected into each microchannel 114a-114c and incubated overnight at 4°C. Overnight incubation is desirable to make sure that covalent interactions between GMBS and NeutrAvidin occur, allowing GMBS to serve as an anchor for NeutrAvidin to the cell adhesion surfaces 116. Following incubation, the microchannels 114a-114c were flushed with PBS in order to remove any remaining non-bound NeutrAvidin.

In order to functionalize each cell adhesion surface 116 for isolation of specific WBC subpopulations from the synovial fluid, biotinylated anti-human CD4+ (i), CD8+ (ii), and CD66b+ (iii) antibody solutions were injected into different microchannels 114a-114c and incubated for 1 hour at 4°C. The microchannels 114a-114c were then washed with PBS once more to clear any non-bound antibody.

## Synovial Fluid Processing and Data collection

19 synovial fluid aspirates were obtained from 17 anonymous patient donors from the Louis Stokes Veterans Affairs Medical Center in Cleveland, Ohio or from an outside source (Anatomy Gift Registry Inc., Hanover, MD) under IRB approval. The samples underwent aspiration to rule out infection of prosthetic knee joints. All of the subjects had negative cultures and none of them went on to develop periprosthetic infections. Blood samples of healthy donors were obtained from Research Blood Components (Boston, MA) under IRB approval. Samples were collected in Ethylenediaminetetraacetic acid (EDTA)-coated vacutainer tubes.

The synovial fluid aspirates were spiked with blood in order to inoculate aspirates with WBCs to obtain clinically relevant cell concentrations to simulate diseased conditions. This approach also allowed the robustness of the synovial chip 100 to be evaluated for clinical cases where hemorrhaging may be present in the joint, which populates synovial fluid with an excess of RBCs.

Synovial fluid samples 120 were either diluted with PBS or treated with lyophilized hyaluronidase enzymatic powder (Sigma-Aldrich, St Louis, MO) in order to reduce viscosity. Lyophilized hyaluronidase enzyme powder was dissolved into synovial fluid samples at a concentration of 0.5 mg/mL, vortexed, and incubated for 15 minutes at room temperature.

Afterwards, samples 120 were loaded into 1 mL disposable syringes and connected to the inlet tubing of the synovial chip 100. Synovial fluid samples 120 of 100 $\mu$L were injected into the microchannels 114a-114c *via* the tubing 160, 162 using a New Era NE-1000 syringe pump system at 10 $\mu$L/min. The microchannels 114a-114c were then washed with 200 $\mu$L PBS at 10 $\mu$L/min to remove non-captured cells. Following the PBS wash, fluorescent anti-human CD4+, CD8+, and CD66b+ antibody solutions were injected into microchannels 114a-114c for fluorescent labeling of captured WBC subpopulations.

Referring to Figs. 13A-13D, phase contrast and fluorescent images of the microchannels 114a-114c with captured WBC subpopulations were obtained *via* an automated, inverted, fluorescent microscope (Olympus IX83, Tokyo, Japan). Microscope images were acquired using an Olympus long working distance objective lens (20x, numerical aperture, NA=0.45). Viscosity of both plain synovial fluid and then enzyme treated synovial fluid (1 cm$^3$) were measured using a rotational viscometer (MCR501, Anton Paar, Ostfildern, Germany).

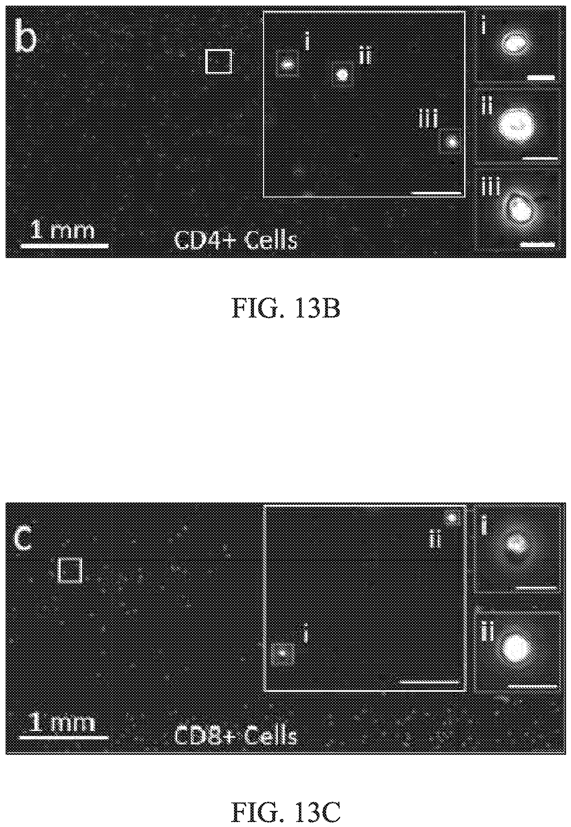

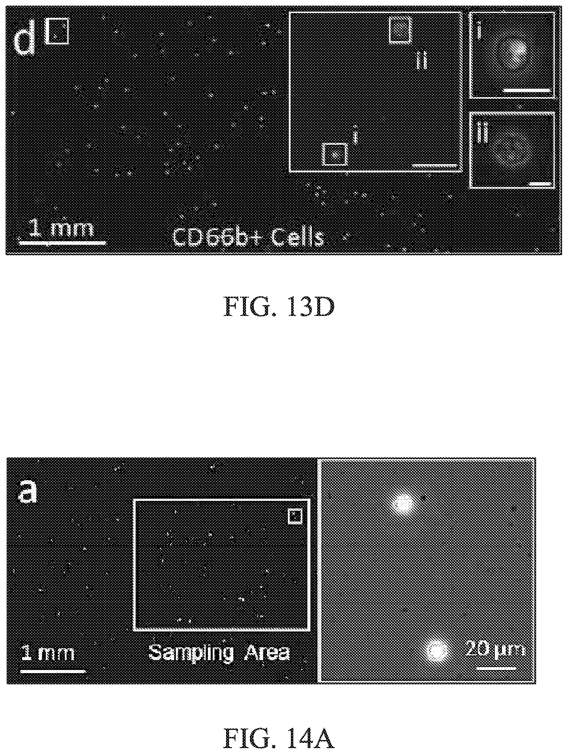

The number of captured WBCs per unit area (22 mm$^2$) in cell adhesion surfaces 116 functionalized with CD4+ (Fig. 13B), CD8+ (Fig. 13C), and CD66b+ (Fig. 13D) antibodies were quantified using high resolution phase contrast images of the microchannels 114a-114c. Food coloring was mixed into the synovial fluid sample 120 for visualization. Scale bars in Figs. 13B-13D and insets represent lengths of 50 $\mu$m and 10 $\mu$m, respectively.

## Flow Cytometry Analysis

Synovial fluid samples 120 processed in the synovial chip 100 were analyzed in parallel with flow cytometry to evaluate efficiency of the developed microfluidic platform. Synovial samples 120 were fluorescently labeled for CD4+, CD8+, and CD66b+ WBCs using the same fluorescent antibodies described above for on-chip staining of the same cell subtypes. Flow cytometry was carried out using a Guava EasyCyte Flow Cytometer (Merck Millipore, Billerica, MA) at Case Comprehensive Cancer Research Center at Case Western Reserve University.

*Data Analysis and Statistics*

Acquired phase contrast and fluorescent images were processed using Adobe Photoshop (San Jose, CA) to quantify the number of captured WBCs in each microchannel 114a-114c. Phase and fluorescent microscopy images were compared to calculate cell capture specificity. The rest of the data analyses was performed based on cell counts obtained from phase contrast images of the microchannels 114a-114c. Data obtained in this study was reported as mean $\pm$ standard error of the mean. Capture efficiency in the synovial chip 100 for samples 120 diluted with PBS and treated with hyaluronidase enzyme were statistically compared (Minitab 16 software, Minitab Inc., State College, PA) using ANOVA. All regression analyses were performed in Minitab 16 software. Statistical significance was set at 95% confidence level (p< 0.05) for all tests.

Results

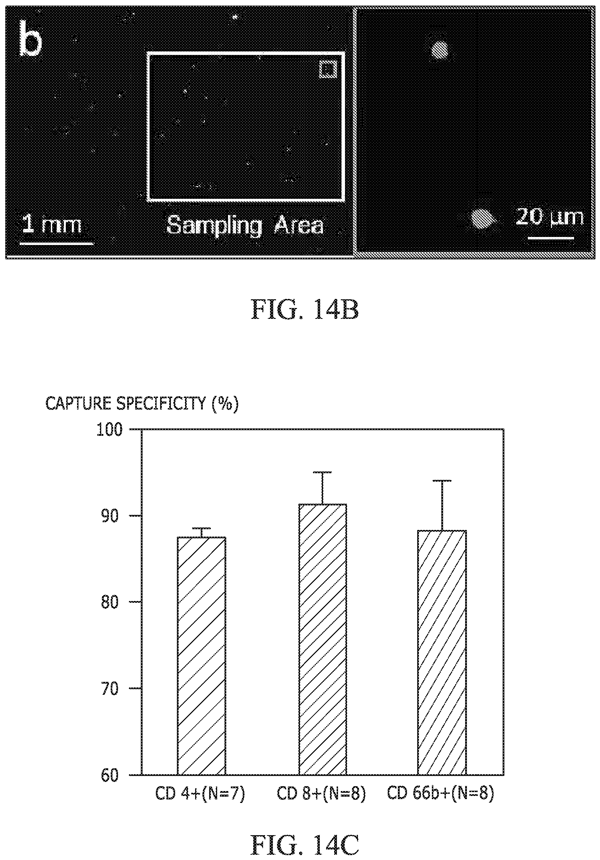

We assessed capture specificity of the synovial chip 100 by comparing phase contrast (all captured cells shown in Fig. 14A) and fluorescent images (cells labeled for a certain subpopulation shown in Fig. 14B) of microchannels 114a-114c after processing synovial fluid samples 120. The CD8+ cells were captured on cell adhesion surfaces 116 *via* immobilized anti-CD8+ antibodies. The capture specificity of the CD8+ cells was assessed by staining captured cells in microchannels 114a-114c with FITC conjugated CD8+ antibodies. The specificity was determined based on cell counts in phase contrast and fluorescent images of the sampling area. The presence of RBCs in synovial aspirates did not interfere with the capture and quantification of target WBC subpopulations. We observed a capture specificity of around 90% for CD4+, CD8+, and CD66b+ WBCs captured from synovial fluid samples (Fig. 14C). Error bars indicate a standard error of the mean.

*Effect of Synovial Fluid Viscosity on Capture Efficiency*

Synovial fluid is known to have high viscosity caused by high hyaluronic acid content. This may pose challenges for microfluidic processing due to high shear stresses and high pressure drops in microchannels. Shear stress on microchannel surfaces affects cell capture efficiency when processing bodily fluids, such as blood. Furthermore, high viscosity adds practical difficulties in terms of handling and loading the synovial samples into syringes. To overcome the challenge of processing high viscosity synovial aspirates in microchannels, we tested diluting synovial samples 120 with PBS and by treating synovial samples with hyaluronidase enzyme.

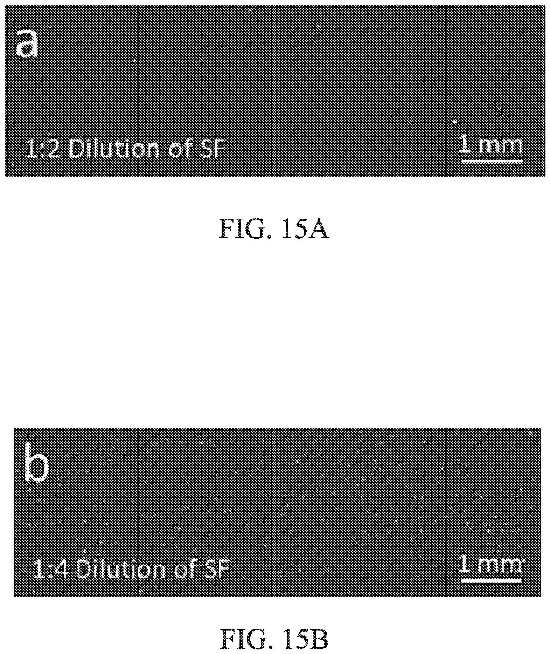

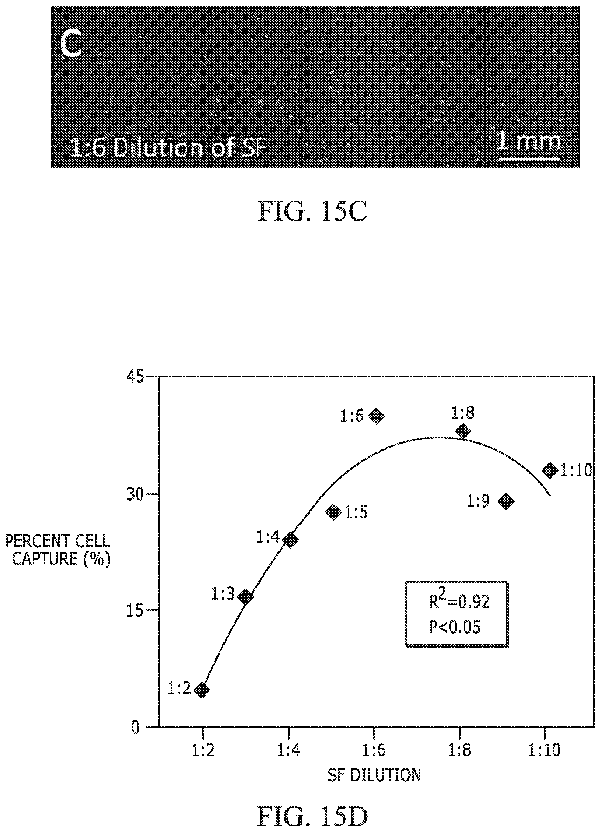

The dilution of synovial fluid with PBS can reduce shear stress on the channel surfaces and, thus, improve CD8+ cell capture efficiency. We diluted synovial aspirates with PBS at ratios from 1:2 to 1:10 (Figs. 15A-15C), and observed a statistically significant correlation ($R^2$=0.92, p<0.05) between the dilution ratio and CD8+ WBC capture efficiency, assessed in comparison to cell capture from whole blood (Fig. 15D). Even though the cell capture efficiency was significantly improved (>30%) with increased PBS dilution, it saturated over a dilution ratio of 1:8.

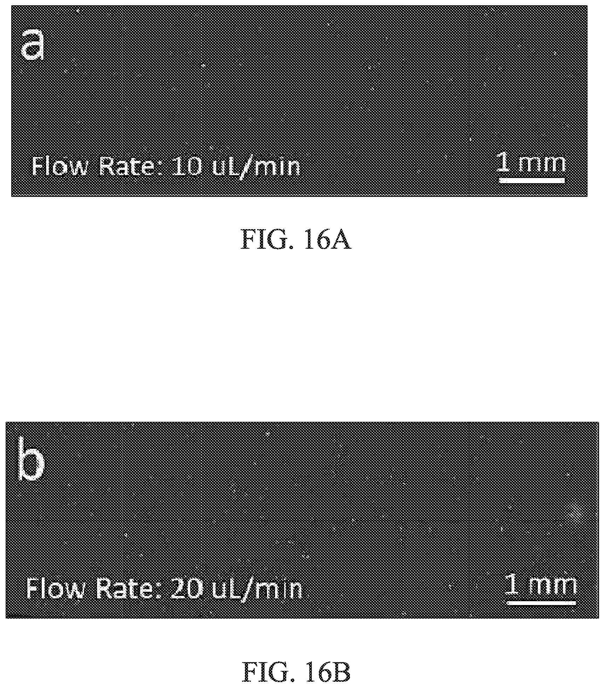

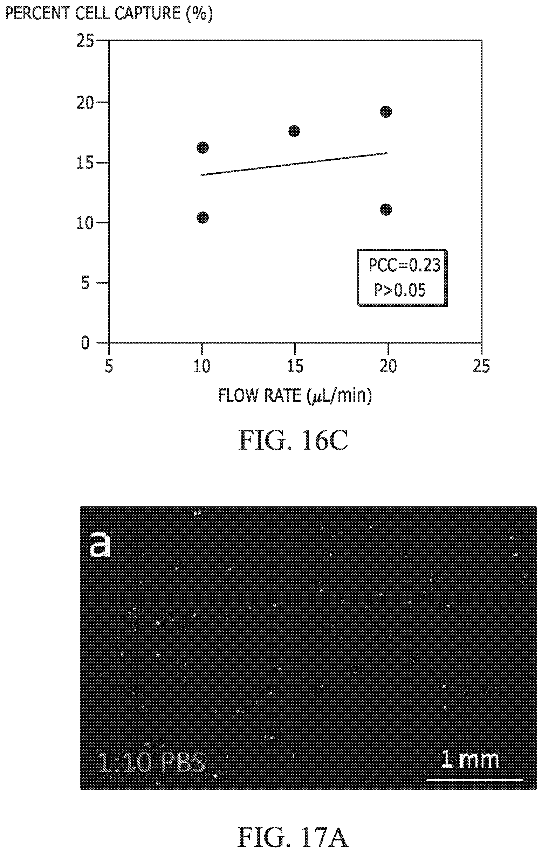

This approach also caused an increase in sample volumes to be processed in the order of the corresponding dilution rate. Such increase in the sample volume caused a dramatic increase in sample processing time, which could limit the feasibility of the system at the POC. In order to improve sample processing time, we analyzed cell capture efficiency for 1: 10 diluted synovial fluid samples processed at different flow rates, namely, 10 $\mu$L (Fig. 16A) and 20 $\mu$L/min (Fig. 16B). However, there was no statistically significant correlation between cell capture efficiency, assessed in comparison to cell capture from whole blood, and synovial sample processing flow rates (PCC = 0.23, p<0.05, Fig. 16C).

To overcome these shortcomings, we utilized hyaluronidase enzyme (0.5 mg/mL, Sigma Aldrich) to digest hyaluronic acid content in synovial fluid, thereby decreasing the viscosity of the synovial fluid samples. Hyaluronic acid is one of the main components contributing to the high viscosity that is observed in synovial fluid. Hyaluronidase enzyme has been commonly used to digest hyaluronic acid and has shown to improve performance of cellular, protein, and crystal analyses.

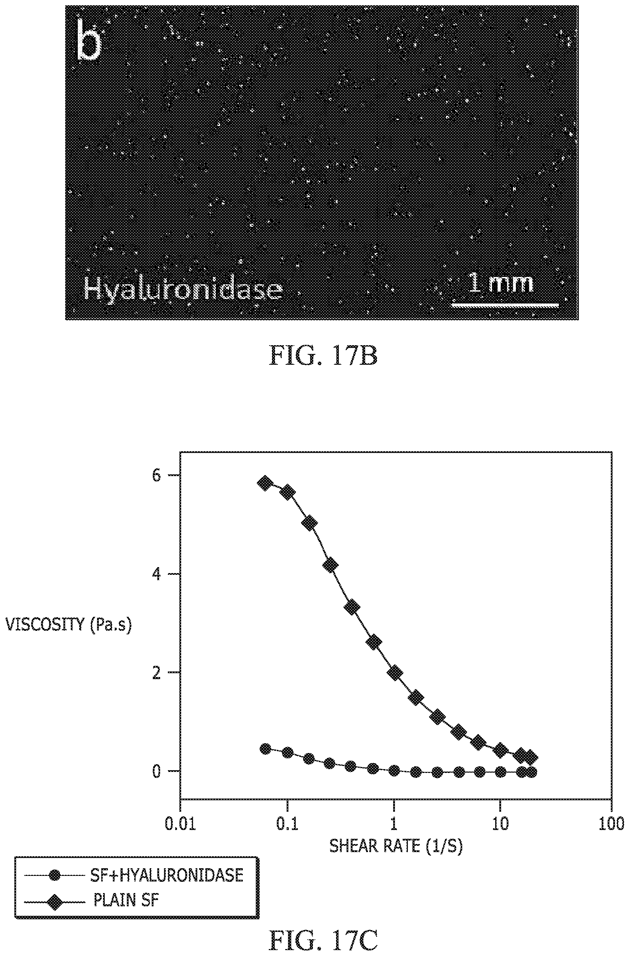

We measured the viscosity of both hyaluronidase enzyme treated and plain synovial fluid using a rheometer. CD8+ WBC capture after processing a synovial fluid sample diluted 1: 10 with PBS (Fig. 17A) and mixed with hyaluronidase enzyme (Figs. 17B). Hyaluronidase enzyme treatment decreased the viscosity of synovial fluid dramatically, more than 15 fold at 0.1 s$^{-1}$ and 60 fold at 1 s$^{-1}$ shear rate (Fig. 17C). The increase in the number of captured cells suggests that enzymatic degradation of hyaluronic acid does not damage cell surface antigens that are necessary for capture. Furthermore, the downward slope of the plain synovial fluid line with increasing shear rate indicates Non-Newtonian behavior of synovial fluid.

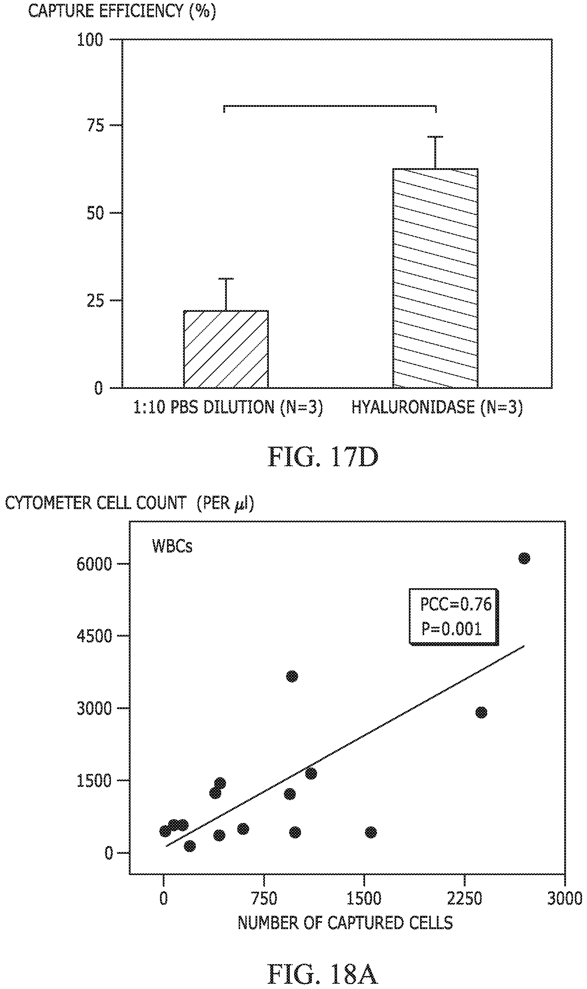

Overall, hyaluronidase enzyme treatment significantly improved cell capture efficiency, assessed in comparison to cell capture from whole blood, to over 60% (more than 3-fold increase) compared to samples diluted with PBS (Fig. 17D, one-way ANOVA test p<0.05, n=3), which also decreased the processing time by 10-fold in the synovial chip 100.

*Microfluidic Processing*

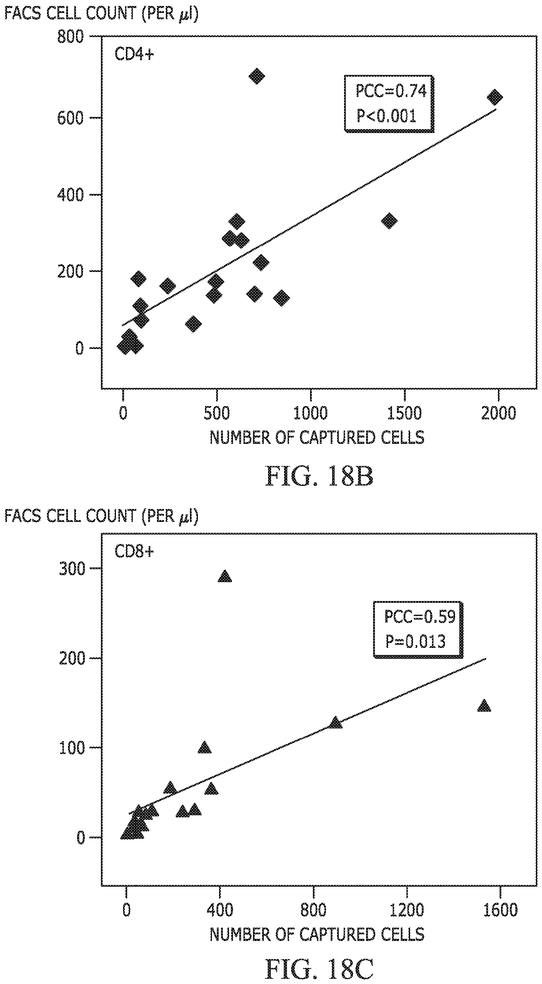

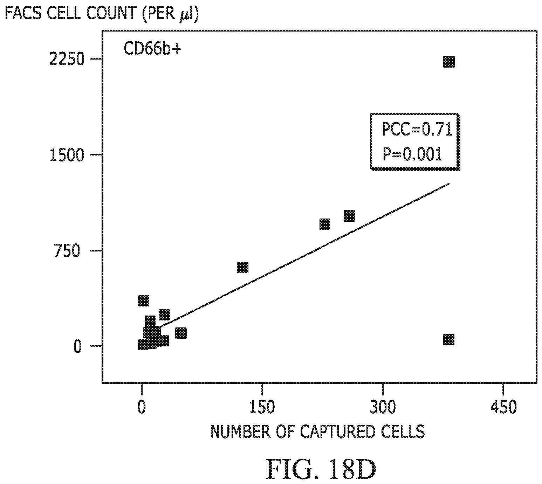

We correlated the number of captured CD4+, CD8+, and CD66b+ cells in the synovial chip 100 with FACS cell counts for these same cell types and also total cytometer WBC counts. The number of captured cells in the synovial chip 100 showed statistically significant correlation with total cytometer WBC counts (Fig. 18A, Pearson correlation coefficient, PCC=0.76, p=0.001, Spearman's rho, $r_s$=0.46, n=15), FACS counts for CD4+ (Fig. 18B, PCC=0.74, p<0.001, $r_s$=0.78, n=19), CD8+ counts (Fig. 18C, PCC=0.59, p=0.013, $r_s$=0.93, n=17), and CD66b+ counts (Fig. 18D, PCC=0.71, p=0.001, $r_s$=0.58, n=17).

## Example 3

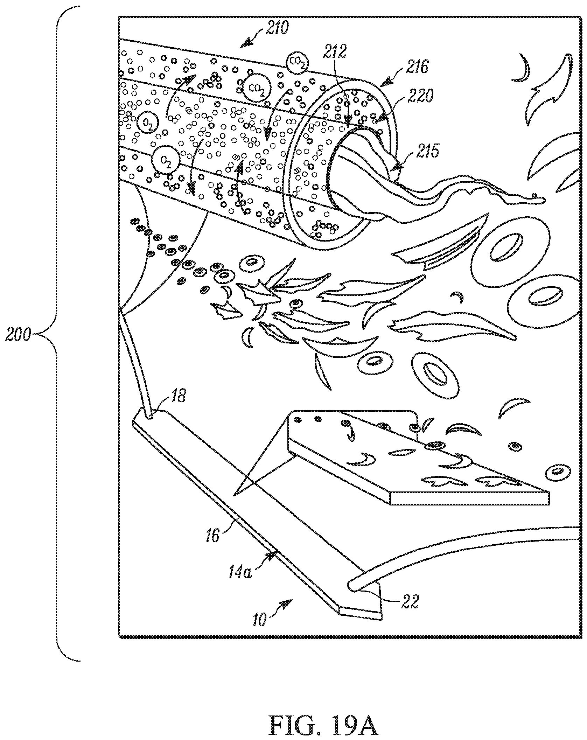

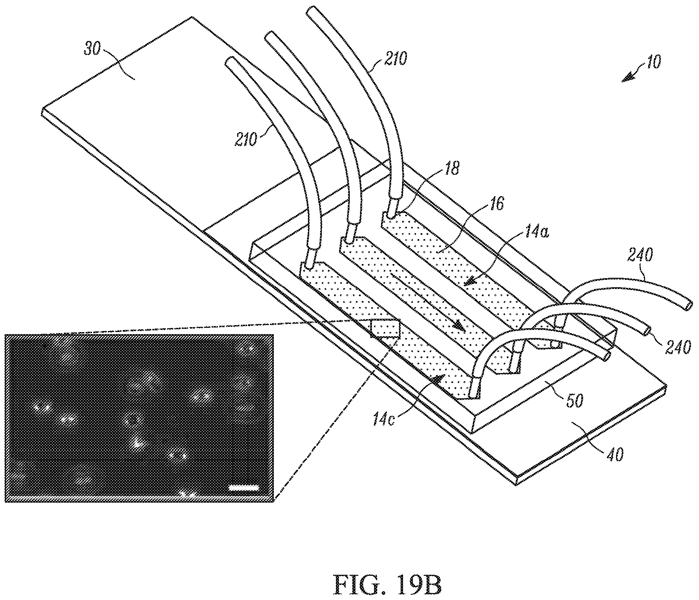

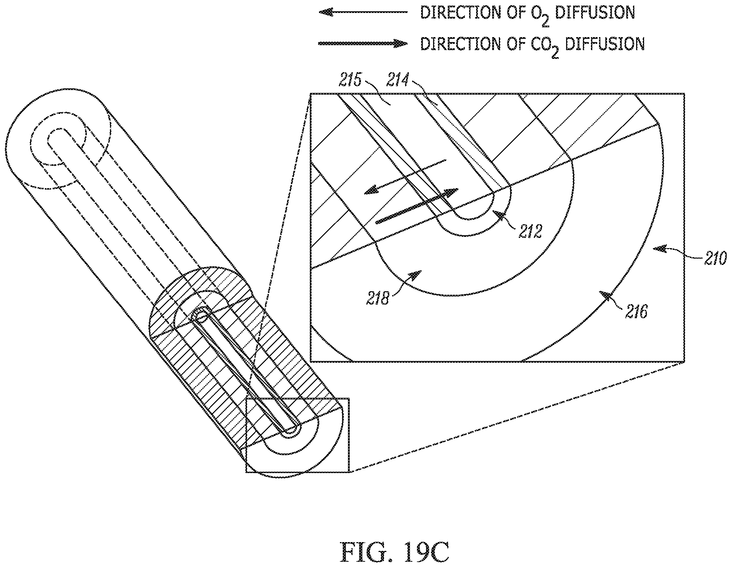

In this study, a microfluidic system 200 (Figs. 19A-19C) having a microfluidic device and at least one micro-gas exchanger was used to adjust the oxygen tension in the blood sample prior to introduction thereof into the microchannels 14a-14c. Blood deoxygenated at the micro-gas exchanger during flow, sickling RBCs, and reached the microchannels 14a-14c with RBCs adhering to the functionalized cell adhesion surfaces. The micro-gas exchanger allowed for easy adaptation to portable POC microfluidic systems.

The microfluidic device integrated with a micro-gas exchanger described herein allows interrogation and manipulation of biological fluid at a single-cell level while being clinically feasible, cost and labor efficient, and easily implementable. The developed microfluidic platforms eliminate the intricate microchannel design and configuration required in PDMS based systems by controlling the oxygen tension of the biological fluid before it reaches the microchannel. We utilized the microfluidic platform to analyze the adhesion of RBCs in blood samples of patients with SCD where oxygen tension control is desirable.

*Blood collection*

Blood samples from SCD and normal subjects were attained with the standard laboratory procedures approved by the institutional review board (IRB). All clinical information such as medical history and treatment course was obtained after the patients were informed and consented.

Upon collection, the samples were treated with an anticoagulant, EDTA (Ethylenediaminetetraacetic acid), in vacutainer tubes and were stored at 4°C. All the experiments were performed within 24 hours of venipuncture, using whole blood samples without dilution. Clinical data such as hemoglobin phenotype (%), complete blood counts (including white blood cell (WBC) ($10^9$/L), RBC ($10^{12}$/L), absolute neutrophil count (ANC) ($10^6$/L), and plts ($10^9$/L)) retics ($10^9$/L), and plasma LDH (IU/L) were received from the Adult Sickle Cell Clinic at University Hospitals Case Medical Center in Cleveland. High-performance liquid chromatography (HPLC) was conducted to identify the proportion of HbS, HbF, HbA, and $HbA_2$ with the Bio-Rad Variant II Instrument (Bio-Rad, Montreal, QC, Canada) at the Core Laboratory of University Hospitals Case Medical Center.

For anti-BCAM blocking experiments, whole blood samples were treated with the antibody of basal cell adhesion molecule, a Lutheran blood group glycoprotein (BCAM: CD239 Antibody, Novus Biologicals), by adding BCAM antibody to the whole blood. 50 μL of BCAM antibody (0.5 mg/mL in PBS) was added to 300 μL of blood samples and were incubated at room temperature for 15 minutes.

*System Preparation and Operation*

The system 200 was comprised of two main components: the microfluidic device 10 previously described, and at least one micro-gas exchanger 210 (Figs. 19A-19C). To mimic a blood vessel wall, the 50 μm depth microchannels 14a-14c were functionalized with N-g-Maleimidobutyryloxy succinimide ester (GMBS: 0.28% v/v). Endothelium associated proteins FN (FN: 1:10 dilution with PBS, Sigma Aldrich) and LN (LN: 1:10 dilution with PBS, Sigma Aldrich) were immobilized on the cell adhesion channel surfaces 16. After 1.5 hours of incubation at room temperature with either protein, the microchannels 14a-14c were flushed with bovine serum albumin solution (BSA: 20 mg/ml) and incubated overnight at 4°C to prevent non-specific binding events.

The micro-gas exchanger 210 includes concentric inner and outer tubes 212, 216. The inner tube 212 has a gas-permeable wall 214 defining a central passage 215 extending the entire length of the inner tube. The outer tube 216 has a gas impermeable wall 218 defining a central passage extending the entire length of the outer tube. An annular space 220 is formed between the tubes 212, 216. The central passage 215 receives the blood sample 20 and is in fluid communication with one or more inlet ports 18 of the microfluidic device 10. As shown, each inlet port 18 is fluidly connected to a different micro-gas exchanger 210 to specifically tailor the blood delivered to each microchannel 14a-14c. An outlet tube 240 is connected to each outlet port 22. A controlled gas flow takes place in the annular space 220 and blood flows inside the inner tube 212. The deoxygenation of blood occurs due to gas diffusion (5% $CO_2$ and 95% $N_2$) through the inner gas-permeable wall 214.

To setup the micro-gas exchanger 210, medical grade gas-permeable silicone tubing 212 (300 $\mu$m inner diameter (ID) x 640 $\mu$m outer diameter (OD), Silastic Silicone Laboratory Tubing, Dow Corning) was placed inside the impermeable tubing 216 (1600 $\mu$m ID x 3200 $\mu$m OD, FEP tubing, Cole-Parmer). The gas permeability of the outer FEP tubing 216 (0.59 Barrer for $CO_2$; 1.4 Barrer for $O_2$) and was less than 0.2% of the inner silicone tubing 212 (2000 Barrer for $CO_2$; 800 Barrer for $O_2$) for both $CO_2$ and $O_2$. Due to this construction, the blood sample could exchange gases through the permeable inner tubing wall 214 with 5% $CO_2$ and 95% $N_2$ controlled gas inside the impermeable tubing annular space 220 by diffusion.

The silicone tubing 212 was filled with PBS (Life Technologies) before flowing the blood therethrough. All possible points of leakage were sealed with an epoxy glue (5 Minute Epoxy, Devcon) to ensure there was no gas diffusion inside the microfluidic device 10.

The blood sample 20 was injected with the syringe pump (NE300, New Era Pump Systems) into the system 200 at 18.5 $\mu$L/min to fill the tubing passage 215 and the microchannels 14a-14c, and at 1.85 $\mu$L/min to impose about 1 dyn/cm$^2$ of shear stress while flowing in the functionalized microchannels. After flowing 15 $\mu$L of blood through the microchannels 14a-14c, flow cytometry staining buffer (FCSB, 1X) was inserted to wash the microchannels at 10 $\mu$L/min, which imposed 1 dyn/cm$^2$ of shear stress, making the adhered RBCs visible through the inverted microscope (Olympus IX83) and microscopy camera (EXi Blue EXI-BLU-R-F-M-14-C) for high-resolution images. At least 180 $\mu$L of FCSB was used to wash the microchannels 14a-14c and the images were taken during buffer flow. For the entire procedure, the medium flowing in each microchannel 14a-14c was deoxygenated by a micro-gas exchanger 210 to achieve physiological flow and hypoxic conditions.

*Computational Modeling and Experimental Validation*

The gas diffusion of blood during flow within the micro-gas exchanger 210 was modeled using COMSOL (AltaSim Technologies, Columbus, OH). Two sets of boundary conditions were chosen to couple laminar flow and gas diffusion through the permeable tubing wall 214. To approximate the diffusion coefficient of $CO_2$ and $O_2$ for blood, the kinematic viscosity of blood was first calculated to be ~0.054 Pa·s at room temperature, assuming 2% increase every 1 °C drop. Then the Hagen-Poiseuille equation was used to approximate the pressure difference ($\Delta$P) along the tubing. As blood was assumed to be Newtonian, the simulation result was used as a baseline reference for the experimental setup (Table 2). The flow of FCSB was also computationally modeled to analyze adequate tubing length and flow rates for constant deoxygenation of the microenvironment.

Table 2

| Parameter | Value |
|---|---|
| Inner Diameter | 300 $\mu$m |
| Outer Diameter | 640 $\mu$m |
| Length | 250 mm |
| Flow Velocity | 18.5 $\mu$l/min |
| Pressure Difference | 3.75 cmHg |
| Initial $O_2$ Concentration | 8.17 mol/m$^3$ |
| Initial $CO_2$ Concentration | 0.01 mol/m$^3$ |
| Controlled $O_2$ Concentration | 0 mol/m$^3$ |
| Controlled $CO_2$ Concentration | 2.04 mol/m$^3$ |
| Permeability ($O_2$) | 800 Barrer |
| Permeability ($CO_2$) | 2000 Barrer |
| Viscosity at room temperature | 5.4 mPa·s |
| Diffusion Coefficient ($O_2$) | 1.6E$^{-9}$ m$^2$/s |
| Diffusion Coefficient ($CO_2$) | 6.0E$^{-10}$ m$^2$/s |

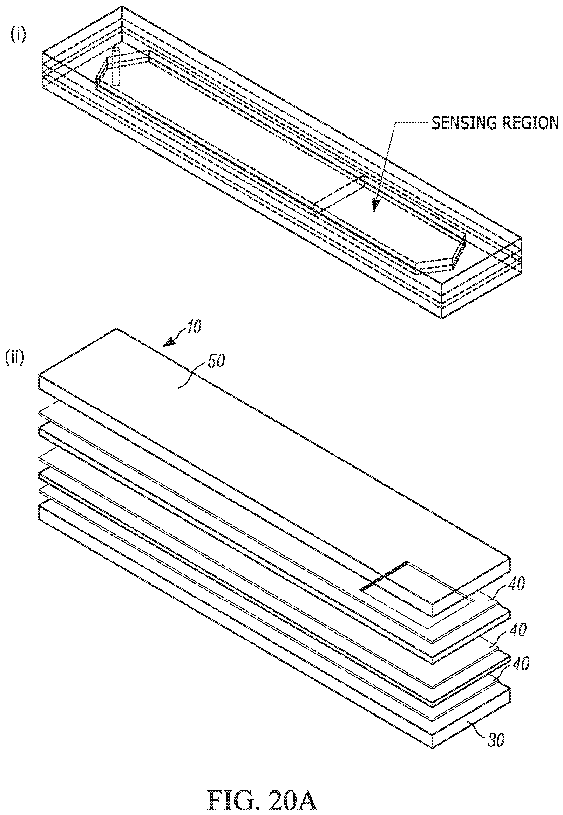

After optimizing the setup parameters, such as tubing length, flow rate, and pressure difference, simulation results were validated by measuring the oxygen saturation level of blood ($SpO_2$) with a finger pulse oximeter (OT-99, Clinical Guard) using a model finger. The model finger was fabricated by stacking lasercut PMMA sheets 50 with DSA films 40 (Figs. 20A-20B). Specific geometries were chosen to prevent bubble formation and maximize sensing capabilities, thereby requiring ~300 $\mu$L of blood.

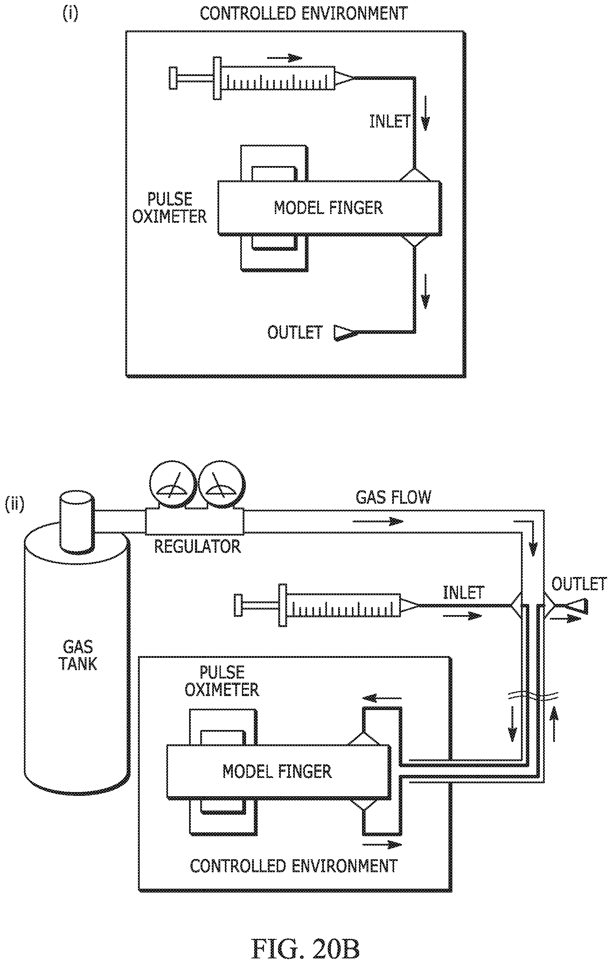

The validation experiment setup was placed at temperature and gas concentration controlled environments to test the *in vitro* measurements of the finger pulse oximeter (Fig. 20B). The pulse-oximeter measurements in the model finger were either performed without the micro-gas exchanger in room, oven, and cell culture incubator conditions (Fig. 20B(i)) or with the micro-gas exchanger in room and oven conditions (Fig. 20B(ii)). In the former case, the oven was set to 37°C and the incubator was set to 37°C and 5% $CO_2$. In the latter case, the pulse-oximeter measurements were taken with the micro-gas exchanger at 95% $N_2$ and 5% $CO_2$ to control the gas concentration of the blood. The oven was set to 37 °C.

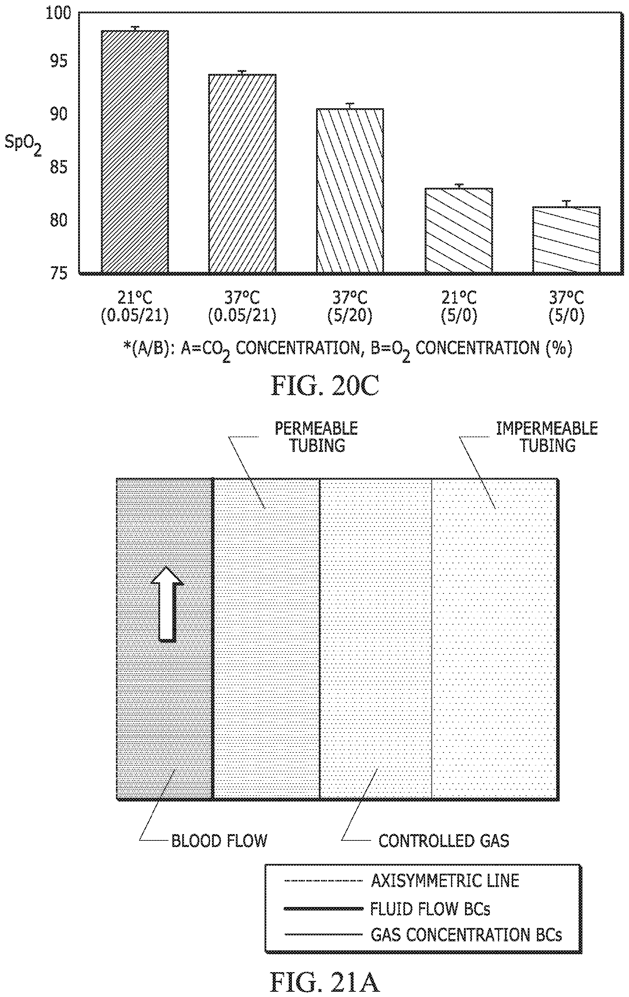

Blood injected into the model finger using the micro-gas exchanger resulted in a $SpO_2$ of 83% at room temperature, measured with the pulse oximeter. The results from computational and experimental analyses matched when the $SpO_2$ values from validation experiments were converted to percent gas concentration using hemoglobin oxygen dissociation curve (Fig. 20C).

The pulse oximeter measurements in the model finger without the micro-gas exchanger resulted in blood oxygen saturation of 98%, 94%, and 91% for room, oven, and cell culture incubator conditions, respectively. All the values were reasonable as their trends were expected according to the Bohr Effect, which states that the oxygen affinity of hemoglobin is affected by pH level shifts caused by temperature and $CO_2$ level changes.

### Image Processing and Quantification

Phase contrast images of the microfluidic channels were obtained using an Olympus long working distance objective lens (20x/0.45 ph2) and commercial software (cellSens Dimension, Olympus Life Science Solutions, Center Valley, PA). This provided mosaic patches of a designated area. Microchannel images were processed using Adobe Photoshop software (San Jose, CA) for the quantification of adhered RBCs per unit area (32 mm$^2$).

### Statistical Analysis

RBC adhesion data was collected from 45 blood samples from 35 patients. One-way ANOVA test was utilized with the minimum confidence level of 95% ($p < 0.05$) to assess the statistically significant difference between related data sets, such as the number of adhered RBCs during hypoxia and normoxia (Microsoft Excel, Seattle, WA). Individual patients' clinical data such as LDH level, reticulocyte count, HbF level were compared between responsive and non-responsive groups. Bar graphs reported show mean $\pm$ standard error of the mean, and the dotted plots show mean lines with p-values from one-way ANOVA test.

### Results

### Microfluidic Platform integrated with the Micro-Gas Exchanger

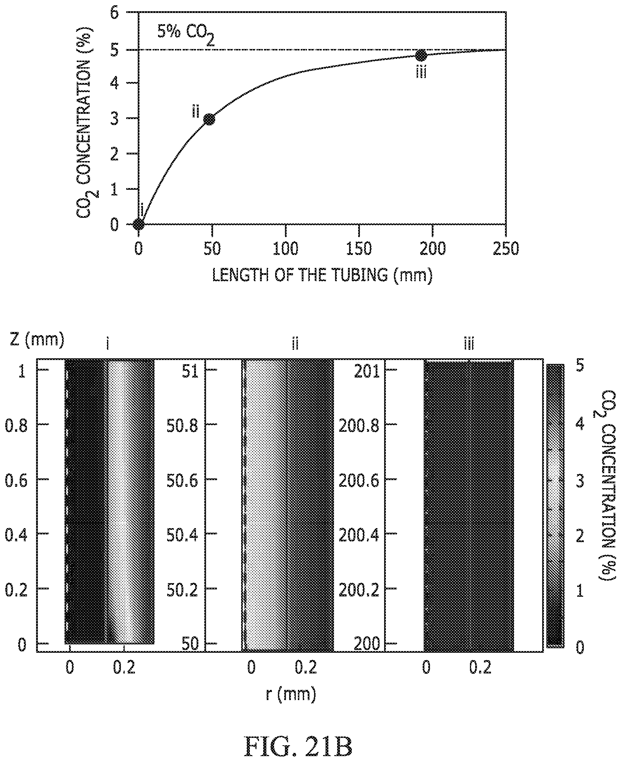

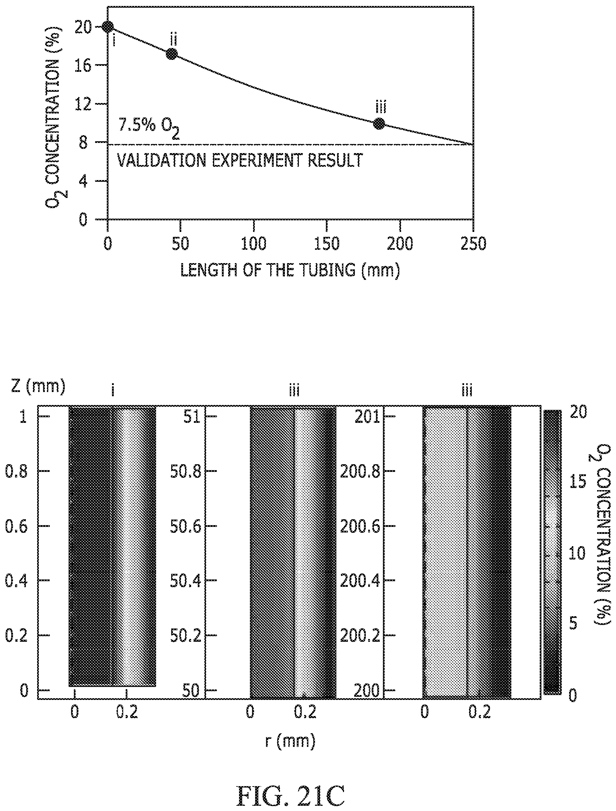

Fig. 21A shows the axisymmetric cross-section of the micro-gas exchanger depicting gas concentration and fluid flow boundary conditions. The gas diffusion rate depends on the flow rate, gas concentration, and tubing length. The micro-gas exchanger length is specifically designed to allow $CO_2$ and $O_2$ equilibrium before blood reaches the microchannel. The micro-gas exchanger design was optimized by computational analysis and validated experimentally. Based on the computational analyses, the length of the micro-gas exchanger was designed to be 250 mm, which provides 5% $CO_2$ and 7.5% $O_2$ concentration at the tubing outlet (*see* Figs. 21B-21C). The $CO_2$ concentration of blood started to increase as blood flows through the permeable tubing and reached equilibrium at 5% in 250 mm. The $O_2$ concentration of blood reached 7.5% in 250 mm through the micro-gas exchanger tubing.

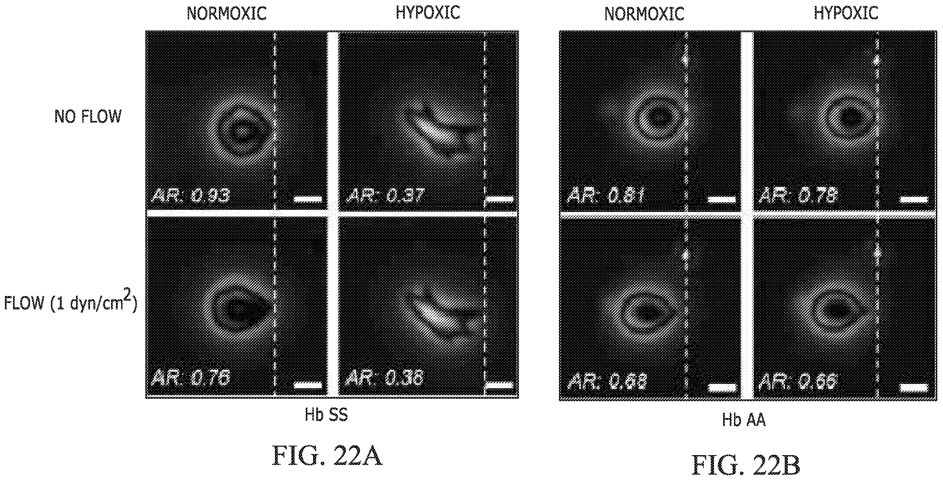

When blood samples from SCD subjects were injected into the microchannels 14a-14c through the micro-gas exchanger tubing 212, we observed single adhered RBCs on functionalized microchannel surfaces 16. Adhered RBCs sickled and their AR decreased upon deoxygenation (hypoxic conditions) from normoxic conditions. Furthermore, their deformability, indicated by the change in the AR in response to an applied flow shear stress of 1 dyne/cm$^2$, was decreased in hypoxic condition (Fig. 22A).

On the other hand, RBCs from healthy HbAA subjects preserved their morphology and deformability in hypoxic condition (Fig. 22B). The micro-gas exchanger 210 allowed continuous oxygenation-deoxygenation of adhered RBCs in the microchannels 14a-14c, which revealed sickling and unsickling of reversible sickle RBCs and accompanying biophysical changes.

*Heterogeneity in RBC Adhesion Response to Hypoxia*

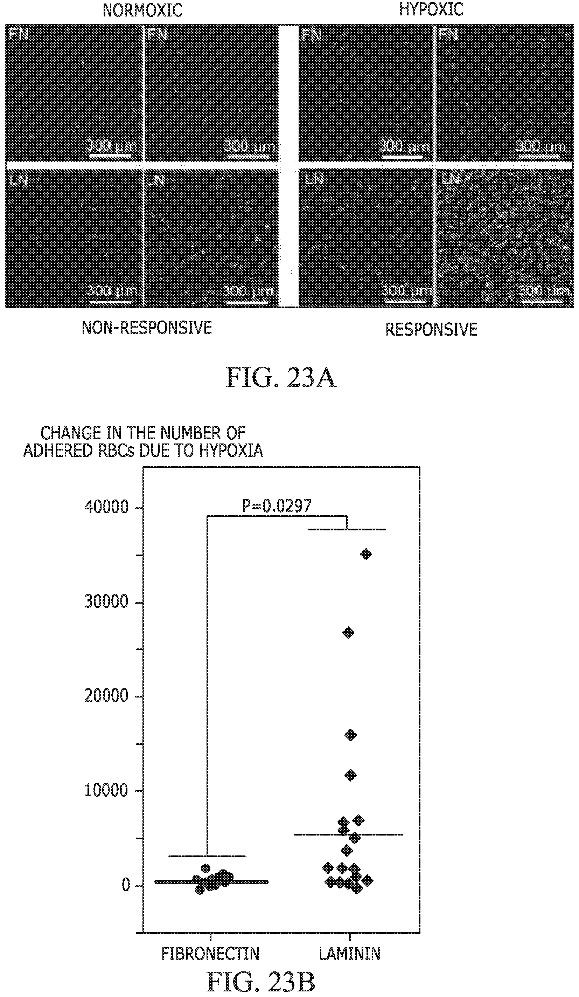

We analyzed the number of adhered RBCs in blood samples obtained from 35 SCD patients in hypoxic and normoxic conditions using the microfluidic device 10 integrated with the micro-gas exchanger 210. Blood samples from SCD patients showed heterogeneity in the increase of adhered RBCs between normoxic and hypoxic conditions (Figs. 23A-23C). A responsive subpopulation of patients showed a larger change in adhesion compared to the non-responsive patients for both FN and LN experiments (Fig. 23A). Colored dots indicate adherent RBCs. We observed a statistically significant change in the number of adhered RBCs to LN compared to FN in response to hypoxia (Fig. 23B, p<0.05, one-way ANOVA).

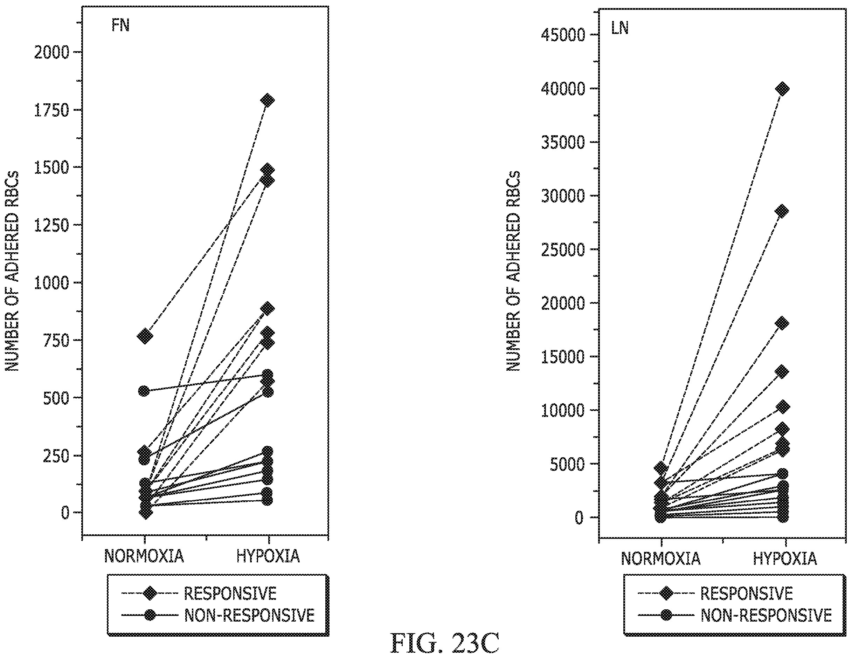

Next, we clustered SCD patient blood samples in two subpopulations as responsive and non-responsive based on the increase in the number of adhered RBCs to FN (N=17) and LN (N=24) in response to hypoxia (Fig. 23C).

*Clinical Association with the Number of Adhered RBCs under Hypoxic Conditions*

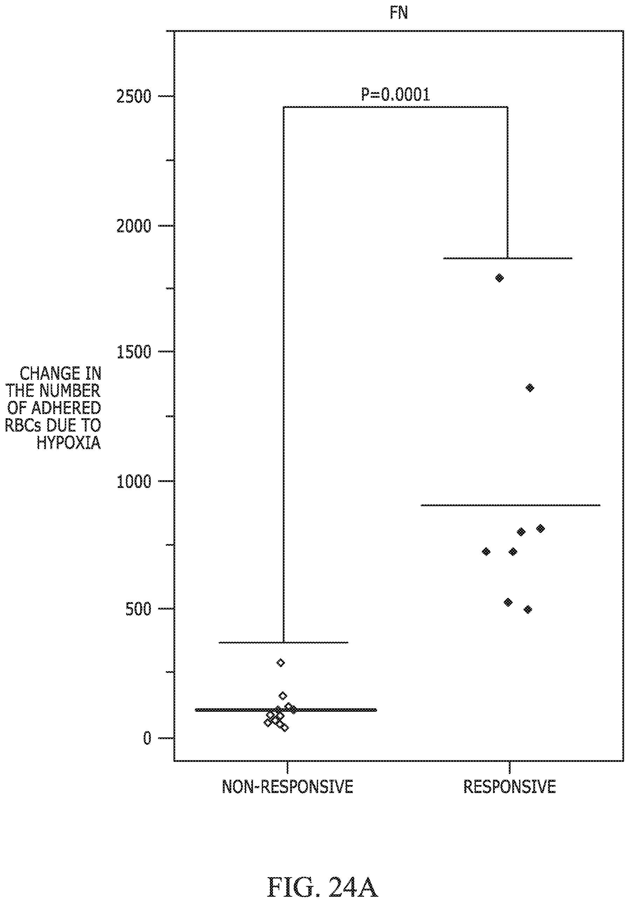

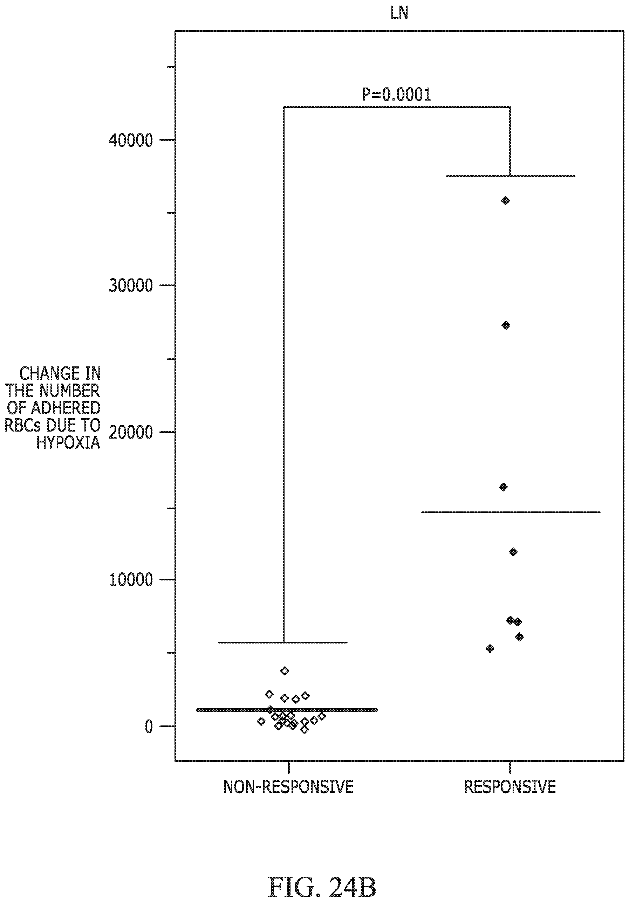

Referring to Figs. 24A-24B, blood samples from responsive SCD patients displayed a significantly greater change in the number of adhered RBCs to FN (N=17) and LN (N=26) in response to hypoxia compared to blood samples from non-responsive SCD subjects.

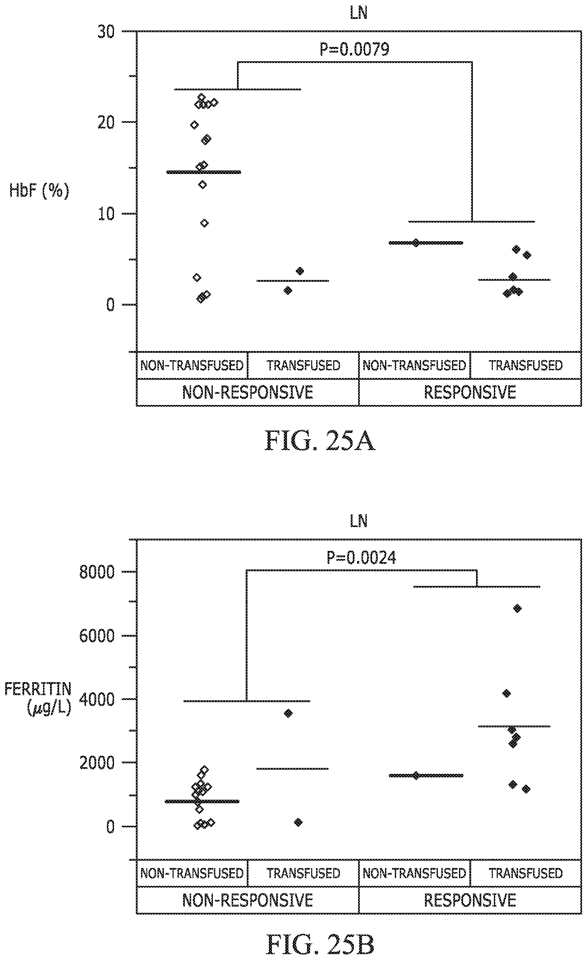

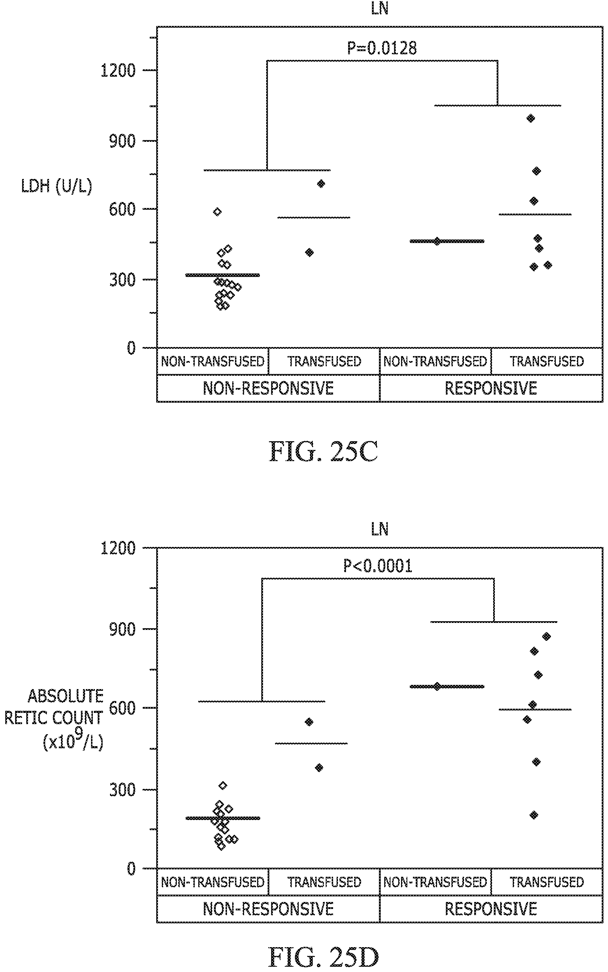

The responsive and non-responsive SCD patients displayed statistically significant differences in clinical phenotypes, including HbF percentages (Fig. 25A), ferritin levels (Fig. 25B), serum LDH levels (Fig. 25C), and retics (Fig. 25D). The non-responsive patient subpopulation showed significantly greater HbF values compared to responsive patient subpopulation (Fig. 25A, p<0.05, one-way ANOVA). All responsive patients showed less than 7% HbF. Moreover, the responsive patient subpopulation showed significantly greater ferritin levels, serum LDH levels, and retics than the non-responsive patient subpopulation (Figs. 25B-25D, p<0.05, one-way ANOVA).

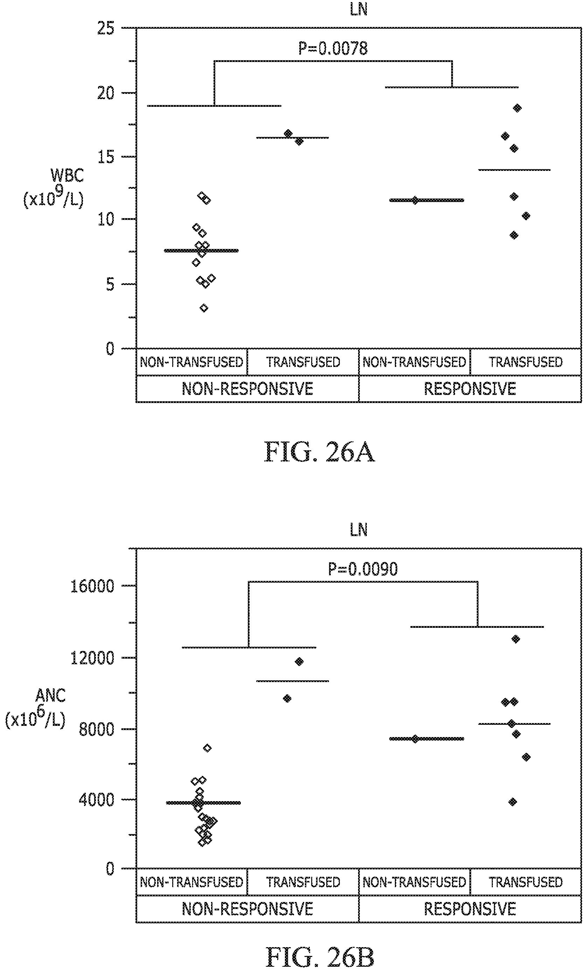

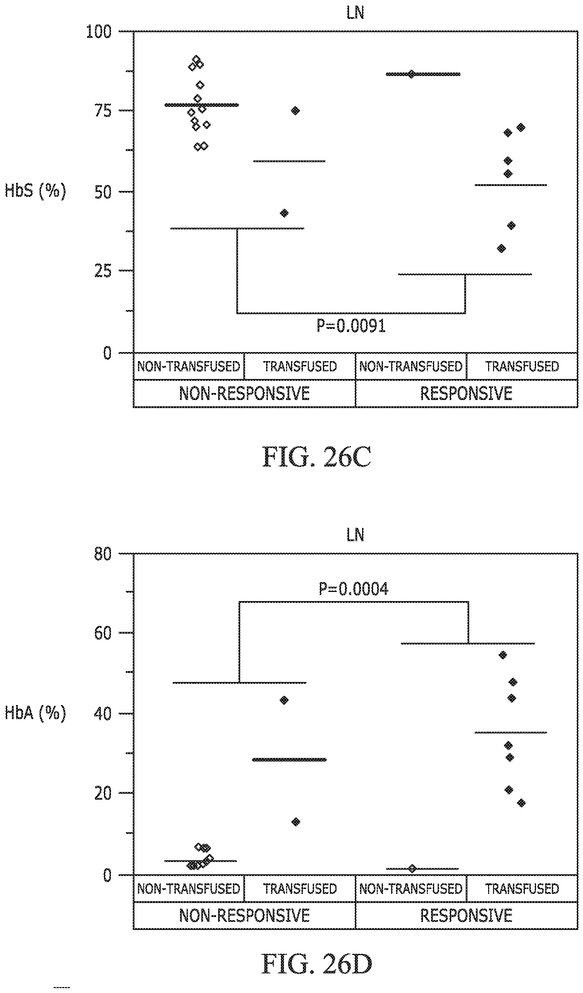

We then compared transfused (>10% HbA) and non-transfused SCD patients, and observed that both 7 out of 8 subjects in the responsive subpopulation were transfused, whereas only 2 out of 15 subjects in the non-responsive subpopulation were transfused (Figs. 25 and 26). Furthermore, the responsive patient subpopulation showed significantly greater WBC counts, greater neutrophil counts, and greater HbA percentage than the non-responsive patient subpopulation (Figs. 26A-26D, p<0.05). The responsive patients showed significantly lower HbS percentage in comparison to non-responsive patients (Figs. 26C-26D, p<0.05).

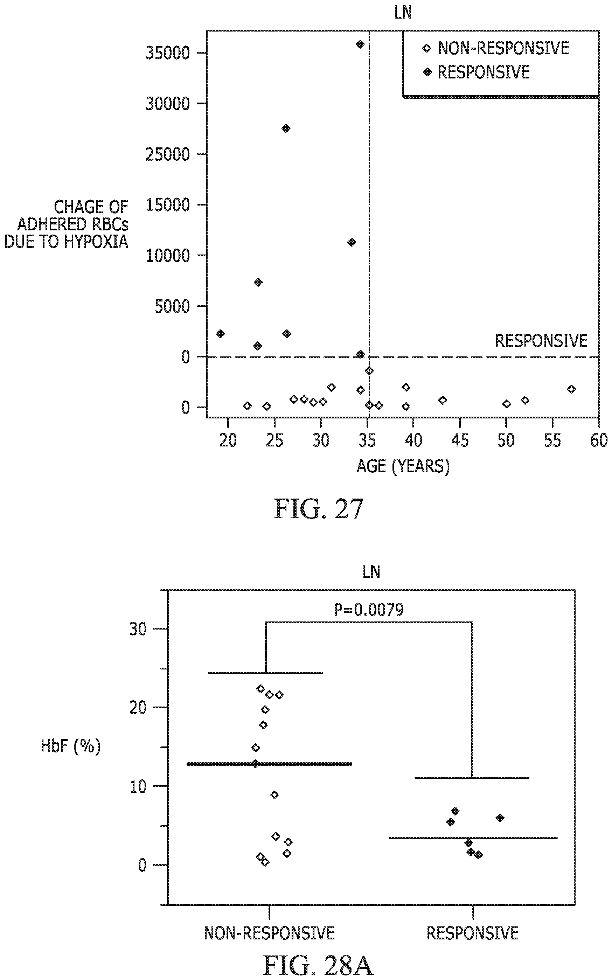

The age distribution of responsive patients was significantly younger (<35 years) than non-responsive patients (Fig. 27, p<0.05, one-way ANOVA). All the patients in the hypoxia responsive subpopulation (N=8) in LN were younger (<35 years). The baseline change in adhesion of RBCs to LN is shown in 16 subjects <35 and 10 subjects ≥35 years of age. 8/16 subjects <35 years old and 0/10 subjects ≥35 years old were responsive to hypoxia. The age distribution difference was statistically significant between responsive and non-responsive patients (p<0.05).

*Role of BCAM/Lu Blocking Under Hypoxia*

BCAM/Lu is indicated to be a critical adhesion receptor for LN, which can be phosphorylated with adrenergic stimulation. To test the role that BCAM/Lu played in the observed effect of hypoxia on adhesion, we analyzed RBC adhesion in hypoxic and normoxic conditions in whole blood samples treated with anti-BCAM antibody (R&D Systems, Minneapolis, MN) in parallel to untreated control samples.

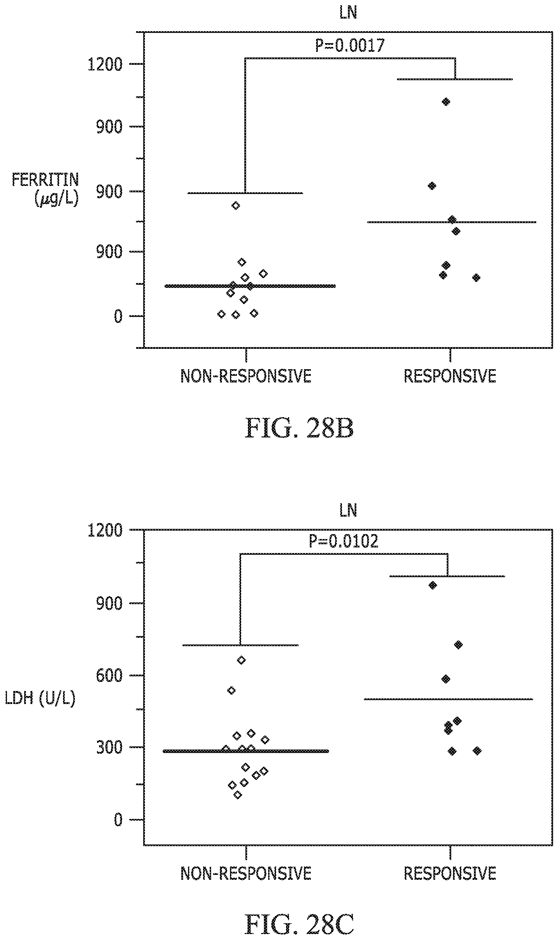

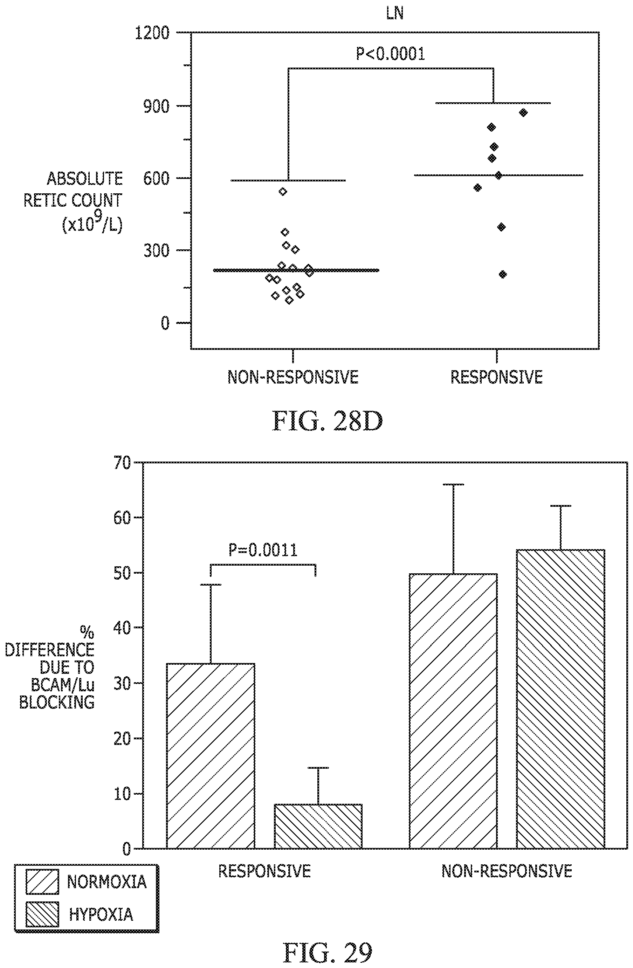

In Figs. 28A, all the patients in hypoxia responsive subpopulation (N=8) displayed less than 7% fetal hemoglobin (HbF). The HbF percentage of the responsive patient subpopulation was significantly lower compared to non-responsive patient subpopulation (N=15). In Fig. 28B, the responsive patient subpopulation showed significantly greater ferritin levels in comparison to non-responsive patient subpopulation. The hypoxia responsive patient subpopulation displayed significantly greater retics (Fig. 28C) and serum LDH (Fig. 28D) levels compared to non-responsive patient subpopulations.

As shown in Fig. 29, under normoxic conditions, the BCAM/Lu blocking decreased the number of adhered RBCs by 34% for responsive samples and 50% for non-responsive samples. Moreover, the BCAM/Lu blocking decreased number of adhered RBCs by 54% for non-responsive samples under hypoxic conditions. The change in the number of adhered RBCs was significantly lower in hypoxic conditions in comparison to normoxic conditions for responsive patient subpopulation. On the other hand, the change in the number of adhered RBCs was not significant between hypoxic and normoxic conditions for non-responsive patient subpopulation.

The percent decrease in the number of adhered RBCs were significantly lower in hypoxic conditions compared to normoxic conditions for the responsive patient subpopulation (N=4). On the other hand, there was no statistically significant difference between percent decrease in the number of adhered RBCs in hypoxic and normoxic conditions for the non-responsive patient subpopulation (N=6).

This example showed the adhesion of RBCs to endothelium associated proteins, FN and LN, in a close microscale environment under physiologically relevant flow and hypoxic conditions. We showed significant associations between the responsive patient subpopulation and clinical phenotypes, including HbF levels, ferritin levels, serum LDH levels, and retics. Hydroxyurea (HU), the only approved drug for SCD, induces production of HbF in RBCs, which interferes with HbS polymerization. It has been shown that HbF induction, overall, alleviates the clinical outcomes of the SCD. In this regard, it is plausible to hypothesize SCD patient blood samples with low HbF levels would be more prone to the deleterious effects of hypoxia, as we showed in Fig. 28.

Furthermore, ferritin levels were shown to increase with iron overload caused by blood transfusion in SCD patients. In parallel to this observation, we found greater serum ferritin levels in the responsive subpopulation, of which 7 out of 8 patients were transfused, compared to non-responsive patients. However, increased ferritin levels were also associated with vaso-occlusive crisis in earlier reports

LDH and retics have been expressed as an indicator of intravascular hemolysis, which is a critical element in pathophysiology of SCD. Hemolysis of RBCs and consequent release of heme has a detrimental impact on endothelial activation. Free hemoglobin and arginase released from lysed RBCs scavenge nitric oxide (NO) and decrease its bioactivity, which is a critical regulator of vascular tone. Moreover, intravascular hemolysis triggers an erythropoietic response in bone marrow, which results in elevated reticulocytes in blood. Even though increased levels of soluble adhesion molecules, such as vascular cell adhesion molecule 1 (VCAM-1), intercellular adhesion molecule 1 (ICAM-1), P-selectin, E-selectin, and Von Willebrand factor (VWF), have been associated with elevated serum LDH levels, the direct relationship between the adhesion of RBCs and intravascular hemolysis has yet to be revealed in SCD. In this study, we identified a unique patient subpopulation designated as responsive, of whose blood samples show an increased number of adhered RBCs in response to hypoxia, with significantly greater serum LDH levels and retics compared to other analyzed subjects. Our responsive patient subpopulation overall shown a hemolytic phenotype, increased LDH and reticulocytosis, which is also indicated for other debilitating clinical complications in SCD patients, such as priapism.

We demonstrated effective oxygen tension control of blood samples from SCD patients and observed increased adhesion of RBCs under hypoxic conditions. By comparing the number of adhered RBCs between normoxic and hypoxic conditions, we reported, for the first time in the literature, that there is a heterogeneity in patients' response to hypoxia. Our results associated with the clinical phenotypes that responsive samples had higher retics, serum LDH levels, lower HbF levels, and higher ferritin levels.

## Example 4

In this example, a microfluidic device having a microchannel with a variable width along its length was constructed, thereby creating a continuously variable shear rate gradient for fluid flow. Blood samples were injected into the microchannels and various RBC data was acquired on the variable shear rate gradient of the microchannels.

### Materials and Methods

### Blood collection

De-identified sickle blood samples were obtained from subjects with homozygous (HbSS) SCD at University Hospitals CWRU (Cleveland, OH, Division of Hematology and Oncology), under an Institutional Review Board approved protocol. Informed content documentation was acquired from all subjects. Initially, blood was drawn into Ethylenediaminetetraacetic acid (EDTA)-containing tubes and then aliquoted in 1 mL sealed micro centrifuge tubes while preventing blood exposure to ambient air.

A small portion of each sample was sent to the Core Laboratory of University Hospitals Cleveland Medical Center (UHCMC) for High Performance Liquid Chromatography (HPLC) analysis with the Bio-Rad Variant II Instrument (Bio-Rad, Montreal, QC, Canada) to determine the hemoglobin proportions (HbA, HbS, HbF, and HbA2). Subject clinical data including WBC count ($10^9$/L), platelet count ($10^9$/L), absolute neutrophil count (ANC) ($10^6$/L), reticulocyte count ($10^9$/L), total hemoglobin (g/dL), plasma LDH (IU/L), and ferritin level ($\mu$g/L) were obtained from the Adult Sickle Cell Clinic at UHCMC in Cleveland, Ohio. All experiments in this study were conducted using whole blood samples within 24 hours following venipuncture without any dilution or further processing.

### Materials

FN and LN stock solutions (1 mg/mL) were purchased from Sigma Aldrich (St. Louis, MO, USA). Prior to surface functionalization, both protein solutions were diluted in 1x PBS (pH=7.4) to have a working concentration of 0.1 mg/mL. Bovine Serum Albumin (BSA) was purchased from Sigma Aldrich in a lyophilized powder form and dissolved in 1x PBS at a concentration of 20 mg/mL. 4-Maleimidobutyric acid N-hydroxysuccinimide ester (GMBS) powder was purchased from ThermoFisher Scientific (Waltham, MA, USA). Stock solutions of recombinant ICAM-1 and VCAM-1 proteins were purchased from Biolegend (San Diego, CA) and diluted to a final concentration of 25 $\mu$g/mL prior to perfusing into the microchannels.

### Microchannel fabrication

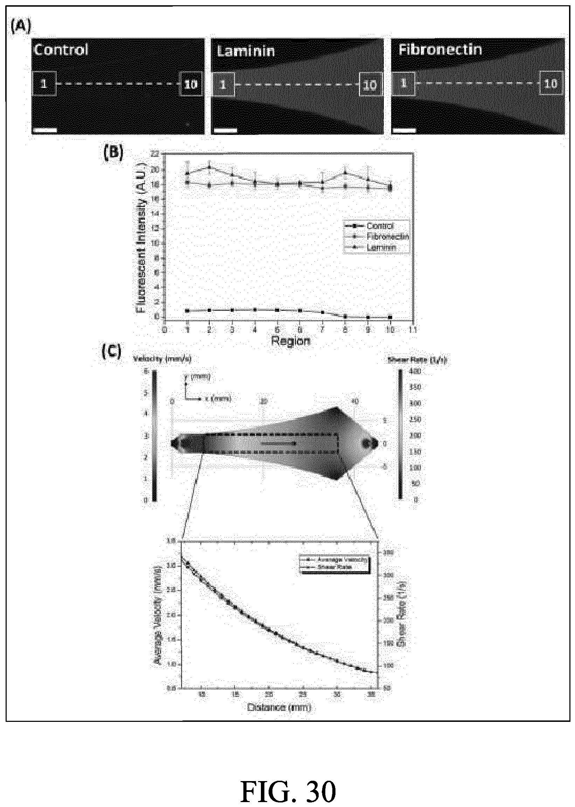

The microchannels were fabricated by assembling a DSA film on a rectangular PMMA piece and fixing the PMMA onto a functionalized microscope glass slide (Gold Seal, coated with APTES, 3-Aminopropyl Triethoxysilane, Electron Microscopy Sciences, Hatfield, PA) through the DSA. The adhesive film was micro-machined using a laser cutting system (VersaLaser) to define the microchannel walls. Two identical holes (0.61 mm in diameter) were drilled on the PMMA cap spaced 41mm from each other to form the inlet and outlet ports using VersaLaser. The microchannel height/depth was determined by the DSA thickness, which was approximately 50 $\mu$m and which mimicked the scale of postcapillary venules. The entire microchannel cross-section is shown in Fig. 30.

### Surface functionalization

A cross-linker agent 4-Maleimidobutyric acid N-hydroxysuccinimide ester (GMBS) was used in order to covalently immobilize the endothelial proteins FN and LN to the microchannel surfaces. The stock GMBS solution was obtained by dissolving 25 mg of GMBS in 0.25 mL of dimethyl sulfoxide (DMSO), and it was diluted with pure ethanol to have a final concentration of 0.28 % v/v GMBS solution. After assembly, the microfluidic devices were rinsed with PBS (1x) and 100% ethanol. Next, a 60 $\mu$l of GMBS working solution was perfused into the microchannels twice followed by 15-minute incubation in room temperature. The microchannels were then washed with 90 $\mu$l of ethanol and PBS twice. 60 $\mu$l of FN, LN, ICAM-1 or VCAM-1 working solutions (100 $\mu$g/mL for FN & LN; 25 $\mu$g/mL for ICAM-1 & VCAM-1) were injected into the microchannels and incubated for 1.5 hours at room temperature. In order to prevent non-specific binding, a 90 $\mu$l of BSA solution was added into the microchannels, held at 4°C overnight.

We characterized the surface coverage of the immobilized proteins -LN and FN - *via* fluorescent labeling. The functionalized microchannels were rinsed with 90 $\mu$L of PBS twice to remove the unbound BSA solution following the overnight incubation. Next, fluorescently labeled antibody solutions against human LN and FN were loaded into the microchannels and incubated for 45 minutes. Thereafter, the microchannels were rinsed with PBS twice and the functionalized surface was imaged on an epiflourescent microscope at 10X.

The same procedure was carried out with the control surface, which was loaded with PBS excluding LN and FN and incubated with BSA at 4° C overnight using the fluorescently labeled antibody solution against human LN. Fig. 30A illustrates the protein coverage in shear-gradient, non-functionalized, LN-functionalized, and FN-functionalized microchannels. The white rectangles indicate the regions along which RBC analyses were quantified. The quantification of fluorescent intensity, assumed to be indicative of the amount of immobilized protein, shows negligible background fluorescence on the control surface and reveals protein coverage over the functionalized surfaces (Fig. 30B).

### Blood processing and data acquisition

The microchannels were placed on an Olympus IX83 inverted motorized microscope stage for visualization following the assembly of inlet tubings. Next, 300 $\mu$l of blood in a 1 mL syringe was connected to a constant displacement syringe pump (New Era NE-300, Farmingdale, NY). Blood was perfused into the microchannels at a constant flow rate of 18.5 $\mu$l/min until they were completely filled. Afterwards, the flow rate was decreased to 1.85 $\mu$l/min, corresponding to an approximate shear stress of 1 dyne/cm$^2$ at the smallest microchannel cross-section, and 45 $\mu$l of blood was injected at this flow rate.

In order to wash the non-adherent cells off the microchannel surfaces, a new syringe containing 1 mL of wash buffer containing 1% FBS and 0.09% Sodium Azide was connected to the device and the buffer was pumped at a constant rate of 40 $\mu$l/min corresponding to a mean velocity of 200 mm/min at the smallest section of the converging cross-section. The buffer solution was allowed to perfuse until all non-adherent cells were completely cleared out of the microchannels. Next, the phase-contrast images of the microchannel surfaces with the adherent cells were recorded at 20X using the Olympus Cell Sense live imaging software. Post processing of recorded images and cell counting were performed using Adobe Photoshop CS5 (San Jose, CA, USA).

### Results and Discussion

### Computational analysis of the fluid flow in the shear gradient microchannel

Numerical simulations were performed by a commercially available FEA software package COMSOL 4.3 to characterize the velocity and shear rate profiles in the shear gradient microchannels using a 3D model. A structured mesh was created with a total number of 45120 hexahedral elements throughout the entire domain. The buffer fluid was modeled as Newtonian with a dynamic viscosity and density of 0.001 Pa.s and 993 kg/m$^3$, respectively. The inlet boundary condition was set to "Velocity Inlet" with a calculated average velocity of 26.7 mm/s from the volumetric flow rate and tubing dimensions. The outlet pressure was set to 0 as the buffer discharged to the atmospheric pressure. The Reynolds number inside the microchannel was computed using Equation 7 below:

$$Re = \frac{\rho Q h}{\mu A} \qquad\qquad (7)$$

where $\rho$ is the fluid density, Q is the volumetric flow rate, h is the characteristic length (microchannel height), $\mu$ is the dynamic viscosity of the fluid, and A is the cross-sectional area. The Reynolds number at the inlet where the width of the microchannel is 4 mm was approximately 0.2, indicating that the flow regime was predominantly in the laminar region.

Fig. 30C illustrates a 2D representation of the velocity and shear rate distributions along the microchannel. In order to mimic the local flow conditions in the vicinity of the surface adherent cells, the numerical results were evaluated on a plane that was positioned 5 $\mu$m above the bottom surface of the microchannel.

*Total RBC adhesion depends on the change of shear rate gradient*

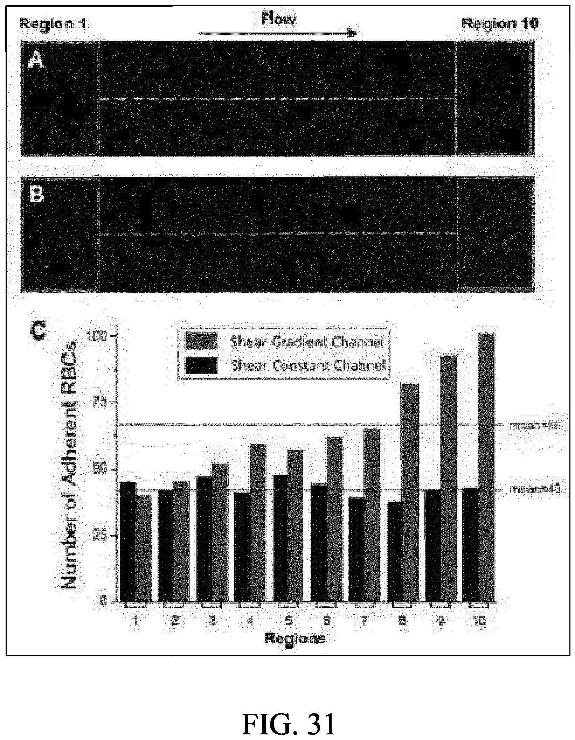

We analyzed the shear-dependent adhesion characteristics of HbSS RBCs to LN and FN in a shear gradient environment. Based on the numerical simulations, we chose a rectangular region of interest (ROI) for experimental data analysis as shown in Fig. 30C. Next, we divided each ROI into 10 identical sub-regions and quantified the mean shear rates for individual sub-regions using COMSOL (Fig. 31A). Then, we correlated the number of adherent cells in each sub-region with the corresponding mean shear rate.

Figs. 31A-31B are visual representations of the adherent RBCs in LN immobilized shear constant (straight rectangular microchannel) and shear gradient (variable width microchannel of the present invention) respectively for a single SCD subject. The shear constant microchannel was fabricated using the same technique described above, but with a constant width corresponding to the inlet width of the shear gradient microchannel (w = 4mm). Figs. 31A-31B utilize the ROIs defined earlier in Fig. 30. The red dots illustrate the individual adherent RBCs (not to scale). Fig. 31C shows the variation in the number of RBCs attached to LN within individual sub-regions. The adherent cells did not change across the shear constant microchannel and were substantially homogeneously distributed along the functionalized microchannel surface.

In the shear gradient microchannel, however, RBC adhesion was enhanced at low shear rates by greater than two-fold relative to the first shear rate region. In the first two sub-regions, where the mean shear rates did not differ significantly, the adhesion rates were comparable. We observed a more than a 1.5-fold increase in average RBC adhesion throughout the ROI in the shear gradient microchannel (Fig. 31C).

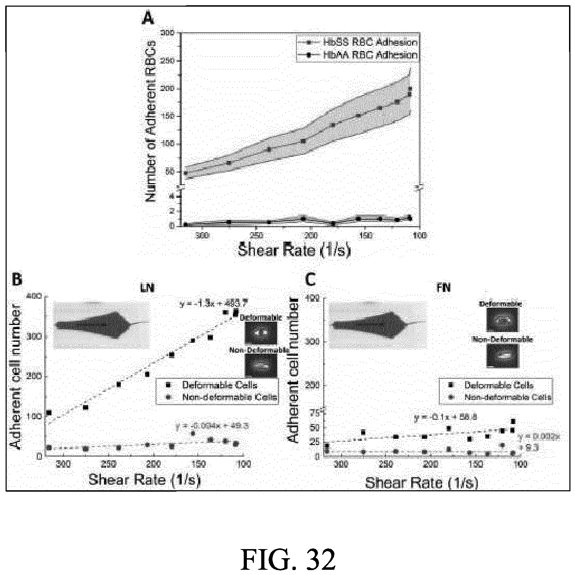

Fig. 32A shows the average adhesion curves for control (non-SCD, healthy individuals, HbAA) and HbSS samples in LN-functionalized microchannels. HbAA samples displayed negligible RBC adhesion to LN with no significant correlation between shear rate and number of adherent RBCs (n=6). HbSS RBCs adhered to LN with a shear-dependent profile showing a more than three-fold increase in average adherent RBC numbers throughout the ROI.

To ensure that the concentration of flowing cells did not significantly change across the width W of the microchannel from Region 1 to Region 10, we developed a CFD model to evaluate the variation in RBC distribution within the ROI. We carried out a computational study of the 3D particle flow to simulate the motion of RBC-sized spherical particles through the shear-gradient microchannel. The 3D transient numerical study for the particle flow was performed using COMSOL 4.3 by introducing spherical particles with a diameter of 3 $\mu$m to simulate flowing RBCs.

The initial particle velocities were adjusted based on the flow field at the inlet. A total number of 2000 particles were released every 1 s for a total simulation duration of 40 seconds, at which point the flow reached steady-state. The particle density was set to 1.1 g/mL, simulating the average density of an individual RBC. In the beginning, the particles were more diluted further downstream due to the inlet continuously receiving particles. As the flow progressed, the particle concentration became more consistent and reached a steady state approximately 40 seconds after flow initiation. At steady state, the particle concentration was consistent throughout the sub-regions of the ROI, with a 5.4% to 7.8% variation of the mean.

Figs. 32B-32C show distributions of surface adherent HbSS RBCs with varying shear rates from a single sample in LN and FN functionalized microchannels. To assess the extent to which individual RBC subpopulations responded to the shear rate gradient, we fit linear regression curves for both deformable and non-deformable RBC adhesion data and observed a significantly greater deformable RBC adhesion rate in LN immobilized channels in comparison to FN channels for the sample tested.

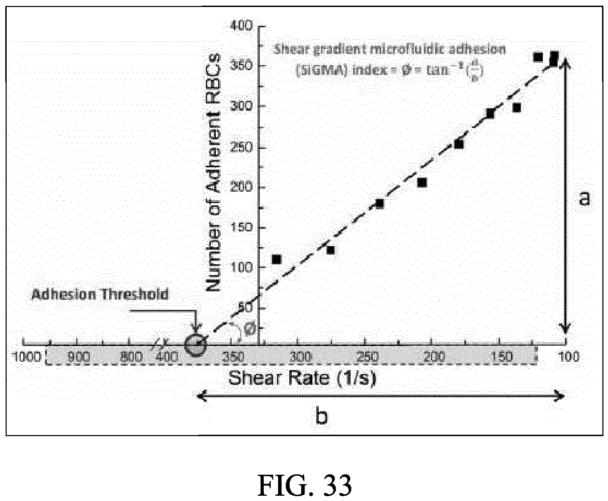

Moreover, the deformable RBC to LN adhesion was highly shear dependent, showing a more than three-fold increase in adhered cell numbers between region 1 and region 10. In order to quantify the shear dependent adhesion of individual samples, the absolute value of the slope of the curves was defined as the "RBC Shear Gradient Microfluidic Adhesion (SiGMA) index", in which a decreased SiGMA index suggests greater adhesion under higher shear rates. The linear regression curves and corresponding regression equations were generated in OriginLab (Northampton, MA) for all the samples tested. Negative slopes in Figs. 32B-32C indicated declining adhesion numbers with increasing shear rate. A graphical illustration of the SiGMA index is shown in Fig. 33.

*Quantification of shear dependent adhesion rate in LN and FN channels*

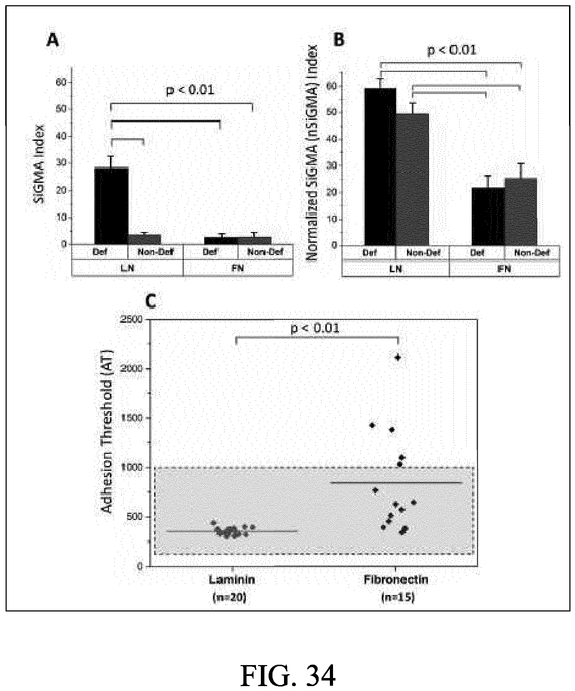

The calculated SiGMA indices for a group of SCD subjects are shown in Fig. 34A. Deformable RBC adhesion to LN was shear dependent, compared with non-deformable RBC adhesion to LN, as well as deformable and non-deformable RBC adhesion to FN (p<0.01). The influence of shear rate may be minimized in relatively low-adhesion samples, where low absolute numbers of adherent RBCs negated the effect of shear rate. We therefore normalized the SiGMA indices based on RBC adhesion in the first sub-region of the entire field by computing the normalized adhesion numbers for each sub-region and generated normalized adhesion curves using Equation 8 below: *Number of adhered RBCs in a subregion*

$$Normalized\ Adhesion\ Number = \frac{Number\ of\ adhered\ RBCs\ in\ a\ subregion}{Number\ of\ adhered\ RBCs\ in\ first\ subregion} \times 100 \qquad (8)$$

Next, we defined the slope of each normalized adhesion curve as normalized Shear Gradient Microfluidic Adhesion (nSiGMA) index. Equation 8 was used to determine the percent increase in RBC numbers in a specific sub-region compared to the first sub-region. Hence, the influence of total adhered cell numbers on SiGMA index calculations was negated, which allowed for a comparison from both low- and high-adhesion samples.

Fig. 34B shows the difference in nSiGMA indices between HbSS RBC adhesion to LN or FN (p<0.01). Although the deformable cells displayed increased nSiGMA, we did not observe a significant correlation between the two RBC subpopulations. Deformable and non-deformable HbSS RBCs had different responses to changes in shear rate. The calculated SiGMA indices indicated that deformable RBCs were more shear dependent than non-deformable ones. In other words, deformable cells were more sensitive to changes in shear rate and could be removed to a significantly greater extent with increasing shear rates. However, this behavior might have been amplified due to the high number of adherent RBCs to LN.

nSiGMA indices revealed that adhesion of both deformable and non-deformable RBCs to LN was more shear dependent than adhesion to FN, as shown in Fig. 34B. We did not observe a significant difference between shear dependent responses by deformable and non-deformable RBCs. In general, FN-adherent RBCs did not respond to the shear gradient and maintained persistent attachment with an approximate nSiGMA value of 20 (Fig. 34B). A high rate (elevated slope) of shear dependent adhesion implied a tendency of adherent RBCs to detach relatively easily at high shear sites in the microchannel. Lower shear dependency corresponded to more persistent RBC adhesion, which might not be influenced as much by elevated shear rates.

*Adhesion threshold in FN microchannels revealed persistent RBC adhesion beyond the physiological limits*

In addition to SiGMA and nSiGMA indices described herein, we further calculated the x-axis intercept of the regression curves obtained for each sample, which was defined as the adhesion threshold, as previously illustrated in Fig. 33. The intercept point represented the predicted shear rate required to remove all adherent RBCs (deformable and non-deformable) from LN or FN. Therefore, an increased adhesion threshold implied that adherent RBCs would persist at high shear rates.

As shown in Fig. 34C, the estimated mean adhesion threshold for HbSS RBCs adherent to LN was 351 s$^{-1}$. All adhesion threshold values were within a physiological shear range of 121s$^{-1}$ (minimum) and 952 s$^{-1}$ (maximum), typical for post-capillary venules. In contrast, the mean adhesion threshold for FN adherent cells was 839 s$^{-1}$. Our data indicated that more attachment-detachment events may take place in a LN functionalized microchannel for a given SCD subject, yet the physiological shear rates could prevent a persistent RBC attachment in the system. On the other hand, FN-adherent RBCs, despite fewer absolute attachments, may remain attached for longer periods of time when exposed to physiological shear rates.

*Shear dependent RBC adhesion associates with clinical phenotype in SCD*

We have observed a heterogeneous response of adherent RBCs to the shear gradient as indicated by several parameters, namely: SiGMA, nSiGMA, and adhesion threshold. This heterogeneity exists both at the individual SCD subject level and in the population. Therefore, we investigated potential associations between adhesion data and clinical phenotypes in SCD.

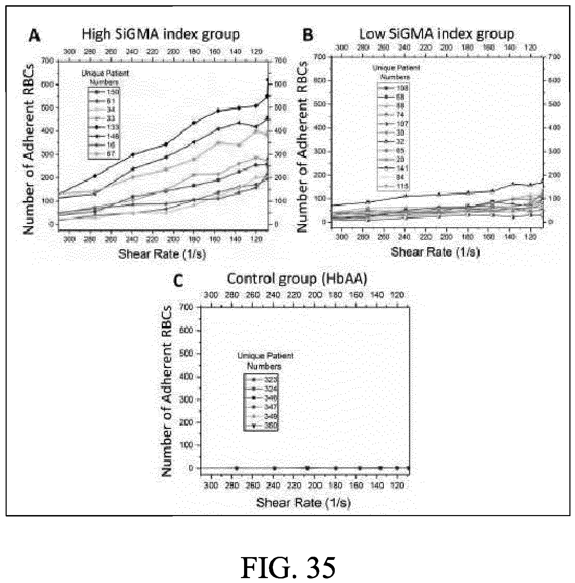

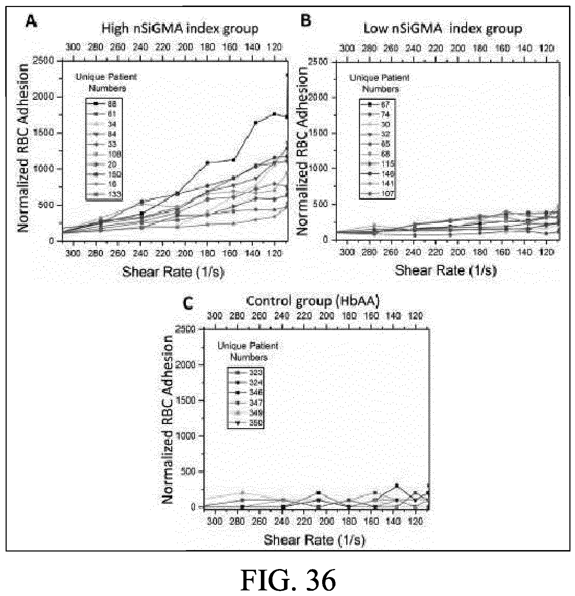

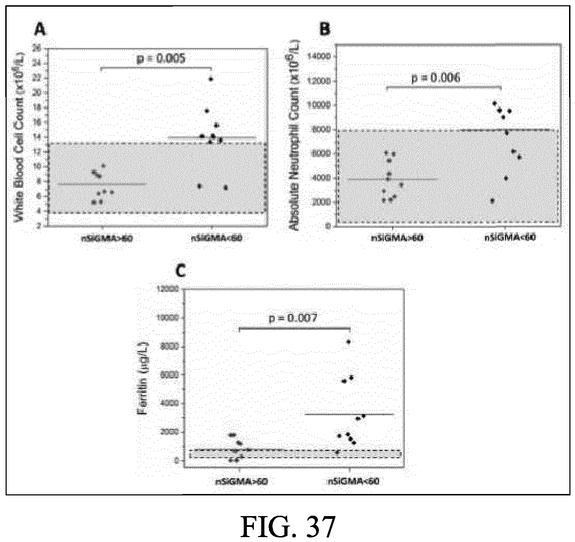

Figs. 35-36 illustrate the individual adhesion curves, from which the adhesion rates and normalized adhesion rates were calculated, for each SCD sample along with a control group (HbAA). In particular, Figs. 35A-35C show the subject adhesion curves based on raw adherent RBC numbers. Figs. 36A-36C show the adhesion curves based on normalized adherent RBC numbers in LN-functionalized microchannels. We separated the subject population to two groups arbitrarily based on a nSiGMA index of 60. The adherent cell numbers represent the summation of both deformable and non-deformable RBC. The statistical analyses were carried out with Minitab 17 (Minitab, State College, PA) software package. The data were analyzed using ANOVA with Turkey's post hoc test for multiple comparisons and the p value for each dataset reported (*see* Figs. 37A-37C).

Subjects with low response to shear dependent adhesion to LN, *i.e.,* less reduction of adhesion with increasing shear rates, lower nSiGMA, displayed elevated levels of WBC, ANC, and ferritin (*see* Figs. 37A-37C having corresponding p values of 0.005, 0.006, and 0.007 respectively; one-way ANOVA) (*see also* Table 3 below).

Table 3 - Clinical phenotype of the study population based on their shear dependent adhesion characteristics

| | High shear dependent adhesion (nSiGMA>60) (Mean ± St. Err.) | Low shear dependent adhesion (nSiGMA>60) (Mean ± St. Err.) | Range | P-value* |
|---|---|---|---|---|
| Age | 33.9 ± 4.57 | 30.4 ± 1.89 | 21-61 | 0.488 |
| Hgb (g/dL) | 8.54 ± 0.49 | 7.35 ± 0.62 | 2.6-10.7 | 0.149 |
| WBC ($10^9$/L) | 7.6 ± 0.56 | 13.9 ± 1.37 | 5.2-21.9 | 0.005 |
| ANC ($10^6$/L) | 3898 ± 479.4 | 7979 ± 1215 | 2130-15930 | 0.006 |
| Platelet Count | 330.8 ± 60.1 | 353.7 ± 24.07 | 174-781 | 0.728 |
| Reticulocyte | 248.4 ± 20.77 | 408.6 ± 75.24 | 122-813 | 0.055 |
| Lactate | 363.3 ± 61.68 | 518 ± 72.75 | 154-813 | 0.122 |
| Ferritin ($\mu$g/L) | 784.5 ± 219.62 | 3272.3 ± 791.9 | 11-8320 | 0.007 |
| Hemoglobin S | 73.48 ± 6.69 | 59.96 ± 6.45 | 27.2-91 | 0.190 |
| Hemoglobin A | 13.22 ± 7.29 | 26.85 ± 6.32 | 1.4-66.8 | 0.206 |
| Hemoglobin F | 7.05 ± 1.95 | 3.89 ± 1.34 | 0.7-18.1 | 0.240 |

A total of 20 blood samples (n) were obtained from 20 subjects (Male=10, Female=10).
All subjects are homozygous HbSS.
WBC: White blood cell count ANC: Absolute neutrophil count
* Computed based on ANOVA one-way test.

Inflammatory cells play a critical role in initiating vaso-occlusive crises and contribute to the pathophysiology of SCD in various ways. Here, we showed that subjects with high WBC counts exhibit lower shear dependent red cell adhesion characteristics to LN, with the implication that RBC attachment in these subjects is stronger and resistant to high shear rates for cell detachment (Fig. 37A). We also observed a similar behavior with absolute neutrophil counts (ANC) (Fig. 37B) in accordance with an earlier study that reported a correlation between peripheral neutrophil count and clinical severity of SCD.

It has been reported that neutrophils are highly activated in sickle cell subjects particularly during vaso-occlusive events. Neutrophil activation in SCD could be triggered by various chemokines present in blood serum such as interleukin-8 (IL-8), sickled RBC-dependent IgG-enhanced as well as complement activation pathways, and activated endothelial line. It has previously been shown that activation of sickle RBCs by IL-8 induced RBC adhesion to the endothelium enhanced by FN.

Here, our findings indicate that ANCs may also play a role in mediating the adhesion strength of RBCs to LN, which could indicate a novel pathway for neutrophils to contribute to severity of the disease. Further studies are required to unearth the mechanism underlying the association between neutrophils and shear dependent RBC adhesion.

SCD subjects on chronic and prolonged RBC transfusion therapy are likely to suffer from iron overload that can lead to serious complications and even higher mortality rates. Therefore, it is critical to accurately monitor the states of iron overload of such subjects. Serum Ferritin (SF) is one of the most common clinical tests in determining iron overload that provides adequately reliable results. In addition to the clinical implications of iron overload in SCD, we have shown that increased SF levels are inversely proportional with the shear dependent adhesion of RBCs to LN (Fig. 37C). Hence, it could be speculated that the presence of excessive intracellular iron levels helps enhance the adhesion strength of sickle RBCs to LN. Alternatively, RBCs from subjects with more severe disease (requiring transfusion) may exhibit higher nSiGMA indices.

Our preliminary findings - from 3 subjects with SCD - also suggest shear-dependent adhesion characteristics of sickle RBCs in LN, FN, ICAM-1, and VCAM-1 functionalized microchannels.

This example examined the shear dependent adhesion of HbSS RBCs to LN and FN in a microfluidic platform in which the shear rate gradient was imposed based on the microchannel geometry along the flow direction. The shear gradient flow was continuous and driven by a single pump at a constant flow rate.

We found subject-specific and clinically relevant parameters: RBC SiGMA index, RBC nSiGMA index, and adhesion threshold (AT) values. These parameters describe the dynamic pathophysiology of SCD and the role of shear rate in vaso-occlusion. We observed a significant, subject-dependent variation of AT for FN-functionalized mircochannels as opposed to LN-functionalized microchannels. This could also be attributed to the high rate of persistently-adhered, non-deformable RBCs to FN. Moreover, our findings suggested a link between nSiGMA and subject clinical phenotypes of inflammation and iron overload - with a lower nSiGMA being associated with a worse outcome.

## Example 5

In this study, we showed the efficacy of using a point-of-care (POC) device for monitoring cellular adhesion in a specific subject and adhesion type.

### Materials

The POC device included a Samsung Galaxy S4, 3D optical attachment, microfluidic device, and custom application software. The 3D optical attachment was designed using a 3D modeling software and built by 3D printing. Optics included an LED, diffuser, and ball lens implemented to evenly illuminate the blood sample and provide magnified images of adhered cells to the Samsung camera.

### Results and Discussion

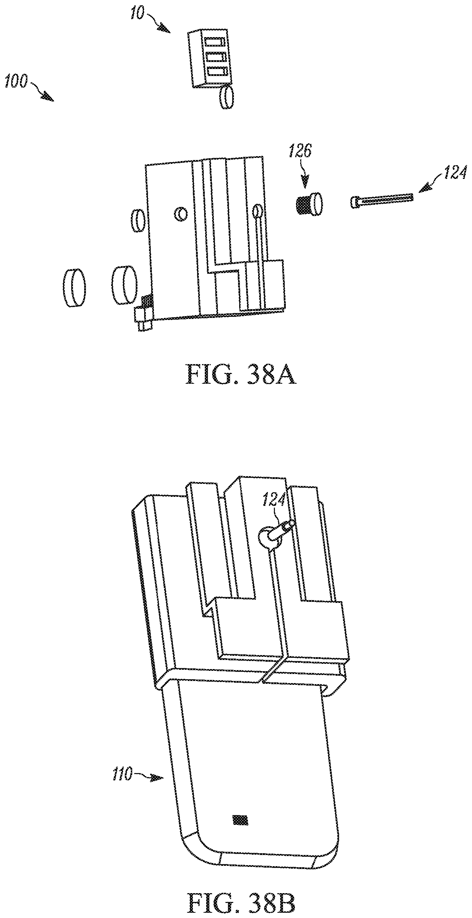

Referring to Figs. 38A-38E, a microfluidic device 10 having multiple microchannels 14 was constructed as described in Example 1 and provided in the optical attachment member 100 of the imaging system 140 (Fig. 38A). The optical attachment member 100 was connected to a cellular phone 110 (Fig. 38B). The microchannels 14 were functionalized with various endothelium proteins.

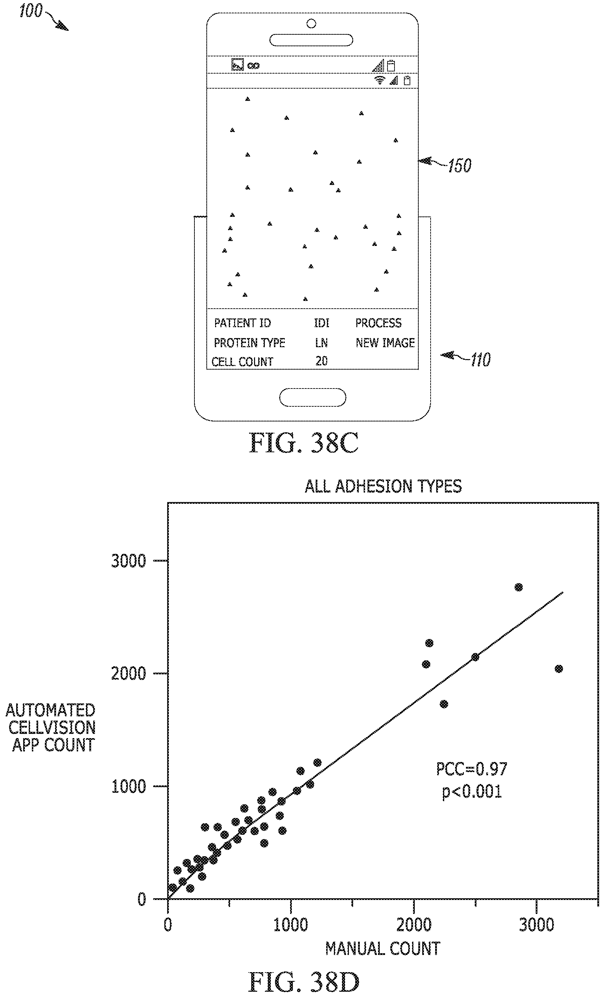

A blood sample 20 was delivered to the microchannels 14. The mobile application on the cellular phone 110 cooperated with the LED 126 to take images of the adhered RBCs in the microchannels 14 (Fig. 38C). The images were automatically processed to quantify the RBC adhesion number for the subject for each endothelium protein. More specifically, image capturing and digital image processing were done by utilizing the OpenCV library for Android. An adaptive threshold was used to turn protential cells white and the background black.

Next, all white contours were identified and filled in with white color. Automated cell counting was then performed using the OpenCV simple blob feature detector, which labeled all properly sized objected in the image as cells. The POC system sampled a 1 mm$^2$ square of the whole microchannel and predicted the number of captured cells in the whole channel by applying a multiplication factor.

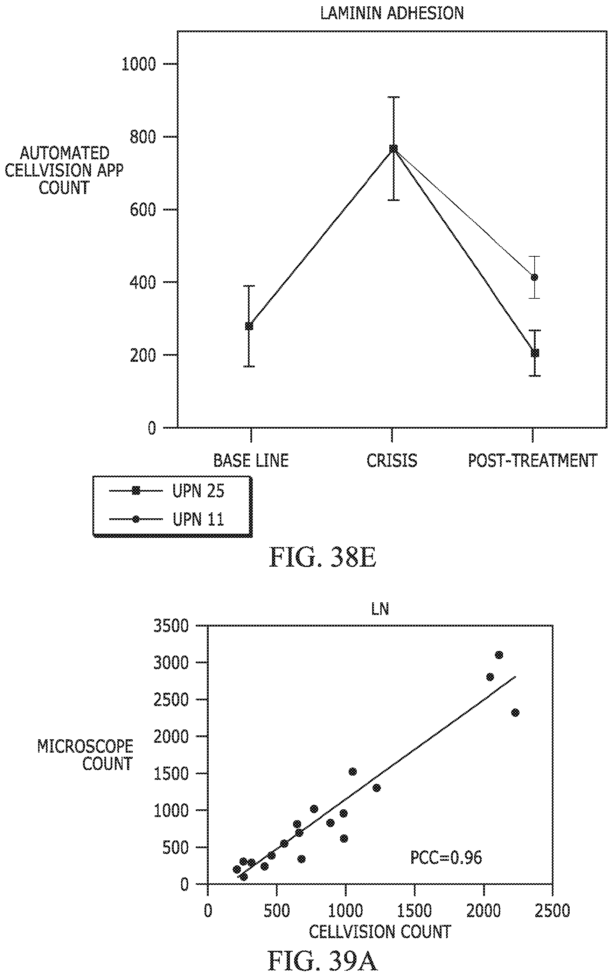

The adhered cell counts were also manually counted. The software then compared and correlated the manual count with the software count for all adhesion types (Fig. 38D). Example preliminary data collected from two subjects using the POC device related to LN-specific adhesion (Fig. 38E). The subjects were receiving anti-adhesion therapy and n = 3-9 measurements for each data point.

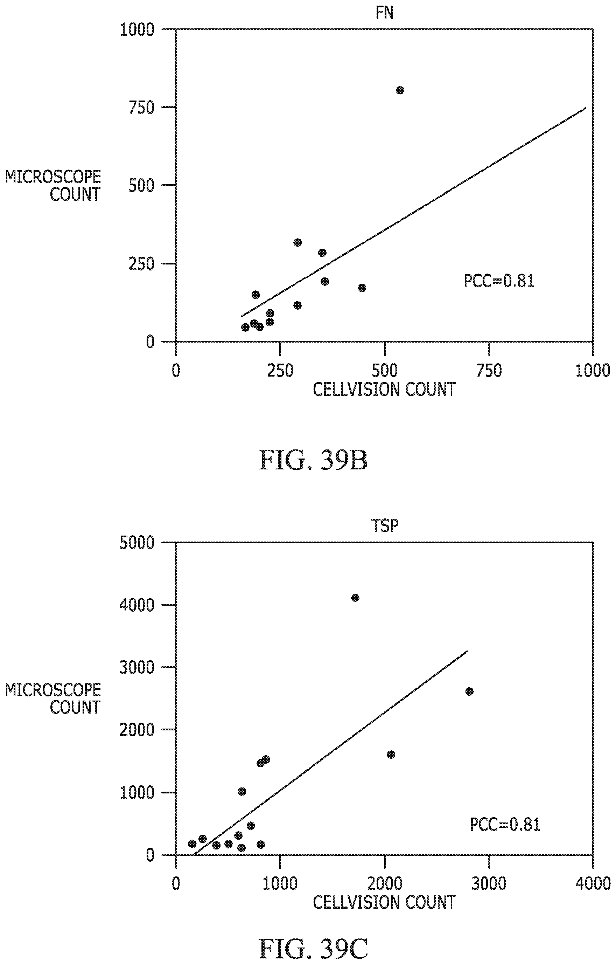

Figs. 39A-39C are additional correlations between counts of adhered RBC obtained by scanning microscope and those obtained using the mobile application CellVision 150. The data was generated for LN-functionalized microchannels (Fig. 39A), FN-functionalized microchannels (Fig. 39B), and TSP-functionalized microchannels (Fig. 39C). We observed variable correlation across the functionalized proteins. LN held the highest PCC of 0.96 (p<0.05) due to its highly homogenous adhesion. Relatively lower PCCs were observed for FN and TSP (both 0.81; p<0.05).

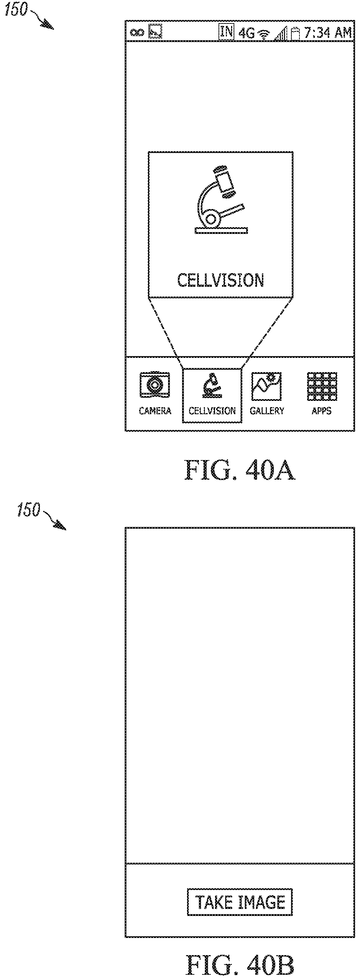

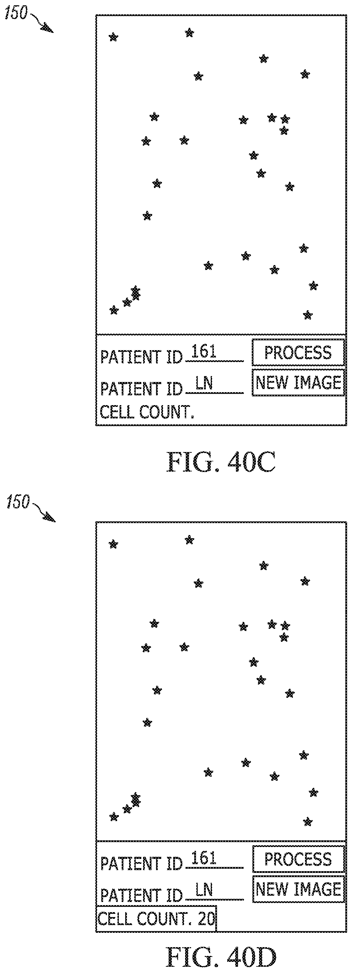

Figs. 40A-40D illustrate specifics of the mobile application 150 - here called CellVision. The mobile application 150 was accessible as an icon on the screen of the cellular phone 110 (Fig. 40A). Once the mobile application 150 was initiated, the screen of the cellular phone 110 became a live image feed of the microfluidic device 10 (Fig. 40B). The subject was able to select a "TAKE IMAGE" icon on the screen to initiate data collection. Once the image was taken (Fig. 40C), the subject manually entered their identification number and the endothelium protein used to functionalize the microchannels. The subject then selected a "PROCESS" icon on the screen, which activated the photo algorithm used to quantify the adhered RBCs. A final still image identifying the quantified RBCs was presented to the subject on the cellular phone 110 screen (Fig. 40D) as well as a cell count of the adhered RBCs. The patient identifier and adhesion level was automatically saved and could be transmitted to electronic records.

**Claims**

1. A microfluidic biochip device (10) comprising:
a housing (12) including at least one microchannel (14) defining at least one cell adhesion region (16), the at least one cell adhesion region (16) being provided with at least one capturing agent that adheres a cell of interest being a red blood cell to a surface of the at least one microchannel (14) when a fluid sample (20) comprising blood containing cells is passed through the at least one microchannel (14); and an imaging system (60) for measuring the morphology and/or quantity of cells of interest being red blood cells adhered by the at least one capturing agent to the surface of the at least one microchannel (14) when the fluid sample (20) comprising blood is passed therethrough, **characterized in that** the at least one microchannel (14) has a width that continuously changes in a direction of fluid flow comprising blood therethrough and the capturing agent comprises a bioaffinity ligand including at least one of fibronectin, laminin, thrombospondin, selectin, von Willebrand Factor (vWF) or a C146 antibody.

2. The biochip device (10) of claim 1 further comprising a micro-gas exchanger (210) fluidly connected to the at least one microchannel for varying and controlling the oxygen content of the blood prior to delivering the blood to the at least one microchannel (14).

3. The biochip device (10) of claim 2, wherein the micro-gas exchanger (210) provides hypoxic blood to the at least one microchannel (14).

4. The biochip device (10) of claim 1, wherein the microchannel (14) has a convergent and divergent cross-sectional area along the direction of flow.

5. The biochip device (10) of claim 1, wherein the shear stress on fluid comprising blood flowing through the microchannel (14) decreases along the length of the microchannel (14).

6. The biochip device (10) of claim 1, the capturing agent being covalently immobilized to surfaces of each microchannel (14) with a cross-linker, the cross-linker preferably being GMBS.

7. The biochip device (10) of claim 1, the imaging system (60) comprising:

   an optical attachment member (100) for connecting the housing (12) to a cellular phone (110); and
   software usable by the cellular phone (110) for quantifying the adhered cells of interest being red blood cells, wherein, preferably, the imaging system (60) further comprises a lens (122), a diffuser, a light emitting diode (LED) (126), and an LED holder (124).

8. A method of evaluating a fluid sample (20) from a subject, comprising:

   providing a housing (12) including at least one microchannel (14) defining at least one cell adhesion region, the at least one cell adhesion region being provided with at least one capturing agent that adheres a cell of interest being a red blood cell in the fluid sample (20) comprising blood to a surface of the at least one microchannel (14); injecting the fluid sample (20) into each microchannel (14); imaging the number of cells of interest adhered to the surface of the at least one microchannel (14) to determine a quantity and/or aspect ratio of the cells of interest being red blood cells; and determining a condition of the subject based on the imaged cells of interest, **characterized in that** the at least one microchannel (14) has a width that continuously changes in a direction of fluid flow comprising blood through the at least one microchannel (14) to provide a shear gradient for fluid flow comprising blood through the device (10) and the step of providing a housing (12) with at least one cell adhesion region comprises functionalizing the at least one microchannel (14) with a bioaffinity ligand including at least one of fibronectin, laminin, thrombospondin, selectin, von Willebrand Factor (vWF) or a C146 antibody.

9. The method of claim 8, wherein the step of determining a condition comprises correlating the quantified red blood cells with a hemoglobin phenotype.

10. The method of claim 8 further comprising correlating the number of adhered red blood cells in different portions of the microchannel (14) to a corresponding mean shear rate.

11. The method of claim 8 further comprising controlling the oxygen content of the blood prior to delivering the blood to the at least one microchannel (14).

12. The method of claim 8 further comprising covalently immobilizing the capturing agent to the surfaces of each microchannel (14) with a cross-linker, wherein, the cross-linker preferably is GMBS.

**Patentansprüche**

1. Mikrofluidische Biochip-Vorrichtung (10) umfassend:
   ein Gehäuse (12), das mindestens einen Mikrokanal (14) enthält, der mindestens einen Zelladhäsionsbereich (16) definiert, wobei der mindestens eine Zelladhäsionsbereich (16) mit mindestens einem Einfangmittel versehen ist, das eine Zelle von Interesse, die eine rote Blutzelle ist, an eine Oberfläche des mindestens einen Mikrokanals (14) anhaftet, wenn eine Fluidprobe (20), die Blut umfasst, das Zellen enthält, durch den mindestens einen Mikrokanal (14) geleitet wird; und ein Abbildungssystem (60) zum Messen der Morphologie und/oder der Menge der interessierenden Zellen, die rote Blutkörperchen sind, die durch das mindestens eine Einfangmittel an der Oberfläche des mindestens einen Mikrokanals (14) haften, wenn die flüssige Probe (20), die Blut enthält, durch diesen hindurchgeleitet wird, **dadurch gekennzeichnet, dass** der mindestens eine Mikrokanal (14) eine Breite aufweist, die sich in einer Richtung des Fluidstroms, der Blut umfasst, kontinuierlich ändert, und das Einfangmittel einen Bioaffinitätsliganden umfasst, der mindestens eines von Fibronektin, Laminin, Thrombospondin, Selectin, von Willebrand-Faktor (vWF) oder einen Cl46-Antikörper einschließt.

2. Biochip-Vorrichtung (10) nach Anspruch 1 ferner umfassend einen Mikrogasaustauscher (210), der fluidisch mit dem mindestens einen Mikrokanal verbunden ist, um den Sauerstoffgehalt des Blutes zu variieren und zu steuern, bevor das Blut in den mindestens einen Mikrokanal (14) geleitet wird.

3. Biochip-Vorrichtung (10) nach Anspruch 2, wobei der Mikrogasaustauscher (210) dem mindestens einen Mikrokanal (14) hypoxisches Blut zuführt.

4. Biochip-Vorrichtung (10) nach Anspruch 1, wobei der Mikrokanal (14) eine konvergente und divergente Querschnittsfläche entlang der Strömungsrichtung aufweist.

5. Biochip-Vorrichtung (10) nach Anspruch 1, wobei die Scherbeanspruchung des durch den Mikrokanal (14) fließenden, Blut umfassenden Fluids über die Länge des Mikrokanals (14) abnimmt.

6. Biochip-Vorrichtung (10) nach Anspruch 1, wobei das Einfangmittel kovalent an den Oberflächen jedes Mikrokanals (14) mit einem Vernetzer immobilisiert ist, wobei der Vernetzer vorzugsweise GMBS ist.

7. Biochip-Vorrichtung (10) nach Anspruch 1, wobei das Abbildungssystem (60) umfasst:

   ein optisches Befestigungselement (100) zum Verbinden des Gehäuses (12) mit einem Mobiltelefon (110); und Software, die von dem Mobiltelefon (110) zur Quantifizierung der anhaftenden Zellen von Interesse, bei denen es sich um rote Blutkörperchen handelt, verwendet werden kann, wobei das Abbildungssystem (60) vorzugsweise ferner eine Linse (122), einen Diffusor, eine lichtemittierende Diode (LED) (126) und einen LED-Halter (124) umfasst.

8. Verfahren zum Auswerten einer Flüssigkeitsprobe (20) von einem Subjekt, umfassend:

   Bereitstellen eines Gehäuses (12) mit mindestens einem Mikrokanal (14), der mindestens einen Zelladhäsionsbereich definiert, wobei der mindestens eine Zelladhäsionsbereich mit mindestens einem Einfangmittel versehen ist, das eine Zelle von Interesse, die eine rote Blutzelle ist, in der Fluidprobe (20), die Blut umfasst, an eine Oberfläche des mindestens einen Mikrokanals (14) anhaftet;
   Injizieren der flüssigen Probe (20) in jeden Mikrokanal (14);
   Abbilden der Anzahl der interessierenden Zellen, die an der Oberfläche des mindestens einen Mikrokanals (14) haften, um eine Menge und/oder ein Seitenverhältnis der interessierenden Zellen, die rote Blutkörperchen sind, zu bestimmen und Bestimmen eines Zustands des Subjekts auf der Grundlage der abgebildeten Zellen von Interesse, **dadurch gekennzeichnet, dass** der mindestens eine Mikrokanal (14) eine Breite aufweist, die sich in einer Richtung des Fluidstroms, der Blut umfasst, durch den mindestens einen Mikrokanal (14) kontinuierlich ändert, um einen Schergradienten für den Fluidstrom, der Blut umfasst, durch die Vorrichtung (10) bereitzustellen, und der Schritt des Bereitstellens eines Gehäuses (12) mit mindestens einem Zelladhäsionsbereich das Funktionalisieren des mindestens einen Mikrokanals (14) mit einem Bioaffinitätsliganden umfasst, der mindestens eines von Fibronektin, Laminin, Thrombospondin, Selectin, von Willebrand Faktor (vWF) oder einem Cl46-Antikörper einschließt.

9. Verfahren nach Anspruch 8, wobei der Schritt der Bestimmung eines Zustands das Korrelieren der quantifizierten roten Blutkörperchen mit einem Hämoglobin-Phänotyp umfasst.

10. Verfahren nach Anspruch 8 ferner umfassend das Korrelieren der Anzahl der anhaftenden roten Blutkörperchen in verschiedenen Abschnitten des Mikrokanals (14) mit einer entsprechenden mittleren Scherrate.

11. Verfahren nach Anspruch 8 ferner umfassend die Kontrolle des Sauerstoffgehalts des Blutes vor der Zuführung des Blutes zu dem mindestens einen Mikrokanal (14).

12. Verfahren nach Anspruch 8 ferner umfassend das kovalente Immobilisieren des Einfangmittels an den Oberflächen jedes Mikrokanals (14) mit einem Vernetzer, wobei der Vernetzer vorzugsweise GMBS ist.


**Revendications**

1. Dispositif microfluidique de type biopuce (10) comprenant :
   un logement (12) comportant au moins un microcanal (14) définissant au moins une région d'adhérence cellulaire (16), l'au moins une région d'adhérence cellulaire (16) étant dotée d'au moins un agent de capture qui fait adhérer une cellule d'intérêt, étant un globule rouge, à une surface de l'au moins un microcanal (14) lorsqu'un échantillon de fluide (20) comprenant du sang contenant des cellules passe à travers l'au moins un microcanal (14) ; et un système de formation d'images (60) pour mesurer la morphologie et/ou la quantité de cellules d'intérêt étant des globules rouges adhérant, par l'au moins un agent de capture, à la surface de l'au moins un microcanal (14) lorsque l'échantillon de fluide (20) comprenant du sang passe à travers celui-ci, **caractérisé en ce que** l'au moins un microcanal (14) présente une largeur qui change continuellement dans une direction d'écoulement de fluide comprenant du sang à travers celui-ci et l'agent de capture comprend un ligand de bioaffinité comportant au moins un élément parmi la fibronectine, la laminine, la thrombospondine, la sélectine, le facteur de von Willebrand (vWF) ou un anticorps en C146.

2. Dispositif de type biopuce (10) selon la revendication 1, comprenant en outre un microéchangeur gazeux (210) connecté fluidiquement à l'au moins un microcanal pour faire varier et réguler la teneur en oxygène du sang avant de distribuer le sang à l'au moins un microcanal (14).

3. Dispositif de type biopuce (10) selon la revendication 2, dans lequel le microéchangeur gazeux (210) fournit du sang hypoxique à l'au moins un microcanal (14).

4. Dispositif de type biopuce (10) selon la revendication 1, dans lequel le microcanal (14) présente une zone de section transversale convergente et divergente le long de la direction d'écoulement.

5. Dispositif de type biopuce (10) selon la revendication 1, dans lequel la contrainte de cisaillement sur le fluide comprenant du sang circulant à travers le microcanal (14) diminue le long du microcanal (14).

6. Dispositif de type biopuce (10) selon la revendication 1, l'agent de capture étant immobilisé de manière covalente sur les surfaces de chaque microcanal (14) avec un agent de réticulation, l'agent de réticulation étant de préférence le GMBS.

7. Dispositif à type biopuce (10) selon la revendication 1, le système de formation d'images (60) comprenant :

   un organe de fixation optique (100) pour connecter le logement (12) à un téléphone portable (110) ; et
   un logiciel utilisable par le téléphone portable (110) pour quantifier les cellules adhérentes d'intérêt étant des globules rouges, dans lequel, de préférence, le système de formation d'images (60) comprend en outre une lentille (122), un diffuseur, une diode électroluminescente (DEL) (126) et un support de DEL (124).

8. Procédé d'évaluation d'un échantillon de fluide (20) provenant d'un sujet, comprenant :

la fourniture d'un logement (12) comportant au moins un microcanal (14) définissant au moins une région d'adhérence cellulaire, l'au moins une région d'adhérence cellulaire étant dotée d'au moins un agent de capture qui fait adhérer une cellule d'intérêt, étant un globule rouge dans l'échantillon de fluide (20) comprenant du sang, à une surface de l'au moins un microcanal (14) ;
l'injection de l'échantillon de fluide (20) dans chaque microcanal (14) ;
la représentation en image du nombre de cellules d'intérêt adhérant à la surface de l'au moins un microcanal (14) pour déterminer une quantité et/ou un rapport d'aspect des cellules d'intérêt étant des globules rouges ; et la détermination d'un état du sujet sur la base des cellules d'intérêt représentées, **caractérisé en ce que** l'au moins un microcanal (14) présente une largeur qui change en continu dans une direction d'écoulement de fluide comprenant du sang à travers l'au moins un microcanal (14) pour fournir un gradient de cisaillement pour l'écoulement de fluide comprenant du sang à travers le dispositif (10) et l'étape de fourniture d'un logement (12) avec au moins une région d'adhérence cellulaire comprend la fonctionnalisation de l'au moins un microcanal (14) avec un ligand de bioaffinité comportant au moins un élément parmi la fibronectine, la laminine, la thrombospondine, la sélectine, le facteur de von Willebrand (vWF) ou un anticorps en C146.

9. Procédé selon la revendication 8, dans lequel l'étape de détermination d'un état comprend la mise en corrélation des globules rouges quantifiés avec un phénotype d'hémoglobine.

10. Procédé selon la revendication 8 comprenant en outre la mise en corrélation du nombre de globules rouges adhérents dans différentes parties du microcanal (14) avec un taux de cisaillement moyen correspondant.

11. Procédé selon la revendication 8 comprenant en outre la régulation de la teneur en oxygène du sang avant la distribution du sang dans l'au moins un microcanal (14).

12. Procédé selon la revendication 8 comprenant en outre l'immobilisation covalente de l'agent de capture sur les surfaces de chaque microcanal (14) avec un agent de réticulation, dans lequel l'agent de réticulation est de préférence le GMBS.

FIG. 1A

FIG. 1B

FIG. 2A

FIG. 2B

EP 3 596 465 B1

FIG. 2C

36

FIG. 2D

FIG. 3

FIG. 4A

FIG. 4B

FIG. 4C

FIG. 5A

FIG. 5B

FIG. 6A

MEASURED FLOW VELOCITY($V_p$, mm/s)

$$Y=0.82X$$
$$R^2=0.88$$

CALCULATED MEAN FLOW VELOCITY($V_m$, mm/s)

■ 70μl/min ◆ 110μl/min ○ 120μl/min
◇ 130μl/min ● 400μl/min ▲ 500μl/min

FIG. 6B

SHEAR STRESS [Pa]

HBA        HBS          HBS
        DEFORMABLE   NON-DEFORMABLE

FIG. 6C

FIG. 6D

NO FLOW  DEVELOPING FLOW  STEADY FLOW  PROJECTED CELL OUTLINES

HbA

t=0sec  t=0.14sec  t=0.28sec

FIG. 7A  FIG. 7B  FIG. 7C  FIG. 7D

HbS
DEFORMABLE

t=0sec  t=0.14sec  t=0.28sec

FIG. 7E  FIG. 7F  FIG. 7G  FIG. 7H

HbS
NON-DEFORMABLE

t=0sec  t=0.14sec  t=0.28sec

FIG. 7I  FIG. 7J  FIG. 7K  FIG. 7L

CELL ASPECT RATIO (AR)

FIG. 8A

DEFORMABILITY (%AR CHANGE)

FIG. 8B

FIG. 9A

FIG. 9B

NUMBER OF ADHERED RBCs

FIG. 9C

NUMBER OF ADHERED RBCs

FIG. 9D

FIG. 9E

FIG. 9F

NUMBER OF ADHERED RBCs

## FIG. 10A

NUMBER OF ADHERED RBCs

## FIG. 10B

NUMBER OF ADHERED RBCs

FIG. 11A

NUMBER OF ADHERED RBCs

FIG. 11B

NUMBER OF ADHERED RBCs

FIG. 11C

NUMBER OF ADHERED RBCs

FIG. 11D

FIG. 11E

FIG. 11F

FIG. 11G

FIG. 11H

FIG. 12A

FIG. 12B

FIG. 12C

FIG. 12D

FIG. 12E

FIG. 12F

FIG. 12G

FIG. 13A

FIG. 13B

FIG. 13C

FIG. 13D

FIG. 14A

FIG. 14B

FIG. 14C

FIG. 15A

FIG. 15B

FIG. 15C

FIG. 15D

Flow Rate: 10 uL/min          1 mm

FIG. 16A

Flow Rate: 20 uL/min          1 mm

FIG. 16B

PERCENT CELL CAPTURE (%)

FIG. 16C

FIG. 17A

FIG. 17B

FIG. 17C

CAPTURE EFFICIENCY (%)

FIG. 17D

CYTOMETER CELL COUNT (PER μl)

FIG. 18A

FACS CELL COUNT (PER μl)

FIG. 18B

FACS CELL COUNT (PER μl)

FIG. 18C

FACS CELL COUNT (PER $\mu l$)

FIG. 18D

FIG. 19A

FIG. 19B

EP 3 596 465 B1

DIRECTION OF $O_2$ DIFFUSION

DIRECTION OF $CO_2$ DIFFUSION

215  214

212

218

216

210

FIG. 19C

(i)

SENSING REGION

(ii)

10

50

40

40

40

30

# FIG. 20A

FIG. 20B

*(A/B): A=$CO_2$ CONCENTRATION, B=$O_2$ CONCENTRATION (%)

FIG. 20C

PERMEABLE TUBING

IMPERMEABLE TUBING

BLOOD FLOW

CONTROLLED GAS

| | |
|---|---|
| ----------- | AXISYMMETRIC LINE |
| ——— | FLUID FLOW BCs |
| ——— | GAS CONCENTRATION BCs |

FIG. 21A

FIG. 21B

FIG. 21C

NORMOXIC   HYPOXIC

NO FLOW

AR: 0.93   AR: 0.37

FLOW (1 dyn/cm²)

AR: 0.76   AR: 0.38

Hb SS

FIG. 22A

NORMOXIC   HYPOXIC

AR: 0.81   AR: 0.78

AR: 0.68   AR: 0.66

Hb AA

FIG. 22B

NORMOXIC                        HYPOXIC

NON-RESPONSIVE                  RESPONSIVE

FIG. 23A

CHANGE IN THE NUMBER OF
ADHERED RBCs DUE TO HYPOXIA

FIG. 23B

FIG. 23C

FN

FIG. 24A

FIG. 24B

FIG. 25A

FIG. 25B

FIG. 25C

FIG. 25D

FIG. 26A

FIG. 26B

FIG. 26C

FIG. 26D

LN

FIG. 27

LN

FIG. 28A

FIG. 28B

FIG. 28C

FIG. 28D

FIG. 29

FIG. 30

FIG. 31

FIG. 32

FIG. 33

FIG. 34

FIG. 35

FIG. 36

FIG. 37

10

100

126

124

FIG. 38A

124

110

FIG. 38B

100

150

| PATIENT ID | IDI | PROCESS |
| PROTEIN TYPE | LN | NEW IMAGE |
| CELL COUNT | 20 | |

110

FIG. 38C

ALL ADHESION TYPES

PCC=0.97
p<0.001

AUTOMATED
CELLVISION
APP COUNT

MANUAL COUNT

FIG. 38D

LAMININ ADHESION

FIG. 38E

FIG. 39A

FIG. 39B

FIG. 39C

150

FIG. 40A

150

TAKE IMAGE

FIG. 40B

*150*

PATIENT ID  161

PATIENT ID  LN

CELL COUNT.

PROCESS

NEW IMAGE

FIG. 40C

*150*

PATIENT ID  161

PATIENT ID  LN

CELL COUNT. 20

PROCESS

NEW IMAGE

FIG. 40D

**EP 3 596 465 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62596630
- US 62570380
- US 62472437

- WO 2016019142 A1
- WO 2016164359 A1

**Non-patent literature cited in the description**

- **CHENG XUANHONG et al.** Lab on a chip. Royal Society of Chemistry, 24 November 2006, vol. 7, 170-178